# FASCICULE DE BREVET EUROPEEN

(11) **EP 1 737 840 B1**
(45) Date de publication et mention de la délivrance du brevet: **12.09.2007**
(21) Numéro de dépôt: 05757289.3
(22) Date de dépôt: 13.04.2005
(51) Int. Cl.: C07D 401/12, C07D 239/32, C07D 239/34, C07C 65/30, C07C 65/32, A61K 31/502

(54) **DÉRIVÉS DE LA 1-AMINO-PHTHALAZINE, LEUR PRÉPARATION, ET LEUR APPLICATION EN THÉRAPEUTIQUE**
1-AMINOPHTHALAZINDERIVATE, DEREN HERSTELLUNG UND DEREN THERAPEUTISCHE VERWENDUNG
1-AMINO-PHTHALAZINE DERIVATIVES, THE PREPARATION AND THE THERAPEUTIC USE THEREOF

(30) Priorité: 13.04.2004 FR 0403806
(43) Date de publication de la demande: 03.01.2007
(73) Titulaire: Sanofi-Aventis, 75013 Paris (FR)
(72) Inventeur: AUGEREAU, Jean Michel, F-31400 Toulouse (FR); GESLIN, Michel, F-31270 Villeneuve Tolosane (FR); COURTEMANCHE, Gilles, F-31120 Lacroix-Falgarde (FR)
(74) Mandataire: Kugel, Dominique
(86) Numéro de dépôt international: PCT/FR2005/000889
(87) Numéro de publication internationale: WO 2005/103033

(56) Documents cités:
- WO-A-03/106452
- DE-B- 1 005 072
- US-A- 2 484 029
- US-B1- 6 258 812
- US-B1- 6 589 951
- MELVIN S. NEWMAN & CO: ""o-Carbonyl assisted alkaline hydrolyses of methyl benzoates" JOURNAL OF THE AMERICAN CHEMICAL SOCIETY, vol. 90, no. 16, 1968, pages 4410-4413, XP002341595 ohio,USA
- J.H.P.TYMAN & CO: ""The structure of 2-keto and 2-aldehydeobenzoic acids"" SPECTROCHIMICA ACTA, vol. 33A, 1977, pages 479-485, XP002341596 Uxbridge, UK
- D.D. WHEELER & CO: ""Reactions of phtalaldehydeic acid"" JOURNAL OF ORGANIC CHEMISTRY, vol. 22, no. 5, 1957, pages 547-556, XP002341597
- E.D. AMSTUTZ & CO: ""The synthesis of benzene derivatives structurally similar to penicillic acid"" JOURNAL OF THE AMERIACAN CHEMICAL SOCIETY, vol. 68, no. 3, 1946, pages 349-354, XP002341598
- GLENN E. ULLYOT & CO: ""Analgesics. II. A new synthesis of aminophtalidylalkanes"" JOURNAL OF THE AMERICAN CHEMICAL SOCIETY, vol. 70, no. 2, 1948, pages 542-545, XP002341599
- WYMAN R. VAUGHAN & CO: ""The preparation of some phtalazines and related substances"" JOURNAL OF THE AMERICAN CHEMICAL SOCIETY, vol. 68, no. 7, 1946, pages 1314-1316, XP002341600
- A. MUSTAFA & CO: ""Reactions with 1(2H)-phtalazinones, 4,5-dihydro-3(2H)-pyridazones and 3-pyrazolin-5-ones" TETRAHEDRON, vol. 20, 1964, pages 531-544, XP002341601
- M.Z.A.BADR & CO: ""Subtitution and ring closure reactions of phtalazine derivatives"" JOURNAL OF HETEROCYCLIC CHEMISTRY, vol. 21, 1984, pages 471-475, XP002341602
- JOHN H. M. HILL & CO: "Nucleophilic heteroaromatic substitution. II. Phtalazines" JOURNAL OF ORGANIC CHEMISTRY, vol. 36, no. 21, 1971, pages 3248-3251, XP002341603
- DATABASE CHEMCATS [Online] STN; catalog name: Otava Stock Chemicals 26 novembre 2003 (2003-11-26), XP002296136 Database accession no. 2002:775677
- DATABASE CHEMCATS [Online] STN; catalog name: Interchim Intermediates 9 juillet 2002 (2002-07-09), XP002296137 Database accession no. 2002:2783954
- DATABASE CHEMCATS [Online] STN; catalog name: Ambinter Screening Library 1 janvier 2004 (2004-01-01), XP002296138 Database accession no. 2003:902721

## Description

La présente invention se rapporte à des dérivés de la 1-amino-phtharazine, à leur préparation et à leur application en thérapeutique.

La recherche d'antagonistes du récepteur 1 de la MCH (Melanin-Concentrating Hormone), le récepteur MCH₁, suscite un intérêt de la part de nombreuses sociétés pharmaceutiques. Un certain nombre de demandes de brevet ont été déposées parmi lesquelles on peut citer WO01/21577 (Takeda), WO02/06245 (Synaptic), WO03/106452 (Millennium). Un certain nombre de publications sont parus parmi lesquelles Ma V.V. et al (Amgen) 224th Nat. Meeting ACS Boston. Poster MEDI 343 (21.08.2002).

Durant ces dix dernières années, il a été démontré que de nombreux neuropeptides sont impliqués dans les régulations centrales régissant le comportement alimentaire ainsi que la balance énergétique (Inui et al, TINS 1999 ; 22(2) : 62-67). La MCH fait partie de ces neuropeptides.

Deux récepteurs de la MCH ont été clonés récemment, le récepteur MCH₁ préalablement appelé récepteur SLC-1 ou GPR24 (Chambers et al, Nature 1999 ; 400 : 261-265), et le récepteur MCH₂ préalablement appelé SLT (Mori et al, Biochem Biophys Res Commun 2001 ; 283 : 1013-1018).

Il existe donc un réel intérêt à trouver de nouveaux composés permettant de moduler l'activité du récepteur MCH₁, le récepteur 1 de la MCH.

Il a maintenant été trouvé que des composés, dérivés de la 1-amino-phtalazine, sont très affins et sélectifs vis-à-vis du récepteur MCH₁.

La présente invention a pour objet des composés répondant à la formule (I) 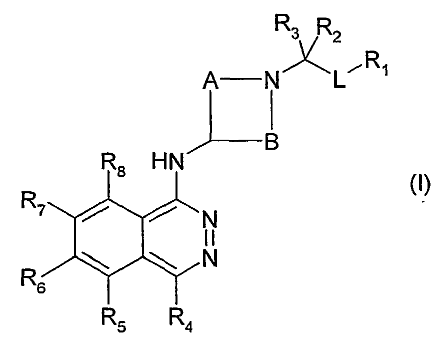
- A représente un groupe C₁₋₄-alkylène éventuellement substitué par un ou plusieurs groupes R₉ identiques ou différents l'un de l'autre ;
- B représente un groupe C₁₋₄-alkylène éventuellement substitué par un ou plusieurs groupes R₁₀ identiques ou différents l'un de l'autre ;
- R₉ et R₁₀ représentent chacun, indépendamment l'un de l'autre, un atome d'hydrogène ou un groupe C₁₋₅-alkyle,
   - ou bien R₉ et R₁₀ forment ensemble une liaison simple ou un groupe C₁₋₄-alkylène ;
- L représente une liaison simple ou un groupe C₁₋₂-alkylène, -CH=CH- ou -C≡C- ; les groupes C₁₋₂-alkylène et -CH=CH- étant éventuellement substitués par un ou plusieurs substituants C₁₋₂-alkyle ; ou bien L représente un groupe cycloprop-1,2-diyle ;
- R₁ représente un aryle ou un hétéroaryle ; les groupes aryle et hétéroaryle étant éventuellement substitués par un ou plusieurs radicaux Z identiques ou différents l'un de l'autre ;
- R₂ et R₃ représentent indépendamment l'un de l'autre, un atome d'hydrogène ou un groupe C₁₋₃-alkyle ou C₁₋₃-fluoroalkyle,
   . ou bien R₂ et R₃ forment ensemble, avec l'atome de carbone qui les porte, un groupe cycloprop-1,1-diyle ;
- R₄ représente un atome d'hydrogène ou un groupe C₁₋₅-alkyle, C₁₋₃-fluoroalkyle, C₃₋₆-cycloalkyle, C₃₋₆-cycloalkyle-C₁₋₃-alkylène, C₁₋₃-alkyle-O-C₁₋₃-alkylène, HO-C₁₋₃-alkylène, C₁₋₃-alkyle-X-C₁₋₃-alkylène où X représente S, SO ou SO₂,
   . ou bien R₄ représente un groupe RₐR_{b}N-C₁₋₃-alkylène, aryle, aryle-C₁₋₃-alkylène, aryle-O-, aryle-O-C₁₋₃-alkylène, aryle-C₁₋₃-alkylène-O-C₁₋₃-alkylène, hétéroaryle ou hétéroaryle-C₁₋₃-alkylène; les groupes aryle, aryle-C₁₋₃-alkylène, aryle-O-, aryle-O-C₁₋₃-alkylène, hétéroaryle et hétéroaryle-C₁₋₃-alkylène étant éventuellement substitués par un ou plusieurs radicaux Z identiques ou différents l'un de l'autre ;
- R₅ représente un atome d'hydrogène ou d'halogène ou un groupe C₁₋₅-alkyle, C₁₋₃-fluoroalkyle, C₁₋₅-alcoxy, C₁₋₃-fluoroalcoxy, HO-C₁₋₃-alkylène, -CN, C₁₋₃-alkyle-X- où X représente S, SO ou SO₂,
   . ou bien R₅ représente un groupe RₐR_{b}N-, RₐR_{b}N-C₁₋₃-alkylène, aryle, aryle-C₁₋₃-alkylène, aryle-O- ou hétéroaryle ; les groupes aryle, aryle-C₁₋₃-alkylène, aryle-O- et hétéroaryle étant éventuellement substitués par un ou plusieurs radicaux Z identiques ou différents l'un de l'autre ;
- R₆ représente un atome d'hydrogène ou d'halogène ou un groupe C₁₋₅-alkyle, C₁₋₃-fluoroalkyle, C₁₋₅-alcoxy, C₁₋₃-fluoroalcoxy, -CN, RₐR_{b}N-, RₐR_{b}N-C₁₋₃-alkylène, aryle ou hétéroaryle ; les groupes aryle et hétéroaryle étant éventuellement substitués par un ou plusieurs radicaux Z identiques ou différents l'un de l'autre ;
- R₇ représente un atome d'hydrogène ou d'halogène ou un groupe C₁₋₅-alkyle, C₁₋₃-fluoroalkyle, C₁₋₅-alcoxy, C₁₋₃-fluoroalcoxy, HO-C₁₋₃-alkylène, -CN, C₁₋₃-alkyle-X- où X représente S, SO ou SO2,
   . ou bien R₇ représente un groupe RₐR_{b}N-, RₐR_{b}N-C₁₋₃-alkylène, RₐR_{b}NC(O)-, C₁₋₃-alkyle-C(O)-, aryle, aryle-O- ou hétéroaryle ; les groupes aryle, aryle-O- et hétéroaryle étant éventuellement substitués par un ou plusieurs radicaux Z identiques ou différents l'un de l'autre ;
- R₈ représente un atome d'hydrogène ou d'halogène ou un groupe C₁₋₅-alkyle, C₁₋₅-alcoxy, C₁₋₃-fluoroalcoxy ;
- Z représente un atome d'hydrogène ou d'halogène ou un groupe C₁₋₅-alkyle, C₁₋₃-fluoroalkyle, C₃₋₆-cycloalkyle, C₃₋₆-cycloalkyle-C₁₋₃-alkylène, un phényle, C₁₋₅-alcoxy, C₁₋₃-fluoroalcoxy, C₁₋₃-alkyle-O-C₁₋₃-alkylène, HO-C₁₋₃-alkylène, NO₂, -CN, C₁₋₃-alkyle-X-, C₁₋₃-alkyle-X-C₁₋₃-alkylène où X représente S, SO ou SO₂ ;
   . ou bien Z représente un groupe RₐR_{b}N-, RₐR_{b}N-C₁₋₃-alkylène, RₐR_{b}NC(O)-, C₁₋₃-alkyle-C(O)-, C₁₋₄-alkyle-CO₂-, C₃₋₆-cycloalkyle-C(O)- ;
   . ou bien Z représente un radical oxo ;
   . ou bien deux radicaux Z adjacents forment ensemble un groupe C₁₋₃-alkylènedioxy ;
- Rₐ et R_{b} représentent chacun, indépendamment de l'autre, un atome d'hydrogène ou un groupe C₁₋₃-alkyle ou C₁₋₃-alkyle-C(O)- ;
   . ou bien Rₐ et R_{b} forment ensemble, avec l'atome d'azote qui les porte, un hétérocycle éventuellement substitué par un ou plusieurs groupes C₁₋₃-alkyle ou oxo ; étant entendu que lorsque R₂, R₃, R₅, R₆, R₇ et R₈ représentent tous un atome d'hydrogène, A et B représentent tous les deux un groupe éthylènyle (-CH₂CH₂-) et L est une liaison simple, R₁ et R₄ ne peuvent pas représenter tous les deux un groupe phényle non substitué.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent comporter un ou plusieurs cycles. Ils peuvent donc exister sous forme d'isomères axial/équatorial, ou endo/exo, ou cis/trans. Ces isomères ainsi que leurs mélanges, font partie de l'invention.

Les composés de formule (I) peuvent comporter une ou plusieurs fonctions oléfiniques. Ils peuvent donc exister sous forme d'isomères Z/E. Ces isomères ainsi que leurs mélanges, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates et/ou de solvats, à savoir sous forme d'associations ou de combinaisons avec une ou plusieurs molécules d'eau et/ou de solvant. De tels hydrates et solvats font également partie de l'invention.

Dans le cadre de la présente invention, on entend par :
- C_{t-z} où t et z peuvent prendre les valeurs de 1 à 6, une chaîne ou un cycle carboné pouvant avoir de t à z atomes de carbone, par exemple C₁₋₃ peut caractériser une chaîne carbonée ayant de 1 à 3 atomes de carbone;
- un atome d'halogène : un fluor, un chlore, un brome ou un iode ;
- un groupe alkyle : un groupe aliphatique monovalent saturé, linéaire ou ramifié. A titre d'exemples, on peut citer les groupes méthyle, éthyle, propyle, isopropyle, butyle, isobutyle, tertbutyle, pentyle, etc ;
- un groupe alkylène : un groupe aliphatique divalent saturé, linéaire ou ramifié. A titre d'exemple un groupe C₁₋₃-alkylène représente une chaîne carbonée divalente de 1 à 3 atomes de carbone, linéaire ou ramifiée, telle que un méthylènyle (-CH₂-), un éthylènyle (-CH₂CH₂-), un 1-méthyléthylènyle (-CH(CH₃)CH₂-), un propylènyle (-CH₂CH₂CH₂-), etc ;
- un groupe cycloalkyle : un groupe aliphatique cyclique saturé. A titre d'exemples, on peut citer les groupes cyclopropyle, méthylcyclopropyle, cyclobutyle, cyclopentyle, cyclohexyle, etc ;
- un groupe alcoxy : un radical alkyle-O- où le groupe alkyle est tel que précédemment défini ;
- un groupe alkylènedioxy : un groupe -O-alkylène-O-, où le groupe alkylène est tel que précédemment défini. A titre d'exemples, on peut citer les groupes méthylènedioxy, éthylènedioxy ou propylènedioxy ;
- un groupe fluoroalkyle : un groupe alkyle dont un ou plusieurs atomes d'hydrogène ont été substitués par un atome de fluor. A titre d'exemples, on peut citer les groupes CF₃-, CF₃CH₂-, etc ;
- un groupe fluoroalcoxy : un groupe alcoxy dont un ou plusieurs atomes d'hydrogène ont été substitués par un atome de fluor. A titre d'exemples, on peut citer les groupes CF₃O-, CHF₂O-, etc ;
- un groupe hétérocycle : un groupe cyclique saturé de 5 à 7 chaînons comportant un à plusieurs hétéroatomes tels que les atomes d'azote, d'oxygène ou de soufre. A titre d'exemples, on peut citer les groupes pyrrolidinyle, pipéridinyle, pipéridonyle, morpholinyle, pipérazinyle, N-méthyl-pipérazinyle, etc ;
- un groupe aryle : système aromatique monocyclique ou polycyclique comprenant de 6 à 14 atomes de carbone, de préférence de 6 à 10 atomes de carbone. Lorsque le système est polycyclique, au moins un des cycles est aromatique. A titre d'exemples, on peut citer les groupes phényle, naphtyle, tétrahydronaphtyle, indanyle, etc ;
- un groupe hétéroaryle : système aromatique monocyclique ou polycyclique comprenant de 5 à 14 chaînons, de préférence de 5 à 10 chaînons et comprenant un à plusieurs hétéroatomes tels que les atomes d'azote, d'oxygène ou de soufre. Lorsque le système est polycyclique, au moins un des cycles est aromatique. Les atomes d'azote peuvent être sous forme de N-oxydes. A titre d'exemples de groupes hétéroaryles monocycliques, on peut citer les groupes thiazolyle, thiadiazolyle, thiényle, imidazolyle, triazolyle, tétrazolyle, pyridyle, furanyle, oxazolyle, isoxazolyle, oxadiazolyle, pyrrolyle, pyrazolyle, pyrimidinyle, pyridazinyle. A titre d'exemples de groupes hétéroaryles bicycliques, on peut citer les groupes indolyle, benzofuranyle, chromèn-2-on-yle, benzimidazolyle, benzothiényle, benzotriazolyle, benzothiazolyle, benzoxazolyle, quinolinyle, isoquinolinyle, indazolyle, indolizinyle, quinazolinyle, phthalazinyle, quinoxalinyle, naphtyridinyle, 2,3-dihydro-1H-indolyle, 2,3-dihydro-benzofuranyle, tétrahydroquinolinyle, tétrahydroisoquinolinyle.

Dans la formule générale (I), le cycle: 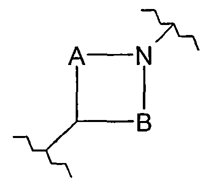 peut représenter par exemple un groupe azétidinyle, pyrrolidinyle, pipéridinyle, azépanyle, 8-azabicyclo[3.2.1]octyle, 8-azabicyclo[3.3.1]nonanyle, décahydroisoquinolinyle, 3-azabicyclo[3.1.0]hexanyle.

Parmi les composé de formule (I) objets de l'invention, on peut citer un premier sous-groupe de composés qui se définissent comme suit :
- A représente un groupe C₁₋₄-alkylène éventuellement substitué par un ou plusieurs groupes R₉ identiques ou différents l'un de l'autre ;
- B représente un groupe C₁₋₄-alkylène éventuellement substitué par un ou plusieurs groupes R₁₀ identiques ou différents l'un de l'autre ;
- R₉ et R₁₀ représentent chacun un atome d'hydrogène, ou bien R₉ et R₁₀ forment ensemble un groupe C₁₋₄-alkylène ;
- L représente une liaison simple ou -CH=CH- ;
- R₁ représente un aryle ou un hétéroaryle ; les groupes aryle et hétéroaryle étant éventuellement substitués par un ou plusieurs radicaux Z identiques ou différents l'un de l'autre ;
- R₂ et R₃ représentent indépendamment l'un de l'autre un atome d'hydrogène ou un groupe C₁₋₃-alkyle ;
- R₄ représente un atome d'hydrogène ou un groupe C₁₋₅-alkyle, C₃₋₆-cycloalkyle, C₃₋₆₋cycloalkyle-C₁₋₃-alkylène, C₁₋₃-alkyle-O-C₁₋₃-alkylène, aryle, aryle-C₁₋₃-alkylène, aryle-C₁₋₃-alkylène-O-C₁₋₃-alkylène, hétéroaryle ou hétéroaryle-C₁₋₃-alkylène ; les groupes aryle, aryle-C₁₋₃-alkylène, hétéroaryle et hétéroaryle-C₁₋₃-alkylène étant éventuellement substitués par un ou plusieurs radicaux Z identiques ou différents l'un de l'autre ;
- R₅ représente un atome d'hydrogène ou un groupe C₁₋₅-alkyle, C₁₋₅-alcoxy ou aryle ; le groupe aryle étant éventuellement substitué par un ou plusieurs radicaux Z identiques ou différents l'un de l'autre ;
- R₆ représente un atome d'hydrogène ;
- R₇ représente un atome d'halogène ou un groupe C₁₋₅-alkyle et plus particulièrement un méthyle, C₁₋₅-alcoxy et plus particulièrement un méthoxy ou un aryle;
- R₈ représente un atome d'hydrogène ;
- Z représente un atome d'hydrogène ou d'halogène ou un groupe C₁₋₅-alkyle, C₁₋₃-fluoroalkyle, C₃₋₆-cycloalkyle, C₃₋₆-cycloalkyle-C₁₋₃-alkylène, un phényle, C₁₋₅-alcoxy, C₁₋₃-fluoroalcoxy, C₁₋₃-alkyle-O-C₁₋₃-alkylène, HO-C₁₋₃-alkylène, NO₂, -CN, C₁₋₃-alkyle-X-, C₁₋₃-alkyle-X-C₁₋₃-alkylène où X représente S, SO ou SO₂ ;
   - ou bien Z représente un groupe RₐR_{b}N-, RₐR_{b}N-C₁₋₃-alkylène, RₐR_{b}NC(O)-, C₁₋₃-alkyle-C(O)-, C₃₋₆-cycloalkyle-C(O)- ;
   - ou bien Z représente un radical oxo ;
   - ou bien deux radicaux Z adjacents forment ensemble un groupe C₁₋₃-alkylènedioxy ;
- Rₐ et R_{b} représentent chacun, indépendamment de l'autre, un atome d'hydrogène ou un groupe C₁₋₃-alkyle ou C₁₋₃-alkyle-C(O)- ;
   . ou bien Rₐ et R_{b} forment ensemble, avec l'atome d'azote qui les porte, un hétérocycle éventuellement substitué par un ou plusieurs groupes C₁₋₃-alkyle ou oxo.

Parmi les composé de formule (I) objets de l'invention, on peut citer un second sous-groupe de composés qui se définissent comme suit :
- A représente un groupe C₁₋₄-alkylène éventuellement substitué par un groupe R₉ ;
- B représente un groupe C₁₋₄-alkylène éventuellement substitué par un groupe R₁₀ ;
- R₉ et R₁₀ représentent chacun un atome d'hydrogène ou R₉ et R₁₀ forment ensemble un groupe C₁₋₄-alkylène ;
- L représente une liaison simple ou -CH=CH- ;
- R₁ représente un aryle éventuellement substitué par un ou plusieurs radicaux Z identiques ou différents l'un de l'autre ; .
- R₂ et R₃ représentent indépendamment l'un de l'autre, un atome d'hydrogène ou un groupe C₁₋₃-alkyle ;
- R₄ représente un atome d'hydrogène ou un groupe C₁₋₅-alkyle, C₃₋₆-cycloalkyle, C₃₋ₐ-cycloalkyle-C₁₋₃-alkylène, C₁₋₃-alkyle-O-C₁₋₃-alkylène, aryle et plus particulièrement un phényle, ou hétéroaryle et plus particulièrement un thiényle ou un pyridinyle ; les groupes aryle et hétéroaryle étant éventuellement substitués par un ou plusieurs radicaux Z identiques ou différents l'un de l'autre ;
- R₅ représente un atome d'hydrogène ;
- R₆ représente un atome d'hydrogène ;
- R₇ représente un atome d'halogène, un méthyle ou un méthoxy ;
- R₈ représente un atome d'hydrogène ;
- Z représente un atome d'hydrogène, un atome d'halogène, un groupe C₁₋₅-alkyle, C₁₋₅-alcoxy et plus particulièrement méthoxy, C₁₋₃-alkyle-O-C₁₋₃-alkylène, un phényle, HO-C₁₋₃-alkylène, -CN, C₁₋₃-alkyle-X- où X représente un atome de soufre ;
   . ou bien Z représente un groupe RₐR_{b}N-, RₐR_{b}N-C₁₋₃-alkylène, RₐR_{b}NC(O)-, C₁₋₃-alkyle-C(O)- ;
   . ou bien Z représente un radical oxo ;
   . ou bien deux radicaux Z adjacents forment ensemble un groupe C₁₋₃-alkylènedioxy, plus particulièrement un méthylènedioxy ;
- Rₐ et R_{b} représentent chacun, indépendamment de l'autre, un atome d'hydrogène ou un groupe C₁₋₃-alkyle ou C₁₋₃-alkyle-C(O)-.

Dans le cadre de la présente invention, conviennent plus particulièrement les composés de formule (I) dans lesquels l'un au moins des substituants R₅, R₆, R₇ ou R₈ est différent d'un atome d'hydrogène.

Parmi les composés de formule (I) objets de l'invention, on peut notamment citer :
- 7-méthoxy-4-(4-méthoxyphényl)-N-[8-(2-naphthylméthyl)-8-azabicyclo[3.2.1]oct-3-yl]phthalazin-1-amine (composé n° 4) ;
- 7-méthoxy-4-(4-méthoxyphényl)-N-[1-(2-naphthylméthyl)pipéridin-4-yl]phthalazin-1-amine (composé n° 3) ;
- 7-méthoxy-4-(3-méthoxyphényl)-N-[1-(2-naphthylméthyl)pipéridin-4-yl]phthalazin-1-amine (composé n° 5) ;
- 7-méthoxy-4-(2-méthoxyphényl)-N-[1-(2-naphthylméthyl)pipéridin-4-yl]phthalazin-1-amine (composé n° 2) ;
- 7-méthoxy-N-[1-(2-naphthylméthyl)pipéridin-4-yl]-4-phénylphthalazin-1-amine (composé n° 1) ;
- 4-(4-méthoxyphényl)-7-méthyl-N-[1-(2-naphthylméthyl)pipéridin-4-yl]phthalazin-1-amine (composé n° 9) ;
- 4-(3,4-diméthoxyphényl)-7-méthoxy-N-[1-(2-naphthylméthyl)pipéridin-4-yl]phthalazin-1-amine (composé n° 10) ;
- Dichlorhydrate de N-[1-(1,3-benzodioxol-5-ylméthyl)pipéridin-4-yl]-7-méthoxy-4-(4-méthoxyphényl)phthalazin-1-amine (composé n° 19) ;
- 4-éthyl-7-méthoxy-N-[1-(2-naphthylméthyl)pipéridin-4-yl]phthalazin-1-amine (composé n° 20) ;
- 4-benzyl-7-méthoxy-N-[1-(2-naphthylméthyl)pipéridin-4-yl]phthalazin-1-amine (composé n° 21) ;
- Chlorhydrate de 7-méthoxy-4-(méthoxyméthyl)-N-[1-(2-naphthylméthyl)pipéridin-4-yl]phthalazin-1-amine (composé n° 24) ;
- N-[1-(1-benzofuran-5-ylméthyl)pipéridin-4-yl]-7-méthoxy-4-(4méthoxyphényl)phthalazin-1-amine (composé n° 26) ;
- N-[1-(3,4-diméthylbenzyl)pipéridin-4-yl]-7-méthoxy-4-(4-méthoxyphényl)phthalazin-1-amine (composé n° 29) ;
- 7-méthoxy-4-(4-méthoxyphényl)-N-{1-[(1-méthyl-1H-indol-2-yl)méthyl]pipéridin-4-yl}phthalazin-1-amine (composé n° 31) ;
- 4-cyclopropyl-7-méthoxy-N-[1-(2-naphthylméthyl)pipéridin-4-yl]phthalazin-1-amine (composé n° 33) ;
- N-[1-(3-fluoro-4-méthoxybenzyl)pipéridin-4-yl]-7-méthoxy-4-(4-méthoxyphényl)phthalazin-1-amine (composé n° 39) ;
- N-[1-(2,3-dihydro-1,4-benzodioxin-6-ylméthyl)pipéridin-4-yl]-7-méthoxy-4-(4-méthoxyphényl)phthalazin-1-amine (composé n° 40) ;
- Dichlorhydrate de 4-(1,3-benzodioxol-5-yl)-7-méthoxy-N-[1-(2-naphthylméthyl)pipéridin-4-yl]phthalazin-1-amine (composé n° 42) ;
- 4-(4-chlorophényl)-7-méthoxy-N-[1-(2-naphthylméthyl)pipéridin-4-yl]phthalazin-1-amine (composé n° 43) ;
- 7-méthoxy-N-[1-(2-naphthylméthyl)pipéridin-4-yl]-4-[4-(trifluorométhyl)phényl] phthalazin-1-amine (composé n° 45) ;
- N-[1-(1-benzothien-2-ylméthyl)pipéridin-4-yl]-7-méthoxy-4-(4-méthoxyphényl) phthalazin-1-amine (composé n° 48) ;
- Dichlorhydrate de 4-cyclopentyl-7-méthoxy-N-[1-(2-naphthylméthyl)pipéridin-4-yl]phthalazin-1-amine (composé n° 51) ;
- N-[1-(2,3-dihydro-1-benzofuran-5-ylméthyl)pipéridin-4-yl]-7-méthoxy-4-(4-méthoxyphényl)phthalazin-1-amine (composé n° 54) ;
- N-[1-(2,3-dihydro-1H-indèn-5-ylméthyl)pipéridin-4-yl]-7-méthoxy-4-(4-méthoxyphényl)phthalazin-1-amine (composé n° 55) ;
- Dichlorhydrate de 7-méthoxy-4-(4-méthoxyphényl)-N-{1-[1-(2-naphthyl)éthyl]pipéridin-4-yl}phthalazin-1-amine (composé n° 56) ;
- N-[1-(1,3-benzothiazol-6-ylméthyl)pipéridin-4-yl]-7-méthoxy-4-(4-méthoxyphényl)phthalazin-1-amine (composé n° 59) ;
- N-[1-(1-benzothièn-5-ylméthyl)pipéridin-4-yl]-7-méthoxy-4-(4-méthoxyphényl)phthalazin-1-amine (composé n° 60) ;
- N-[1-(1H-indol-3-ylméthyl)pipéridin-4-yl]-7-méthoxy-4-(4-méthoxyphényl)phthalazin-1-amine (composé n° 65) ;
- 7-méthoxy-4-(4-méthylphényl)-N-[1-(2-naphthylméthyl)pipéridin-4-yl]phthalazin-1-amine (composé n° 66) ;
- 4-butyl-7-méthoxy-N-[1-(2-naphthylméthyl)pipéridin-4-yl]phthalazin-1-amine (composé n° 67) ;
- Dichlorhydrate de 7-méthoxy-4-(4-méthoxyphényl)-N-{1-[(2E)-3-phénylprop-2-èn-1-yl]pipéridin-4-yl}phthalazin-1-amine (composé n° 69) ;
- 7-méthoxy-N-[1-(2-naphthylméthyl)pipéridin-4-yl]phthalazin-1-amine (composé n° 70) ;
- 4-(cyclopropylméthyl)-7-méthoxy-N-[1-(2-naphthylméthyl)pipéridin-4-yl]phthalazin-1-amine (composé n° 71) ;
- N-[1-(3-fluoro-4-méthylbenzyl)pipéridin-4-yl]-7-méthoxy-4-(4-méthoxyphényl)phthalazin-1-amine (composé n° 74) ;
- 7-méthoxy-4-(4-méthoxyphényl)-N-[1-(4-méthylbenzyl)pipéridin-4-yl]phthalazin-1-amine (composé n° 75) ;
- 4-(4-fluorophényl)-7-méthoxy-N-[1-(2-naphthylméthyl)pipéridin-4-yl]phthalazin-1-amine (composé n° 82) ;
- N-[1-(1H-indol-5-ylméthyl)pipéridin-4-yl]-7-méthoxy-4-(4-méthoxyphényl)phthalazin-1-amine (composé n° 87) ;
- 7-méthoxy-4-[4-(méthoxyméthyl)phényl]-N-[1-(2-naphthylméthyl)pipéridin-4-yl]phthalazin-1-amine (composé n° 88) ;
- 7-méthoxy-N-[1-(4-méthoxy-3-méthylbenzyl)pipéridin-4-yl]-4-(4-méthoxyphényl)phthalazin-1-amine (composé n° 89) ;
- 7-méthoxy-4-méthyl-N-[1-(2-naphthylméthyl)pipéridin-4-yl]phthalazin-1-amine (composé n° 95) ;
- Dichlorhydrate de 4-(2,5-diméthoxyphényl)-7-méthoxy-N-[1-(2-naphthylméthyl) pipéridin-4-yl]phthalazin-1-amine (composé n° 97) ;
- 7-méthoxy-4-(4-méthoxyphényl)-N-[1-(quinolin-6-ylméthyl)pipéridin-4-yl]phthalazin-1-amine (composé n° 99) ;
- 7-méthoxy-4-(4-méthoxyphényl)-N-{1-[(1-méthyl-1H-indol-5-yl)méthyl]pipéridin-4-yl}phthalazin-1-amine (composé n° 100) ;
- Dichlorhydrate de 4-(2,4-diméthoxyphényl)-7-méthoxy-N-[1-(2-naphthylméthyl) pipéridin-4-yl]phthalazin-1-amine (composé n° 102) ;
- Dichlorhydrate de 4-(3,5-diméthoxyphényl)-7-méthoxy-N-[1-(2-naphthylméthyl) pipéridin-4-yl]phthalazin-1-amine (composé n° 103) ;
- Dichlorhydrate de 7-méthoxy-N-[1-(2-naphthylméthyl)pipéridin-4-yl]-4-(2-phényléthyl)phthalazin-1-amine (composé n° 104) ;
- Trichlorhydrate de 4-{4-[(diéthylamino)méthyl]phényl}-7-méthoxy-N-[1-(2-naphthylméthyl)pipéridin-4-yl]phthalazin-1-amine (composé n° 105) ;
- 7-fluoro-4-(4-fluorophényl)-N-[1-(2-naphthylméthyl)pipéridin-4-yl]phthalazin-1-amine (composé n° 106) ;
- Trichlorhydrate de 7-méthoxy-N-[1-(2-naphthylméthyl)pipéridin-4-yl]-4-pyridin-3-ylphthalazin-1-amine (composé n° 109) ;
- Dichlorhydrate de N-[1-(4-chlorobenzyl)pipéridin-4-yl]-7-méthoxy-4-(4-méthoxyphényl)phthalazin-1-amine (composé n° 110) ;
- Dichlorhydrate de {4-[(4-{[7-méthoxy-4-(4-méthoxyphényl)phthalazin-1-yl]amino}pipéridin-1-yl)méthyl]phényl}méthanol (composé n° 112) ;
- Trichlorhydrate de N-{1-[4-(aminométhyl)benzyl]pipéridin-4-yl}-7-méthoxy-4-(4-méthoxyphényl)phthalazin-1-amine (composé n° 113) ;
- Dichlorhydrate de N-{4-[(4-{[7-méthoxy-4-(4-méthoxyphényl)phthalazin-1-yl]amino}pipéridin-1-yl)méthyl]phényl}acétamide (composé n° 114) ;
- Dichlorhydrate de 1-{4-[(4-{[7-méthoxy-4-(4-méthoxyphényl)phthalazin-1-yl]amino}pipéridin-1-yl)méthyl]phényl}éthanone (composé n° 121) ;
- Dichlorhydrate de N-[1-(1-benzofuran-5-ylméthyl)pipéridin-4-yl]-4-(2,5-diméthoxyphényl)-7-méthoxyphthalazin-1-amine (composé n° 124) ;
- Dichlorhydrate de N-[1-(1,3-benzodioxol-5-ylméthyl)pipéridin-4-yl]-4-(2,5-diméthoxyphényl)-7-méthoxyphthalazin-1-amine (composé n° 125) ;
- Dichlorhydrate de 6-[(4-{[7-méthoxy-4-(4-méthoxyphényl)phthalazin-1-yl]amino}pipéridin-1-yl)méthyl]-2H-chromèn-2-one (composé n° 127) ;
- N-[1-(1H-indol-6-ylméthyl)pipéridin-4-yl]-7-méthoxy-4-(4-méthoxyphenyl)phthalazin-1-amine (composé n° 129) ;
- Dichlorhydrate de 7-méthoxy-N-[1-(4-méthoxybenzyl)pipéridin-4-yl]-4-(4-méthoxyphényl)phthalazin-1-amine (composé n° 130) ;
- Dichlorhydrate de N-[1-(4-éthylbenzyl)pipéridin-4-yl]-7-méthoxy-4-(4-méthoxyphényl)phthalazin-1-amine (composé n° 132) ;
- N-[1-(1,3-benzothiazol-6-ylméthyl)pipéridin-4-yl]-4-(2,5-diméthoxyphényl)-7-méthoxyphthalazin-1-amine (composé n° 135) ;
- Dichlorhydrate de 7-méthoxy-N-[1-(2-naphthylméthyl)pipéridin-4-yl]-4-(phénoxyméthyl)phthalazin-1-amine (composé n° 136) ;
- N-[1-(1H-benzimidazol-5-ylméthyl)pipéridin-4-yl]-7-méthoxy-4-(4-méthoxyphényl)phthalazin-1-amine (composé n° 138) ;
- Dichlorhydrate de 7-chloro-4-(4-méthoxyphényl)-N-[1-(2-naphthylméthyl)pipéridin-4-yl]phthalazin-1-amine (composé n° 143) ;
- Dichlorhydrate de 4-(4-bromophényl)-7-méthoxy-N-[1-(2-naphthylméthyl)pipéridin-4-yl]phthalazin-1-amine (composé n° 145) ;
- Dichlorhydrate de N-[1-(1,3-benzodioxol-5-ylmethyl)pipéridin-4-yl]-7-méthoxy-4-(méthoxyméthyl)phthalazin-1-amine (composé n° 146) ;
- Dichlorhydrate de 4-[(benzyloxy)méthyl]-7-méthoxy-N-[1-(2-naphthylméthyl)pipéridin-4-yl]phthalazin-1-amine (composé n° 147) ;
- Dichlorhydrate de 7-méthoxy-5-phényl-N-[1-(2-naphthylméthyl)pipéridin-4-yl]phthalazin-1-amine (composé n° 17) ;
- 4-(hydroxyméthyl)-7-méthoxy-N-[1-(2-naphthylméthyl)pipéridin-4-yl]-4-(phénoxyméthyl)phthalazin-1-amine (composé n° 148) ;
- (-)7-méthoxy-4-(4-méthoxyphényl)-N-{1-[1-(2-naphthyl)éthyl]pipéridin-4-yl}phthalazin-1-amine (composé n° 149) ;
- (+)7-méthoxy-4-(4-méthoxyphényl)-N-{1-[1-(2-naphthyl)éthyl]pipéridin-4-yl}phthalazin-1-amine (composé n° 150) ;
- Trichlorhydrate de N-{1-[4-(diméthylaminométhyl)benzyl]pipéridin-4-yl}-7-méthoxy-4-(4-méthoxyphényl)phthalazin-1-amine (composé n° 151).

Conformément à l'invention, on peut préparer les composés de formule générale (I) selon le procédé illustré par le schéma 1 qui suit.

Dans les schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Selon le schéma 1, le composé de formule générale (I) est préparé à partir d'un composé de formule générale (III), dans laquelle R₄, R₅, R₆, R₇ et R₈ sont tels que définis dans la formule générale (I), selon la voie A, par substitution nucléophile du chlore par l'amine du composé de formule générale (II), dans laquelle R₁, R₂, R₃, L, A et B sont tels que définis dans la formule générale (I). Cette réaction peut être réalisée par chauffage des composés de formules générales (II) et (III) dans un alcool tel que le n-butanol en présence de chlorure d'ammonium selon la méthode décrite par Contreras et al (J. Med. Chem. 2001, 44, 2707-2718), ou bien peut être catalysée par un métal de transition tel que le palladium par exemple sous la forme tris(dibenzylidène-acétone)-dipalladium en présence d'un ligand tel que le BINAP (2,2'-bis(diphénylphosphino)-1,1'-binaphtyle) et d'une base telle que le tert-butylate de sodium selon la méthode décrite par Wolfe et al (J.A.C.S., 1996, 118, 7215-7216).

Dans le cas où R₃ est un atome d'hydrogène, le composé de formule générale (I) peut aussi être préparé, selon la voie B, à partir du composé de formule générale (Ib), dans laquelle A, B, R₄, R₅, R₆, R₇ et R₈ sont tels que définis dans la formule générale (I) et dans laquelle l'azote du cycle azoté n'est pas substitué, notamment par une réaction d'amination réductrice avec un aldéhyde ou une cétone de formule générale R₁-L-C(O)R₂, dans laquelle L, R₁ et R₂ sont tels que définis dans la formule générale (1). Cette réaction peut être par exemple effectuée en présence de triacétoxy-borohydrure de sodium dans un solvant comme le dichlorométhane ou le 1,2-dichloroéthane selon une des méthodes décrites dans Abdel-Magid et al (J. Org. Chem. 1996, 61, 3849-3862). Le composé de formule générale (Ib) est obtenu par déprotection du composé de formule générale (Ia) qui possède sur l'azote du cycle azoté un groupement protecteur PG. Ce groupement protecteur peut être par exemple un benzyle ou un tert-butoxycarbonyle et la déprotection peut être effectuée selon les méthodes citées dans Protective Groups in Organic Synthesis 3eme édition John Wiley & Sons, New York 1999. Le composé de formule générale (Ia) est synthétisé en faisant réagir un composé de formule générale (III) tel que défini ci-dessus avec un composé de formule générale (IIa) selon les procédés déjà décrits précédemment pour le composé (I).

Le composé de formule générale (IIa), lorsqu'il n'est pas commercial peut être préparé par analogie aux méthodes décrites dans la littérature (Mach et al, J. Med. Chem. 1993, 36, 3707-3720, Dostert et al, Eur. J. Méd. Chem. Ther. 1984, 19(2), 105-110).

L'amine de formule (II) lorsqu'elle n'est pas commerciale peut être préparée par analogie aux méthodes précédemment citée pour (IIa) ainsi qu'aux méthodes décrites dans les publications suivantes: Moragues et al (Farmaco. Ed. Sci. 1980, 35(11), 951-964) et Shum et al (Nucleosides Nucleotides 2001, 20(4-7), 1067-1078).

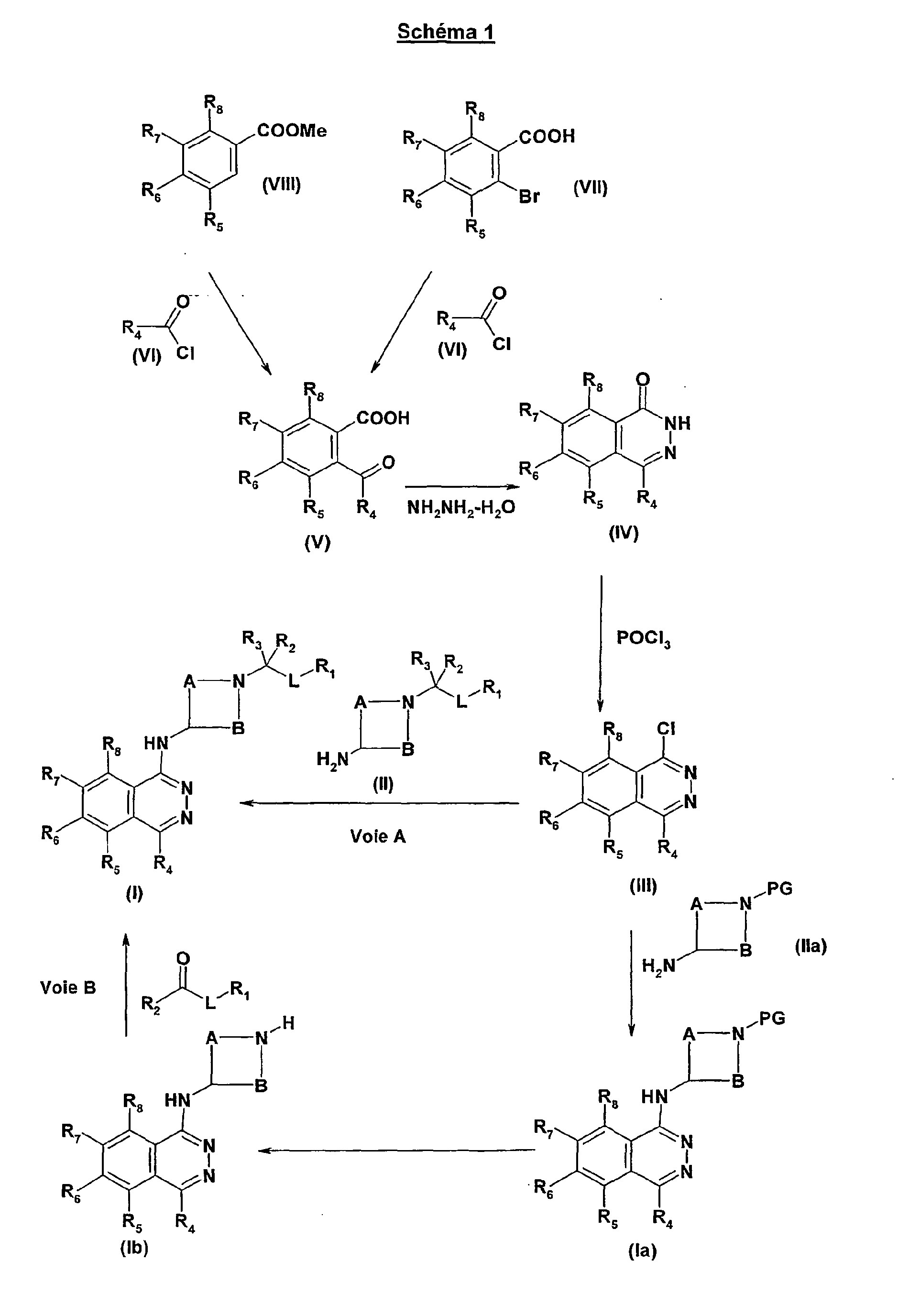

La 1-chlorophthalazine de formule générale (III) peut être obtenue par chauffage de la 2*H*-phthalazin-1-one de formule générale (IV) dans le chlorure de phosphoryle par exemple.

La 2*H*-phthlazin-1-one de formule générale (IV) peut être préparée à partir d'un acide de type 2-acyl-benzoïque de formule générale (V) par chauffage dans un alcool comme l'éthanol, en présence d'hydrate d'hydrazine par exemple.

L'acide de type 2-acyl-benzoïque de formule générale (V) peut être synthétisé à partir d'un acide de type 2-bromo-benzoïque de formule générale (VII) par échange halogène-métal puis réaction avec un chlorure d'acide de formule générale (VI) ou avec une amide de Weinreb, de type N-méthoxy, N-méthylamine de formule (VI') :

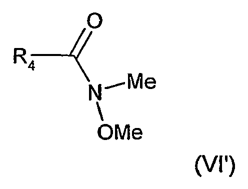

L'échange halogène-métal peut être réalisé par du n-butyllithium dans le tétrahydrofurane à -78°C. Dans le cas où R₄ représente un atome d'hydrogène, le chlorure d'acide de formule générale (Vl) est remplacé par du diméthylformamide. Les composés de formule (VI') peuvent être obtenus à partir du composé de formule (VI) et de N-méthoxy-N-méthylamine, notamment selon la méthode décrite par Weinreb (Tetrahedron Letters, (1981), 22(39) : 3815-3818).

L'acide de type 2-acyl-benzoïque de formule générale (V) peut être aussi obtenu par d'autres réactions comme par exemple la réaction de Friedel et Crafts sur un composé de formule générale (VIII) en présence d'un composé de formule générale (VI). Cette réaction est effectuée en présence de chlorure d'aluminium dans un solvant comme le dichlorométhane.

L'invention a également pour objet les composés de formules (V), (IV), et (III) qui suivent : 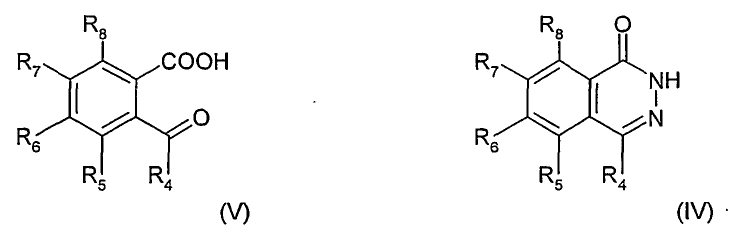 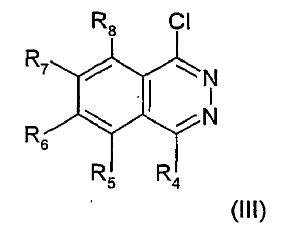 dans lesquelles R₄, R₅, R₆, R₇ et R₈ sont tels que précédemment définis pour le composés de formule (I).

Des exemples de préparation de composés de formule (V), (IV) et (III) sont donnés dans ce qui suit.

Les composés de formule (I) sont très affins et sélectifs vis-à-vis du récepteur 1 de la Melanin-Concentrating Hormone (MCH), MCH₁.

Des essais *in vitro* ont démontré l'affinité des composés pour les récepteurs MCH et en particulier MCH₁.

La MCH étant un important régulateur de la prise alimentaire, de petites molécules non peptidiques capables d'antagoniser son action stimulante du récepteur MCH₁ constituent une thérapie de choix pour traiter les problèmes métaboliques dus à l'obésité mais aussi à la boulimie. En effet, l'utilisation d'un antagoniste des récepteurs MCH₁ tel que le SNAP-7941 (décrit par les Laboratoires Synaptic) confirme le rôle important de la MCH dans la régulation de la balance énergétique et le développement de l'obésité (Katsuura et al, Curr Med Chem 2003 ; 3 : 217-227).

Les composés selon l'invention représentent donc une thérapie de choix pour le traitement de maladies présentant des troubles de la régulation de la balance énergétique ainsi que pour le traitement du développement de l'obésité.

La MCH est un antagoniste fonctionnel du système mélanocortine, contrecarrant les effets de celui-ci sur la prise alimentaire et sur l'axe hypothalamo-hypophyso-surrénalien (Ludwig et al, Am J Physiol 1998 ; 274 : E627-E633). Elle est également impliquée dans la régulation de l'axe hypothalamo-hypophyso-surrénalien et dans la réponse au stress via la libération de CRF hypothalamique (Kennedy et al, J Neuroendocrinol 2003 ; 15(3) : 268-272). L'utilisation d'un antagoniste du récepteur MCH₁ a récemment confirmé l'effet anxiogénique de la MCH. En effet, le SNAP-7941 présente un profil anxiolytique et/ou antidépresseur dans différents modèles animaux tels que le conflit social et la nage forcée chez le rat ainsi que la séparation maternelle chez le cobaye (Katsuura et al, Curr Med Chem 2003 ; 3 : 217-227). Des molécules antagonistes du récepteur MCH₁ ont donc un intérêt thérapeutique dans la dépression et/ou l'anxiété.

La MCH semble être impliqué d'autres systèmes de régulation. Par sa localisation testiculaire (Hervieu et al, Biology of Reproduction 1996 ; 5 : 1161-1172) et hypothalamique (oestrogène dépendante, Viale et al, Peptides 1999 ; 20 : 553-559) et par ses effets stimulants de l'activité sexuelle du rat mâle (Gonzales et al, Peptides 1996 ; 17 : 171-177) et de la sécrétion d'hormone lutéinisante (Chiocchio et al, Biology of Reproduction 2001 ; 64 : 1466-1472), la MCH semble donc jouer un rôle dans les fonctions reproductrices.

Il est également observé que la MCH est impliquée dans des comportements liés aux fonctions cognitives en augmentant l'extinction de l'évitement passif chez le rat, suggérant qu'un antagoniste du récepteur MCH₁ puisse être utile lors des troubles de mémoire (MacBride et al, Peptides 1994 ; 15(4) : 757-759). Ainsi, les composés selon l'invention peuvent constituer une thérapie de choix pour le traitement des troubles de mémoire.

Ainsi, les composés de l'invention trouvent leur emploi en thérapeutique, notamment dans le traitement de l'obésité, de la cellulite, de troubles métaboliques et de leurs pathologies associées telles que le diabète, les troubles cardiovasculaires, le syndrome X, dans le traitement de pathologies liées au stress telles que l'anxiété et la dépression, ainsi que dans le traitement de toutes autres maladies impliquant un dysfonctionnement lié au récepteur MCH₁ que ce soit au niveau central et/ou périphérique.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat.

Selon un autre de ses aspects, la présente invention concerne des compositions pharmaceutiques comprenant, en tant que principe actif, au moins un composé selon l'invention. Ces compositions pharmaceutiques comprennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50.0 mg |
| Mannitol | 223.75 mg |
| Croscaramellose sodique | 6.0 mg |
| Amidon de maïs | 15.0 mg |
| Hydroxypropyl-méthylcellulose | 2.25 mg |
| Stéarate de magnésium | 3.0 mg |

Lesdites formes unitaires sont dosées pour permettre une administration journalière de 0.5 mg à 800 mg de principe actif par individu, plus particulièrement de 0.5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou d'un de ses sels pharmaceutiquement acceptables ou hydrates ou solvats.

Les exemples suivants décrivent la préparation de composés conformes à l'invention.

Ces exemples ne sont pas limitatifs et ne font qu'illustrer la présente invention. Les numéros des composés exemplifiés renvoient à ceux donnés dans le tableau I.

Dans les préparations sont décrites les méthodes de synthèse des différents intermédiaires permettant d'obtenir les composés de l'invention. Ces intermédiaires sont tous obtenus selon des méthodes bien connues de l'homme de l'art.

Les points de fusion (PF) sont exprimés en degré Celsius. Ils ont été mesurés soit avec un appareil de Köffler (mention (K) dans le tableau), soit avec un appareil Mettler-Toledo FP62 (mention (M) dans le tableau), soit avec un appareil Büchi B540 (mention (B) dans le tableau)

Les conditions d'analyse par chromatographie liquide couplée à la spectrométrie de masse (LC/UV/MS) sont les suivantes:

Pour la partie chromatographie liquide :
- colonne *symmetry* C18 (2.1 x 50 mm) 3.5 µm.
- Eluant A = H₂O + 0.005 % TFA, pH = 3.14 ;
- Eluant B = CH₃CN + 0.005 % TFA.
- Gradient de 100 % de A à 90% de B en 10 minutes, puis élution par 90 % de B pendant 5 minutes.
- Débit 0.4ml/min
- Injection de 2µL de solution à 0.5mg/ml dans le méthanol
- Les produits sont détectés en UV à 210 nm.

Pour la partie spectrométrie de masse :
- Mode d'ionisation: électrospray positif.
- Balayage de 120 à 1500 uma

Les caractéristiques analytiques LC/MS des produits sont le rapport m/z de l'ion MH⁺ et le temps de rétention (tR) du pic correspondant, observé en UV et exprimé en minutes.

Les spectres de résonnance magnétique nucléaire du proton (RMN ^{1H}) ont été effectués à 250 MHz ou à 300 MHz sur des appareils Brüker. Les abbréviations utilisées pour caractérisées les signaux sont les suivantes: s = singulet, m = multiplet, d = doublet, t = triplet, q = quadruplet.

Les pouvoirs rotatoires des produits optiquement actifs sont caractérisés par leur [α]_{D}^{t°} (les concentrations c des solutions analysées sont exprimées en grammes pour 100mL).

La quantification des sels et des solvats est déterminée à l'aide de l'analyse élémentaire, du dosage de l'eau par la technique de Karl-Fischer et de l'intégration des signaux caractéristiques des solvants en RMN^{1H}.

Les composés de l'invention ainsi que leur caractéristiques analytiques (PF, LC/MS, sels et solvats) sont inventoriés dans le tableau I.

### Exemple 1 : (composé n° 1)

### 7-Méthoxy-N-[1-(naphthalèn-2-ylméthyl)pipéridin-4-yl]-4-phénylphthalazin-1-amine

### 1.1. Acide 2-benzoyl-5-méthoxy-benzoïque

Une solution d'acide 2-bromo-5-méthoxy-benzoïque (5 g, 21.6 mmol) dans 50 mL de THF est agitée sous argon à -78°C, puis est additionnée goutte à goutte d'une solution de n-butyllithium 1.6 M dans l'hexane (29 mL, 45 mmol). Le mélange réactionnel est agité pendant 1 heure à -78°C, puis est additionné goutte à goutte de chlorure de benzoyle (3.2 g, 22.7 mmol). Le mélange réactionnel est agité pendant 1 heure 30 minutes à -78 °C, puis 18 heures à température ambiante. Le mélange est hydrolysé par 100 mL d'eau puis lavé par l'acétate d'éthyle. La phase aqueuse est acidifiée par addition d'acide chlorhydrique 5 N puis extraite par l'acétate d'éthyle. La phase organique obtenue est lavée à l'eau salée puis séchée sur sulfate de sodium anhydre. Après filtration et évaporation sous pression réduite, on obtient 5.4 g d'une huile incolore qui est utilisée sans purification.

### 1.2. 7-Méthoxy-4-phényl-2H-phthalazin-1-one

Une solution de 5.4 g d'acide 2-benzoyl-5-méthoxy-benzoïque dans 50 mL d'éthanol est additionnée de 10.55 g (210 mmol) d'hydrate d'hydrazine et est portée à reflux pendant 2 heures 30⁻ minutes. Le milieu réactionnel est refroidi puis filtré. Le précipité obtenu est lavé à l'éthanol puis séché sous pression réduite à 40°C. On obtient 740 mg de produit sous forme de solide blanc.
PF = 215°C (banc Kofler)
LC/MS: MH⁺= 253 (tR = 7.05 minutes)
RMN ^{1H} δ en ppm (DMSO d 6) : 3.97 (s, 3H); 7.48 (dd, J₁ = 8.8 Hz, J₂ = 2.7 Hz, 1H); 7.54-7.60 (m, 5H); 7.64 (d, J = 8.8 Hz, 1H); 7.75 (d, J = 2.7 Hz, 1H); 12.78 (s, 1H, NH).

### 1.3. 4-Chloro-6-méthoxy-1-phényl-phthalazine

La 7-méthoxy-4-phényl-2H-phthalazin-1-one (740 mg, 2.9 mmol) est dissoute dans 7 mL de chlorure de phosphoryle. La solution est chauffée pendant 2 heures à 80°C. Le milieu réactionnel est refroidi à température ambiante puis est versé lentement dans 200 mL d'eau sous agitation. Le mélange est ensuite agité à 5°C, rendu alcalin par une solution de soude à 35 %, puis extrait par du dichlorométhane. La phase organique est lavée à l'eau, puis à l'eau salée et séchée sur sulfate de sodium anhydre. Après filtration et évaporation sous pression réduite, on obtient 750 mg de produit sous forme de poudre beige.
PF = 199°C (Mettler FP62)
LC/MS: MH⁺= 271 (tR = 7.89 minutes)
RMN^{1H} δ en ppm (DMSO d 6) : 4.06 (s, 3H); 7.60-7.64 (m, 4H); 7.67-7.72 (m, 3H); 7.95 (d, J = 9.2 Hz, 1H).

### 1.4. 7-Méthoxy-N-[1-(naphthalèn-2-ylméthyl)pipéridin-4-yl]-4-phénylphthalazin-1-amine

Une suspension de 350 mg (1.3 mmol) de 4-chloro-6-méthoxy-1-phényl-phthalazine, dans 6 mL de n-butanol est additionnée de 373 mg (1.5 mmol) de 4-amino-1-(naphthalèn-2-yl-méthyl)-pipéridine et de 69 mg (1.3 mmol) de chlorure d'ammonium. Le mélange est chauffé à 135°C durant 20 heures. Le milieu réactionnel est refroidi à température ambiante, hydrolysé par 50 mL d'eau, puis alcalinisé à pH 10 par de la soude 1 N. Le mélange est ensuite extrait par l'acétate d'éthyle. La phase organique est lavée à l'eau salée, séchée sur sulfate de sodium anhydre, puis évaporée sous pression réduite. Le résidu obtenu est purifié par chromatographie sur colonne de gel de silice (solvant: dichlorométhane/méthanol 95/5 (v/v)). L'huile jaune ainsi obtenue forme un solide par trituration dans l'éther isopropylique. Après filtration et séchage on obtient 275 mg de produit sous forme de poudre beige.
PF = 121°C (banc Kofler)
MH⁺ = 475 (tR =5.15 minutes)
RMN ^{1H} δ en ppm (DMSO d 6) : 1.69-1.83 (m, 2H); 2.08-2.25 (m, 4H); 2.96-3.01 (m, 2H); 3.71 (s, 2H); 3.99 (s, 3H); 4.27-4.37 (m, 1H); 7.06 (d, J = 7.5 Hz, 1H, NH); 7.44 (dd, J₁ = 8.8 Hz, J₂ = 2.7 Hz, 1H); 7.49-7.59 (m, 8H); 7.73 (d, J = 8.8 Hz, 1H); 7.79 (d, J = 2.7 Hz, 1H); 7.85-7.95 (m, 4H).

### Exemple 2: (composé n° 11)

### N-(1-benzylpipéridin-4-yl)-5,7-diméthoxy-4-(4-méthoxyphényl)phthalazin-1-amine

### 2.1. Acide 3,5-diméthoxy-2-(4-méthoxy-benzoyl)-benzoïque

Une solution 3,5-diméthoxy-benzoate de méthyle (6.4 g, 33 mmol) dans 50 mL de dichlorométhane sous agitation à température ambiante est additionnée successivement de 5.7 g (43 mmol) de chlorure d'aluminium, puis d'une solution de chlorure d'acide 4-méthoxy-benzoïque (5.6 g, 33 mmol). Le mélange est agité pendant une nuit à température ambiante, puis est versé dans 200 mL d'eau glacée sous agitation. Le milieu hydrolysé est extrait au dichlorométhane. La phase organique est lavée par une solution saturée d'hydrogénocarbonate de sodium, puis par de l'eau, séchée sur sulfate de sodium anhydre, puis évaporée sous pression réduite. L'extrait brut est purifié par chromatographie sur colonne de gel de silice (solvant: cyclohexane / acétate d'éthyle 3/1 (v/v)).

L'ester méthylique ainsi obtenu est mis en solution dans 80 mL de méthanol puis additionné de 17 mL de soude 2N. Le mélange est agité une nuit à température ambiante, puis 3 heures à 60°C. Le méthanol est ensuite évaporé sous pression réduite. La solution résiduelle est diluée par 100 mL d'eau, acidifiée par addition d'acide chlorhydrique 2N, puis extraite au dichlorométhane. La phase organique est séchée sur sulfate de sodium anhydre, puis évaporée sous pression réduite. Un solide blanc est obtenu (5.4 g).
PF = 191°C.(Mettler FP62)
RMN ^{1H} δ en ppm (DMSO d 6) : 3.69 (s, 3H); 3.83 (s, 3H); 3.90 (s, 3H); 6.93 (d, J = 2.2 Hz, 1H); 9.91-7.03 (m, 2H); 7.09 (d, J = 2.2 Hz, 1H); 7.56-7.63 (m, 2H); 13.1 (s, 1H, COOH).

### 2.2. 5,7-diméthoxy-4-(4-méthoxy-phényl)-2H-phthalazin-1-one.

Ce produit est préparé à partir de l'acide 3,5-diméthoxy-2-(4-méthoxy-benzoyl)-benzoïque, obtenu à l'étape 2.1, selon la méthode décrite à l'étape 1.2 de l'exemple 1.
MH⁺ = 313 (tR = 7.04 minutes)
RMN 1H en ppm (DMSO d 6) : 3.55 (s, 3H); 3.82 (s, 3H); 3.96 (s, 3H); 6.9-6.98 (m, 3H); 7.25-7.32 (m, 2H); 7.37 (d, 1H); 12.66 (s, NH).

### 2.3. 1-chloro-5,7-diméthoxy-4-(4-méthoxy-phényl)-phthalazine

Ce produit est préparé à partir de la 5,7-diméthoxy-4-(4-méthoxy-phényl)-2H-phthalazin-1-one, obtenue à l'étape 2.2, selon la méthode décrite à l'étape 1.3 de l'exemple 1.
MH⁺ = 331 (tR = 7.73 minutes)
RMN ^{1H} δ en ppm (DMSO d 6) : 3.65 (s, 3H); 3.86 (s, 3H); 4.06 (s, 3H); 6.99-7.05 (m, 2H); 7.15 (d, J = 2.2 Hz, 1H); 7.22 (d, J = 2.2 Hz, 1H); 7.39-7.45 (m, 2H).

### 2.4. N-(1-benzylpipéridin-4-yl)-5,7-diméthoxy-4-(4-méthoxyphényl)phthalazin-1-amine

Dans un tube sous argon sont introduits successivement : 400 mg (1.2 mmol) de 4-chloro-6,8-diméthoxy-1-(4-méthoxy-phényl)-phthalazine en solution dans 5 mL de toluène, 276 mg (1.4 mmol) de 4-amino-1-benzylpipéridine, 163 mg (1.7 mmol) de tert-butylate de sodium, 6 mg (0.009 mmol) de BINAP (2,2'-bis(diphénylphosphino)-1,1'-binaphtyle), et de 5.5 mg (0.006 mmol) de tris(dibenzylidène-acétone)-dipalladium. Le tube est fermé hermétiquement et le mélange est agité à 80°C pendant 24 heures. Après retour à température ambiante, le milieu réactionnel est hydrolysé par une solution d'hydrogénocarbonate de sodium, puis extrait par l'acétate d'éthyle. La phase organique est lavée par de l'eau, par de l'eau salée, séchée sur sulfate de sodium anhydre, puis évaporée sous pression réduite. Le résidu obtenu est purifié par chromatographie sur colonne de gel de silice (solvant: dichlorométhane/méthanol 95/5 (v/v)). Le produit huileux obtenu forme un solide par trituration dans l'éther éthylique. On obtient 270 mg de produit sous forme de poudre beige.
PF = 168°C (Köffler)
MH⁺ = 485 (tR = 4.82 minutes)
RMN ^{1H} δ en ppm (DMSO d 6) : 1.67-1.76 (m, 2H); 2.04-2.17 (m, 4H); 2.90-2.94 (m, 2H); 3.54 (s, 5H); 3.82 (s, 3H); 3.98 (s, 3H); 4.20-4.30 (m, 1H); 6.84 (d, 1H, NH); 6.88-6.96 (m, 3H); 7.23-7.37 (m, 8H).

### Exemple 3: (composé n° 7)

### N-(1-benzylpipéridin-4-yl)-7-méthoxy-4-(4-méthoxyphényl)phthalazin-1-amine

### 3.1. N,4-diméthoxy-N-méthylbenzamide

Une solution de 25 g (147 mmoles) de chlorure de l'acide 4-méthoxybenzoïque dans 450 mL de dichlorométhane, agitée à 0-5°C sous azote, est additionnée par fractions de 14.7 g (151 mmoles) de chlorhydrate de N,O-diméthylhydroxylamine. Le mélange agité à 0-5°C est ensuite additionné lentement de 51 mL (370 mmoles) de triéthylamine. Le milieu réactionnel de couleur orange est agité à température ambiante pendant 3 heures. Le mélange est hydrolysé avec 250 mL d'eau, puis est extrait au dichlorométhane. La phase organique est lavée avec 100 mL d'HCl 1N, 150 mL de NaOH 1N, puis à l'eau et à l'eau salée. Elle est séchée sur sulfate de sodium anhydre, filtrée et évaporée à sec. On obtient 26.9 g d'huile orange qui sont purifiés sur colonne de silice avec comme éluant du dichlorométhane puis un mélange dichlorométhane-méthanol 95-5. 25.4 g d'huile jaune sont obtenus (rendement 89%).
LC/MS: MH⁺= 196 (tR = 5.21 minutes)
RMN ^{1H} δ en ppm (DMSO d 6) : 3.26 (s, 3H); 3.56 (s, 3H); 3.82 (s, 3H); 7.00 (d, J=8.5 Hz, 2H); 7.63 (d, J = 8.5 Hz, 2H).

### 3.2. Acide 5-méthoxy-2-(4-méthoxybenzoyl)benzoïque

Une solution de 18.5 g (80 mmoles) d'acide 2-bromo-5-méthoxybenzoïque dans 150 mL de tétrahydrofuranne est agitée sous azote à -78°C dans un bain de carboglace/acétone. 100 mL (160 mmoles) d'une solution 1.6M de n-butyllithium dans l'hexane sont ajoutés goutte à goutte pendant environ 1 heure en veillant à ce que la température n'excède pas -70°C. A la demi-introduction on remarque la formation d'un précipité beige qui correspond à la formation du carboxylate de lithium. Après l'addition, le mélange est agité à -78°C pendant 1 heure, puis une solution de 15.9 g (80 mmoles) de N,4-diméthoxy-N-méthylbenzamide dans 20 mL de THF est ajouté goutte à goutte. Le milieu réactionnel est agité à -78°C pendant 1 heure puis le bain de carboglace/acétone est retiré et le milieu réactionnel est agité en laissant la température remonter progressivement à l'ambiante pendant 18 heures. Le mélange est hydrolysé avec 100 mL d'eau, basifié à pH = 12 à l'aide d'une solution de NaOH 2N et extrait avec du tert-butylméthyléther. La phase aqueuse contenant le carboxylate est acidifiée par une solution de HCl 5 N jusqu'à pH=1 et extraite au dichlorométhane. La phase dichlorométhane est lavée à l'eau salée, séchée sur sulfate de sodium anhydre, filtrée et évaporée. Le produit est cristallisé dans l'éther isopropylique ; après filtration et séchage 14.6 g de cristaux blancs sont obtenus (rendement = 64 %).
PF = 170°C (Mettler FP62)
LC/MS: MH⁺= 287 (tR = 7.07 minutes)
RMN ^{1H} δ en ppm (DMSO d 6) : 3.85 (s, 3H); 3.90 (s, 3H); 7.03 (d, J = 8.5Hz, 2H); 7.24 (dd, J₁ = 8.5 Hz, J₂ = 2.5 Hz, 1H); 7.35 (d, J = 8.5Hz, 1H); 7.42 (d, J = 2.5 Hz, 1H); 7.61 (d, J = 8.5Hz, 2H); 13.1 (s, 1H, COOH).

### 3.3. 7-Méthoxy-4-(4-méthoxy-phényl)-2H-phtalazin-1-one

Une solution de 21.6 g (75 mmol) d'acide 5-méthoxy-2-(4-méthoxybenzoyl)benzoïque dans 150 mL d'éthanol est additionnée de 18.5 mL (380 mmol) d'hydrate d'hydrazine et est portée à reflux pendant 2 heures. Le milieu réactionnel est refroidi puis filtré. Le précipité obtenu est lavé à l'éthanol puis séché sous pression réduite à 40°C. 19 g d'un solide blanc sont obtenus (rendement 90%).
PF = 245°C (Mettler FP62)
LC/MS: MH⁺= 283 (tR = 6.98 minutes)
RMN ^{1H} δ en ppm (DMSO d 6) : 3.86 (s, 3H); 3.98 (s, 3H); 7.12 (d, J = 8.5Hz, 2H); 7.48 (dd, J₁ = 9 Hz, J₂ = 2.7 Hz, 1H); 7.52 (d, J = 8.5 Hz, 2H); 7.67 (d, J = 9.0 Hz, 1H); 7.75 (d, J = 2.7 Hz, 1H); 12.72 (s, 1H, NH).

### 3.4. 1-Chloro-7-méthoxy-4-(4-méthoxy-phényl)-phthalazine

19 g (67 mmol) de 7-méthoxy-4-(4-méthoxy-phényl)-2H-phthalazin-1-one sont dissouts dans 100 mL de chlorure de phosphoryle. La solution est chauffée pendant 2 heures à 80°C. Le milieu réactionnel est refroidi à température ambiante puis est versé lentement dans 500 mL d'eau à 40-50°C sous agitation. Le mélange est ensuite agité à 5°C, rendu alcalin par addition d'une solution de soude à 35%, puis extrait par du dichlorométhane. La phase organique est lavée à l'eau, puis à l'eau salée et séchée sur sulfate de sodium anhydre. Après filtration et évaporation sous pression réduite, on obtient 19.3 g d'une poudre beige (rendement = 96 %).
PF = 173°C
LC/MS: MH⁺= 301 (tR = 8.52 minutes)
RMN ^{1H} δ en ppm (DMSO d 6) : 3.90 (s, 3H); 4.07 (s, 3H); 7.19 (d, J = 8.5Hz, 2H); 7.60 (d, J = 2.5 Hz, 1H); 7.68 (d, J = 8.5 Hz, 2H); 7.72 (dd, J₁ = 9.2 Hz, J₂ = 2.5 Hz, 1H); 8.02 (d, J = 9.2 Hz, 1H).

### 3.5. 1-(1-Benzylpipéridin-4-yl)-7-méthoxy-4-(4-méthoxyphényl)-phthalazin-1-amine

Une suspension de 8 g (26.6 mmol) de 1-Chloro-7-méthoxy-4-(4-méthoxy-phényl)-phthalazine dans 50 mL de 1-butanol est additionnée de 10.1 g (53 mmol) de 4-amino-1-benzyl)-pipéridine et de 1.44 g (27 mmol) de chlorure d'ammonium. Le mélange est chauffé au reflux (bain d'huile à 140°C) durant 7 heures. Le milieu réactionnel est refroidi à température ambiante, hydrolysé par 50 mL d'une solution aqueuse saturée en hydrogénocarbonate de sodium puis extrait par l'acétate d'éthyle. La phase organique est lavée à l'eau salée, séchée sur sulfate de sodium anhydre, puis évaporée sous pression réduite. Le résidu obtenu est purifié par chromatographie sur colonne de gel de silice (solvant: dichlorométhane/méthanol 95/5 (v/v)). L'huile jaune ainsi obtenue forme un solide par trituration dans l'éther isopropylique. Après filtration et séchage on obtient 10.5 g de poudre blanche (rendement = 87 %).
PF = 150°C (Mettler FP62)
LC/MS: MH⁺= 455 (tR = 4.71 minutes)
RMN ^{1H} δ en ppm (DMSO d 6) : 1.7-1.8 (m, 2H); 2.0-2.2 (m, 4H); 2.90-3.0 (m, 2H); 3.54 (s, 2H); 3.86 (s, 3H); 4.0 (s, 3H); 4.2-4.3 (m, 1H); 7.0 (d, J = 7.5 Hz, 1H, NH); 7.10 (d, J = 8.5 Hz, 2H); 7.25-7.40 (m, 5H); 7.43 (dd; J₁ = 9.0 Hz ; J₂ = 2.5 Hz, 1H); 7.53 (d, J = 8.5 Hz, 2H); 7.75-7.77 (m, 2H).

### Exemple 4: (composé n° 4)

### 7-Méthoxy-4-(4-méthoxyphényl)-N-[8-(naphthalèn-2-ylméthyl)-8-azabicyclo[3.2.1]oct-3-yl]phthalazin-1-amine

### 4.1. N-[8-(naphthalèn-2-ylméthyl)-8-azabicyclo[3.2.1]oct-3-yl]-acétamide

1.6 g (9.5 mmol) de N-[8-azabicyclo[3.2.1]oct-3-yl]-acétamide préparé selon Dostert et al (European Journal of Medicinal Chemistry 1984, 19 (2), 105-110) sont dissouts dans 45 mL d'acétone et additionnés de 5.2 g de carbonate de potassium et de 2.5g (11.4 mmol) de 2-(bromométhyl)naphthalène. Le mélange est agité et chauffé au reflux pendant 18 heures. Après refroidissement, l'acétone est partiellement évaporée. Le résidu est additioné d'eau et extrait au dichlorométhane. La phase organique est extraite par de l'acide chlorhydrique 1N. La phase aqueuse acide obtenue est alcalinisée par de la soude 1N puis extraite au dichlorométhane. L'extrait est lavé à l'eau, à l'eau salée, séché sur sulfate de sodium anhydre, puis évaporé sous pression réduite. Le résidu obtenu est filtré sur gel de silice (solvant: dichlorométhane/méthanol 95/5 (v/v)). 2.2g d'une huile pâteuse sont obtenus (rendement 84 %)
LC/MS: MH⁺= 309 (tR = 4.49 minutes)
RMN ^{1H} δ en ppm (DMSO d 6) : 1.5-1.65 (m, 6H); 1.77 (s, 3H); 2.0-2.1 (m, 2H); 3.1-3.2 (m, 2H); 3.70 (s, 2H); 3.9-4.0 (m, 1H); 7.4-7.6 (m, 3H); 7.7 (d, J = 8.2 Hz, 1H, NH); 7.82-7.95 (m, 4H).

### 4.2. N-[8-(naphthalèn-2-ylméthyl)-8-azabicyclo[3.2.1]oct-3-yl]-amine

4 g (12.9 mmol) de N-[8-(naphthalèn-2-ylméthyl)-8-azabicyclo[3.2.1]oct-3-yl]-acétamide sont versés dans 13 mL d'acide chlorhydrique 5N. Le mélange est chauffé au reflux pendant 4 heures, puis refroidit au bain de glace, alcalinisé par de la soude à 35% et extrait 3 fois par de l'acétate d'éthyle. Les extraits réunis sont lavés à l'eau, à l'eau salée, séchés sur sulfate de sodium anhydre, puis évaporés sous pression réduite. 3.2g de produit huileux sont obtenus (rendement 94%).
LC/MS: MH⁺= 267 (tR = 1.71 minutes)
RMN ^{1H} δ en ppm (DMSO d 6) : 1.3-1.45 (m, 2H); 1.5-1.65 (m, 6H); 1.9-2.05 (m, 2H); 2.8 (m, 1H, CH axial); 3.1-3.2 (m, 2H); 3.65 (s, 2H); 7.4-7.6 (m, 3H); 7.75-7.9 (m, 4H).

### 4.3 7-Méthoxy-4-(4-méthoxyphényl)-N-[8-(naphthalèn-2-ylméthyl)-8-azabicyclo[3.2.1]oct-3-yl]phthalazin-1-amine

Ce composé est obtenu selon le mode opératoire décrit en 1.4. par réaction de la N-[8-(naphthalèn-2-ylméthyl)-8-azabicyclo[3.2.1]oct-3-yl]-amine et de la 1-Chloro-7-méthoxy-4-(4-méthoxy-phényl)-phthalazine.

### Exemple 5: (composé n° 8)

### N-(1-benzylpipéridin-4-yl)-7-méthoxy-4-(thiophèn-2-yl)phthalazin-1-amine

### 5.1. Acide 5-méthoxy-2-(thiophène-2-carbonyl)-benzoïque

Ce composé est obtenu selon le mode opératoire décrit en 1.1. par réaction du chlorure de l'acide thiophène-2-yl et de l'acide 2-bromo-5-méthoxy-benzoïque après échange halogène-lithium. Il est utilisé brut dans la réaction suivante.

### 5.2. 7-Méthoxy-4-(thiophène-2-yl)-2H-phthalazin-1-one

Ce composé est obtenu selon le mode opératoire décrit en 1.2. par réaction de l'acide 5-méthoxy-2-(thiophène-2-carbonyl)-benzoïque non purifié avec de l'hydrate d'hydrazine
LC/MS: MH⁺= 259 (tR = 7.50 minutes)

### 5.3. 1-Chloro-7-méthoxy-4-(thiophène-2-yl)-phthalazine

Ce composé est obtenu selon le mode opératoire décrit en 1.3. par réaction de la 7-méthoxy-4-(thiophène-2-yl)-2H-phthalazin-1-one avec le chlorure de phosphoryle.
PF = 199°C (Mettler FP62)
LC/MS: MH⁺= 277 (tR = 7.99 minutes)
RMN ^{1H} δ en ppm (DMSO d 6) : 4.07 (s, 3H); 7.34-7.38 (m, 1H); 7.60 (d, J = 2.7 Hz, 1H); 7.78 (dd, J₁ = 9.2 Hz, J₂ = 2.7 Hz, 1H); 7.86 (d, J = 3.7 Hz, 1H); 7.95 (d, J = 5.2 Hz, 1H); 8.50 (d, J = 9.2 Hz, 1H).

### 5.4. N-(1-benzylpipéridin-4-yl)-7-méthoxy-4-(thiophèn-2-yl)phthalazin-1-amine

Ce composé est obtenu selon le mode opératoire décrit en 1.4. par réaction de la 1-Chloro-7-méthoxy-4-(thiophène-2-yl)-phthalazine avec la 4-amino-1-benzylpipéridine.

### Exemple 6: (composé 16)

### N-(1-benzylpipéridin-4-yl)-7-méthoxy-4-(5-méthylthiophèn-2-yl)phthalazin-1-amine

### 6.1 Acide 5-méthoxy-2-(5-méthyl-thiophène-2-carbonyl)-benzoïque

Ce composé est obtenu selon le mode opératoire décrit en 1.1. par réaction du chlorure de l'acide 5-méthylthiophène-2-yl et de l'acide 2-bromo-5-méthoxy-benzoïque après échange halogène-lithium. Il est utilisé brut dans la réaction suivante.

### 6.2. 7-Méthoxy-4-(5-méthylthiophène-2-yl)-2H-phthalazin-1-one

Ce composé est obtenu selon le mode opératoire décrit en 1.2. par réaction de l'acide 5-méthoxy-2-(5-méthyl-thiophène-2-carbonyl)-benzoïque non purifié avec de l'hydrate d'hydrazine.
LC/MS: MH⁺= 273 (tR = 7.58 minutes)

### 6.3. 1-Chloro-7-méthoxy-4-(5-méthylthiophène-2-yl)-phthalazine

Ce composé est obtenu selon le mode opératoire décrit en 1.3. par réaction de la 7-méthoxy-4-(5-méthylthiophène-2-yl)-2H-phthalazin-1-one avec le chlorure de phosphoryle.
LC/MS: MH⁺= 291 (tR = 8.74 minutes)
RMN ^{1H} δ en ppm (DMSO d 6) : 2.59 (s, 3H); 4.08 (s, 3H); 7.06 (m, 1H); 7.60 (d, J = 2.7 Hz, 1H); 7.68 (d, J = 3.5 Hz, 1H); 7.77 (dd, J₁ = 9.2 Hz, J₂ = 2.7 Hz, 1H); 8.51 (d, J = 9.2 Hz, 1H).

### 6.4. N-(1-benzylpipéridin-4-yl)-7-méthoxy-4-(5-methylthiophèn-2-yl)phthalazin-1-amine

Ce composé est obtenu selon le mode opératoire décrit en 1.4. par réaction de la 1-chloro-7-méthoxy-4-(5-méthylthiophène-2-yl)-phthalazine avec la 4-amino-1-benzylpipéridine.

### Exemple 7: (composé n°6)

### N-(1-benzylpipéridin-4-yl)-7-méthoxy-4-(3-méthoxyphényl)phthalazin-1-amine

### 7.1 Acide 5-méthoxy-2-(3-méthoxybenzoyl)benzoïque

Ce composé est obtenu selon le mode opératoire décrit en 1.1. par réaction du chlorure de l'acide 3-méthoxybenzoique et de l'acide 2-bromo-5-méthoxy-benzoïque après échange halogène-lithium. Il est utilisé brut dans la réaction suivante.

### 7.2. 7-Méthoxy-4-(3-méthoxy-phényl)-2H-phthalazin-1-one

Ce composé est obtenu selon le mode opératoire décrit en 1.2. par réaction de l'acide 5-méthoxy-2-(3-méthoxybenzoyl)benzoïque non purifié avec de l'hydrate d'hydrazine.
PF= 219°C (Mettler FP62)
LC/MS: MH⁺= 283 (tR = 7.41 minutes)
RMN ^{1H} δ en ppm (DMSO d 6) : 3.83 (s, 3H); 3.97 (s, 3H); 7.10-7.16 (m, 3H); 7.44-7.51 (m, 2H); 7.67 (d, J = 9 Hz, 1H); 7.75 (d, J = 2.5Hz, 1H); 12.80 (s, 1H, NH).

### 7.3. 1-Chloro-7-méthoxy-4-(3-méthoxy-phényl)-phthalazine

Ce composé est obtenu selon le mode opératoire décrit en 1.3. par réaction de la 7-méthoxy-4-(3-méthoxy-phényl)-2H-phthalazin-1-one avec le chlorure de phosphoryle.
PF = 98°C (Mettler FP62)
LC/MS: MH⁺= 301 (tR = 8.07 minutes)
RMN ^{1H} δ en ppm (DMSO d 6) : 3.86 (s, 3H); 4.08 (s, 3H); 7.18-7.27 (m, 3H); 7.52-7.58 (m, 1H); 7.62 (d, J = 2.5 Hz, 1H); 7.72 (dd, J₁ = 9.2 Hz, J₂ = 2.5 Hz, 1H); 8.02 (d, J = 9.2 Hz, 1H).

### 7.4. 1-(1-Benzylpipéridin-4-yl)-7-méthoxy-4-(3-méthoxyphényl)-phthalazin-1-amine

Ce composé est obtenu selon le mode opératoire décrit en 1.4. par réaction de la 1-Chloro-7-méthoxy-4-(3-méthoxy-phényl)-phthalazine avec la 4-amino-1-benzylpipéridine.

### Exemple 8: (composé n°3)

### 7-méthoxy-4-(4-méthoxyphényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-(4-méthoxy-phényl)-phthalazine décrite en 3.4 avec la 4-amino-1-(naphthalèn-2-yl-méthyl)-pipéridine.

### Exemple 9: (composé n°5)

### 7-méthoxy-4-(3-méthoxyphényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-(3-méthoxy-phényl)-phthalazine décrite en 7.3 avec la 4-amino-1-(naphthalèn-2-yl-méthyl)-pipéridine.

### Exemple 10: (composé n°2)

### 7-méthoxy-4-(2-méthoxyphényl)-N-[1-(naphthalèn-2-ylméthyl)pipéridin-4-yl]phthalazin-1-amine

### 10.1. Acide 5-méthoxy-2-(2-méthoxybenzoyl)-benzoïque

Ce composé est obtenu selon le mode opératoire décrit en 1.1. par réaction du chlorure de l'acide 2-méthoxybenzoique et de l'acide 2-bromo-5-méthoxy-benzoïque après échange halogène-lithium. Il est utilisé brut dans la réaction suivante.

### 10.2. 7-Méthoxy-4-(2-méthoxy-phényl)-2H-phthalazin-1-one

Ce composé est obtenu selon le mode opératoire décrit en 1.2. par réaction de l'acide 5-méthoxy-2-(2-méthoxybenzoyl)-benzoïque non purifié avec de l'hydrate d'hydrazine.
PF= 275°C (Mettler FP62)
LC/MS: MH⁺= 283 (tR = 7.76 minutes)
RMN ^{1H} δ en ppm (DMSO d 6) : 3.70 (s, 3H); 3.96 (s, 3H); 7.11-7.15 (m, 1H); 7.20-7.23 (d, J = 8.7 Hz, 2H); 7.32-7.35 (m, 1H); 7.39-7.44 (m, 1H); 7.50-7.55 (m, 1H); 7.69 (d, J = 2.7 Hz, 1H); 12.70 (s, 1H, NH).

### 10.3. 1-Chloro-7-méthoxy-4-(2-méthoxy-phényl)-phthalazine

Ce composé est obtenu selon le mode opératoire décrit en 1.3. par réaction de la 7-méthoxy-4-(2-méthoxy-phényl)-2H-phthalazin-1-one avec le chlorure de phosphoryle.
PF = 157°C (Mettler FP62)
LC/MS: MH⁺= 301 (tR = 7.81 minutes)
RMN ^{1H} δ en ppm (DMSO d 6): 3.69 (s, 3H); 4.06 (s, 3H); 7.15-7.22 (m, 1H); 7.28 (d, J = 7.6 Hz, 1H); 7.42 (dd, J₁ = 7.6 Hz, J₂ = 1.7 Hz, 1H); 7.55-7.68 (m, 4H).

### 10.4. 7-méthoxy-4-(2-méthoxyphényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-(2-méthoxy-phényl)-phthalazine décrite avec la 4-amino-1-(naphthalèn-2-yl-méthyl)-pipéridine.

### Exemple 11: (composé n°10)

### 4-(3,4-Diméthoxyphényl)-7-méthoxy--N-[1-(naphthalèn-2-ylméthyl)pipéridin-4-yl]phthalazin-1-amine

### 11.1 Acide 2-(3,4-diméthoxybenzoyl)-5-méthoxy-benzoïque

Ce composé est obtenu selon le mode opératoire décrit en 1.1. par réaction du chlorure de l'acide 3,4-diméthoxybenzoique et de l'acide 2-bromo-5-méthoxy-benzoïque après échange halogène-lithium. Il est utilisé brut dans la réaction suivante.

### 11.2. 4-(3,4-Diméthoxy-phényl)-7-méthoxy-2H-phthalazin-1-one

Ce composé est obtenu selon le mode opératoire décrit en 1.2. par réaction de l'acide 2-(3,4-diméthoxybenzoyl)-5-méthoxy-benzoïque non purifié avec de l'hydrate d'hydrazine.
PF= 258°C (Mettler FP62)
LC/MS: MH⁺= 313 (tR = 6.54 minutes)
RMN ^{1H} δ en ppm (DMSO d 6) : 3.81 (s, 3H); 3.86 (s, 3H); 3.97 (s, 3H); 7.12-7.16 (m, 3H); 7.48 (dd, J₁ = 9 Hz, J₂ = 2.7 Hz, 1H); 7.71-7.76 (m, 2H); 12.71 (s, 1H, NH).

### 11.3. 1-Chloro-4-(3,4-diméthoxy-phényl)-7-méthoxyphthalazine

Ce composé est obtenu selon le mode opératoire décrit en 1.3. par réaction de la 4-(3,4-diméthoxy-phényl)-7-méthoxy-2H-phthalazin-1-one avec le chlorure de phosphoryle.
PF = 190°C (Mettler FP62)
LC/MS: MH⁺= 331 (tR = 7.43 minutes)
RMN ^{1H} δ en ppm (DMSO d 6) : 3.85 (s, 3H); 3.89 (s, 3H); 4.08 (s, 3H); 7.18-7.32 (m, 3H); 7.61 (d, J = 2.5 Hz, 1H); 7.72 (dd, J₁ = 9.2 Hz, J₂ = 2.5 Hz, 1H); 8.01 (d, J = 9.2 Hz, 1H).

### 11.4. 4-(3,4-Diméthoxyphényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(3,4-diméthoxy-phényl)-7-méthoxyphthalazine avec la 4-amino-1-(naphthalèn-2-yl-méthyl)-pipéridine.

### Exemple 12: (composé n°15)

### Dichlorhydrate de la 5,7-diméthoxy-4-(4-méthoxyphényl)-N-[1-(naphthalèn-2-ylméthyl)pipéridin-4-yl]phthalazin-1-amine

Ce composé est obtenu selon le mode opératoire décrit en 2.4 par réaction de la 4-chloro-6,8-diméthoxy-1-(4-méthoxy-phényl)-phthalazine décrite en 2.3 avec la 4-amino-1-(naphthalèn-2-yl-méthyl)-pipéridine.

Par traitement avec de l'éther chlorhydrique, il se forme un précipité qui est filtré, lavé à l'éther éthylique et séché sous pression réduite. Le chlorhydrate obtenu est sous la forme d'un solide blanc.

### Exemple 13: (composé n°13)

### N-(1-benzylpipéridin-4-yl)-5,7-diméthoxyphthalazin-1-amine

### 13.1. 5,7-Diméthoxy-2H-phthalazin-1-one

3g (13.4 mmol) de 2-formyl-3,5-diméthoxy-benzoate de méthyle sont mis en solution dans 80 mL de méthanol et 10 mL de soude 2N. Le mélange est agité pendant 18 heures à température ambiante. Le méthanol est évaporé sous pression réduite et le concentré est acidifié par de l'acide chlorhydrique 2N. Le précipité formé est filtré puis séché. 2.7g d'acide sous forme de solide jaune sont obtenus.

L'acide 2-formyl-3,5-diméthoxy-benzoique (2.7 g, 13 mmol) est partiellement dissout dans 300 mL d'éthanol puis est additionné de 6.2 mL d'hydrate d'hydrazine. Le mélange est chauffé au reflux pendant 6 heures. Après refroidissement, le précipité formé est filtré puis séché. 2.3 g d'un solide blanc sont obtenus (rendement 86%)
LC/MS: MH⁺= 207 (tR = 5.35 minutes)
RMN ^{1H} δ en ppm (DMSO d 6) : 3.95 (s, 3H); 3.98 (s, 3H); 7.02 (d, J = 2.5 Hz, 1H); 7.21 (d, J = 2.5 Hz, 1H); 8.30 (s, 1H); 12.61 (s, 1H, NH)

### 13.2. 1-Chloro-5,7-diméthoxy-phthalazine

1g (4.9 mmol) de 5,7-diméthoxy-2H-phthalazin-1-one et 10mL de chlorure de phosphoryle sont chauffé à 80°C pendant 2 heures. Après retour à température ambiante, le milieu réactionnel est versé goutte à goutte dans 50mL d'eau puis le mélange est alcalinisé par de la soude 6N et extrait au dichlorométhane. La phase organique est lavée à l'eau, à l'eau salée, séchée sur sulfate de sodium anhydre, puis évaporée sous pression réduite. 1g d'un solide beige est obtenu (rendement 91%).
PF = 167°C (Mettler FP62)
LC/MS: MH⁺= 225 (tR = 6.15 minutes)
RMN ^{1H} δ en ppm (DMSO d 6) : 4.03 (s, 3H); 4.06 (s, 3H); 7.04 (d, J = 2.2 Hz, 1H); 7.15 (d, J = 2.2 Hz, 1H); 9.49 (s, 1H).

### 13.3. N-(1-benzylpipéridin-4-yl)-5,7-diméthoxyphthalazin-1-amine

Ce composé est obtenu selon le mode opératoire décrit en 2.4 par réaction de la 1-chloro-5,7-diméthoxy-phthalazine avec la 4-amino-1-benzylpipéridine..

### Exemple 14: (composé n°14)

### 5,7-diméthoxy-N-[1-(naphthalèn-2-ylméthyl)pipéridin-4-yl]phthalazin-1-amine

Ce composé est obtenu selon le mode opératoire décrit en 2.4 par réaction de la 1-chloro-5,7-diméthoxy-phthalazine décrite en 13.2 avec la 4-amino-1-(naphthalèn-2-yl-méthyl)-pipéridine.

### Exemple 15: (composé n°9)

### 4-(4-méthoxyphényl)-7-méthyl-N-[1-(naphthalèn-2-ylméthyl)pipéridin-4-yl]phthalazin-1-amine

### 15.1 Acide 2-(4-méthoxybenzoyl)-5-méthyl-benzoïque

Ce composé est obtenu selon le mode opératoire décrit en 1.1. par réaction du chlorure de l'acide 4-méthoxybenzoique et de l'acide 2-bromo-5-méthyl-benzoïque après échange halogène-lithium. Il est utilisé brut dans la réaction suivante.

### 15.2. 4-(4-Méthoxy-phényl)-7-méthyl-2H-phthalazin-1-one

Ce composé est obtenu selon le mode opératoire décrit en 1.2. par réaction de l'acide 2-(4-méthoxybenzoyl)-5-méthyl-benzoïque non purifié avec de l'hydrate d'hydrazine.
LC/MS: MH⁺= 267 (tR = 7.29 minutes)
RMN ^{1H} δ en ppm (DMSO d 6) : 2.55 (s, 3H); 3.86 (s, 3H); 7.12 (d, J = 8.5 Hz, 2H); 7.53 (d, J = 8.5Hz, 2H); 7.62 (d, J = 8.2 Hz, 1H); 7.74 (dd, J₁ = 8.2 Hz, J₂ = 2 Hz, 1H); 8.16 (d, J = 2 Hz, 1H); 12.7 (s, 1H, NH).

### 15.3. 1-Chloro-4-(4-méthoxy-phényl)-7-méthyl-phthalazine

Ce composé est obtenu selon le mode opératoire décrit en 1.3. par réaction de la 4-(4-méthoxy-phényl)-7-méthyl-2H-phthalazin-1-one avec le chlorure de phosphoryle.
PF = 154°C (Mettler FP62)
LC/MS: MH⁺= 285 (tR = 8.36 minutes)
RMN ^{1H} δ en ppm (DMSO d 6) : 2.67 (s, 3H); 3.90 (s, 3H); 7.20 (d, J = 8.5 Hz, 2H); 7.70 (d, J = 8.5Hz, 2H); 7.95-7.99 (m, 2H); 8.18 (m, 1H).

### 15.4. 4-(4-méthoxyphényl)-7-méthyl-N-[1-(naphthalèn-2-ylméthyl)pipéridin-4-yl]phthalazin-1-amine

Ce composé est obtenu selon le mode opératoire décrit en 1.4. par réaction de la 1-chloro-4-(4-méthoxy-phényl)-7-méthyl-phthalazine avec la 4-amino-1-(naphthalèn-2-yl-méthyl)-pipéridine.

### Exemple 16: (composé n° 20)

### 4-Ethyl-7-méthoxy-N-[1-(naphthalèn-2-ylméthyl)pipéridin-4-yl]phthalazin-1-amine

### 16.1. Acide 5-méthoxy-2-propionyl-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-propionamide. Il est utilisé brut dans la réaction suivante.

### 16.2. 4-Ethyl-7-méthoxy-2H-phthalazin-1-one

Ce composé est obtenu selon le mode opératoire décrit en 1.2. par réaction de l'acide 5-méthoxy-2-propionyl-benzoïque non purifié avec de l'hydrate d'hydrazine.
LC/MS: MH⁺= 205 (tR = 6.32 minutes)
RMN ^{1H} δ en ppm (DMSO d 6) : 1.25 (t, J = 7.5 Hz, 3H); 2.92 (q, J = 7.5 Hz, 2H); 3.94 (s, 3H); 7.50 (dd, J₁ = 9 Hz, J₂ = 2.7 Hz, 1H); 7.60 (d, J = 2.7 Hz, 1H); 7.94 (d, J = 9 Hz, 1H); 12.38 (s, 1H, NH).

### 16.3. 1-Chloro-4-éthyl-7-méthoxy-phthalazine

Ce composé est obtenu selon le mode opératoire décrit en 1.3. par réaction de la 4-éthyl-7-méthoxy-2H-phthalazin-1-one avec le chlorure de phosphoryle.
PF = 191°C (Mettler FP62)
LC/MS: MH⁺= 223 (tR = 6.84 minutes)
RMN ^{1H} δ en ppm (DMSO d 6) : 1.36 (t, J = 7.5 Hz, 3H); 3.30 (q, J = 7.5 Hz, 2H); 4.04 (s, 3H); 7.50 (d, J = 2.7 Hz, 1H); 7.71 (dd, J₁ = 9 Hz, J₂ = 2.7 Hz, 1H); 8.30 (d, J = 9 Hz, 1H).

### 16.4. 4-Ethyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-éthyl-7-méthoxy-phthalazine avec la 4-amino-1-(naphthalèn-2-yl-méthyl)pipéridine.

### Exemple 17: (composé n° 21)

### 4-Benzyl-7-méthoxy-N-[1-(naphthalèn-2-ylméthyl)pipéridin-4-yl]phthalazin-1-amine

### 17.1 Acide 5-méthoxy-2-(phénylacétyl)-benzoïque

Ce composé est synthétisé selon la méthode décrite en 3.2. en faisant réagir de l'acide 2-bromo-5-méthoxy-benzoïque préalablement traité avec du n-butyl-lithium avec la phényl-N-méthoxy-N-acétamide. Il est utilisé brut dans la réaction suivante.

### 17.2. 4-Benzyl-7-méthoxy-2H-phthalazin-1-one

Ce composé est obtenu selon le mode opératoire décrit en 1.2. par réaction de l'acide 5-méthoxy-2-(phénylacétyl)-benzoïque non purifié avec de l'hydrate d'hydrazine.
LC/MS: MH⁺= 267 (tR = 6.50 minutes)
RMN ^{1H} δ en ppm (DMSO d 6) : 3.92 (s, 3H); 4.27 (s, 2H); 7.15-7.33 (m, 5H); 7.44 (dd, J₁ = 8.8 Hz, J₂ = 2.7 Hz, 1H); 7.66 (d, J = 2.7 Hz, 1H); 7.90 (d, J = 8.8 Hz, 1H); 12.52 (s, 1H, NH).

### 17.3. 4-Benzyl-1-chloro-7-méthoxy-phthalazine

Ce composé est obtenu selon le mode opératoire décrit en 1.3. par réaction de la 4-benzyl-7-méthoxy-2H-phthalazin-1-one avec le chlorure de phosphoryle.
LC/MS: MH⁺= 285 (tR = 7.34 minutes)
RMN ^{1H} δ en ppm (DMSO d 6) : 4.02 (s, 3H); 4.68 (s, 2H); 7.17-7.35 (m, 5H); 7.52 (d, J = 2.5 Hz, 1H); 7.69 (dd, J₁ = 9.2 Hz, J₂ = 2.5 Hz, 1H); 8.31 (d, J = 9.2 Hz, 1H).

### 17.4. 4-Benzyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 4-benzyl-1-chloro-7-méthoxy-phthalazine avec la 4-amino-1-(naphthalèn-2-yl-méthyl)pipéridine.

### Exemple 18: (composé n° 22)

### 4-Benzyl-5,7-diméthoxy-N-[1-(naphthalèn-2-ylméthyl)pipéridin-4-yl]phthalazin-1-amine

### 17.1 Acide 3,5-diméthoxy-2-(phénylacétyl)-benzoïque

Ce composé est synthétisé en faisant réagir du 3,5-diméthoxy-benzoate de méthyle avec du chlorure de phénylacétyle par la réaction de friedel-Crafts, suivie de la saponification de l'ester obtenu, selon la méthode décrite en 2.1. Il est utilisé brut dans la réaction suivante.

### 18.2. 4-Benzyl-5,7-diméthoxy-2H-phthalazin-1-one

Ce composé est obtenu selon le mode opératoire décrit en 1.2. par réaction de l'acide 3,5-diméthoxy-2-(phénylacétyl)-benzoïque non purifié avec de l'hydrate d'hydrazine.
LC/MS: MH⁺= 297 (tR = 7.98 minutes)
RMN ^{1H} δ en ppm (DMSO d 6) : 3.75 (s, 3H); 3.90 (s, 3H); 4.30 (s, 2H); 6.89 (d, J = 2.4 Hz, 1H); 7.12-7.16 (m, 3H); 7.20-7.30 (m, 3H); 12.51 (s, 1H, NH).

### 18.3. 4-Benzyl-1-chloro-5,7-diméthoxy-phthalazine

Ce composé est obtenu selon le mode opératoire décrit en 1.3. par réaction de la 4-benzyl-5,7-diméthoxy-2H-phthalazin-1-one avec le chlorure de phosphoryle.
RMN ^{1H} δ en ppm (DMSO d 6) : 3.88 (s, 3H); 4.0 (s, 3H); 4.71 (s, 2H); 7.03-7.28 (m, 7H).

### 18.4. 4-Benzyl-5,7-diméthoxy-N-[1-(naphthalèn-2-ylméthyl)pipéridin-4-yl]phthalazin-1-amine

Ce composé est obtenu selon le mode opératoire décrit en 1.4. par réaction de la 4-benzyl-1-chloro-5,7-diméthoxy-phthalazine avec la 4-amino-1-(naphthalèn-2-yl-méthyl)pipéridine.

### Exemple 19: (composé n° 23)

### 5,7-Diméthoxy-4-éthyl-N-[1-(naphthalèn-2-ylméthyl)pipéridin-4-yl]phthalazin-1-amine

### 19.1 Acide 3,5-diméthoxy-2-propionyl-benzoïque

Ce composé est synthétisé en faisant réagir du 3,5-diméthoxy-benzoate de méthyle avec du chlorure de propionyle par la réaction de friedel-Crafts, suivie de la saponification de l'ester obtenu, selon la méthode décrite en 2.1. Il est utilisé brut dans la réaction suivante.

### 19.2. 5,7-Diméthoxy-4-éthyl-2H-phthalazin-1-one

Ce composé est obtenu selon le mode opératoire décrit en 1.2. par réaction de l'acide 3,5-diméthoxy-2-propionyl-benzoïque non purifié avec de l'hydrate d'hydrazine.
RMN ^{1H} δ en ppm (DMSO d 6) : 1.15 (t, J = 7.2 Hz, 3H); 2.99 (q, J = 7.2 Hz, 2H); 3.92 (s, 3H); 3.94 (s, 3H); 6.99 (d, J = 2.7 Hz, 1H); 7.3 (d, J = 2.7 Hz, 1H); 12.35 (s, 1H, NH).

### 19.3. 1-Chloro-5,7-diméthoxy-4-éthyl-phthalazine

Ce composé est obtenu selon le mode opératoire décrit en 1.3. par réaction de la 5,7-diméthoxy-4-éthyl-2H-phthalazin-1-one avec le chlorure de phosphoryle.
RMN ^{1H} δ en ppm (DMSO d 6) : 1.26 (t, J = 7.2 Hz, 3H); 3.34 (q, J = 7.2 Hz, 2H); 4.0 (s, 3H); 4.03 (s, 3H); 7.12 (d, J = 2.4 Hz, 1H); 7.15 (d, J = 2.4 Hz, 1H).

### 19.4. 5,7-Diméthoxy-4-éthyl-N-[1-(naphthalèn-2-ylméthyl)pipéridin-4-yl]phthalazin-1-amine

Ce composé est obtenu selon le mode opératoire décrit en 1.4. par réaction de la 1-chloro-5,7-diméthoxy-4-éthyl-phthalazine avec la 4-amino-1-(naphthalèn-2-yl-méthyl)-pipéridine.

### Exemple 20: (composé n° 24)

### Chlorhydrate de la 7-méthoxy-4-méthoxyméthyl-N-[1-(naphthalèn-2-ylméthyl)pipéridin-4-yl]phthalazin-1-amine

### 20.1 Acide 5-méthoxy-2-(méthoxyacétyl)-benzoïque

Ce composé est synthétisé selon la méthode décrite en 3.2. en faisant réagir de l'acide 2-bromo-5-méthoxybenzoïque préalablement traité avec du n-butyl-lithium avec la 2,N-diméthoxy-N-méthyl-acétamide. Il est utilisé brut dans la réaction suivante.

### 20.2. 7-Méthoxy-4-méthoxyméthyl-2H-phthalazi n-1-one

Ce composé est obtenu selon le mode opératoire décrit en 1.2. par réaction de l'acide 5-méthoxy-2-(méthoxyacétyl)-benzoïque non purifié avec de l'hydrate d'hydrazine.
PF = 184°C (Mettler FP62)
LC/MS: MH⁺= 221 (tR = 5.33 minutes)
RMN ^{1H} δ en ppm (DMSO d 6) : 3.33 (s, 3H); 3.96 (s, 3H); 4.62 (s, 2H); 7.53 (dd, J₁ = 8.8 Hz, J₂ = 2.7 Hz, 1H); 7.66 (d, J = 2.7 Hz, 1H); 8.0 (d, J = 8.8 Hz, 1H); 12.61 (s, 1H, NH).

### 20.3. 1-Chloro-7-méthoxy-4-méthoxyméthyl-phthalazine

Ce composé est obtenu selon le mode opératoire décrit en 1.3. par réaction de la 7-méthoxy-4-méthoxyméthyl-2H-phthalazin-1-one avec le chlorure de phosphoryle.
RMN ^{1H} δ en ppm (DMSO d 6) : 3.33 (s, 3H); 4.06 (s, 3H); 5.02 (s, 2H); 7.56 (d, J = 2.7 Hz, 1H); 7.77 (dd, J₁ = 9.2 Hz, J₂ = 2.7 Hz, 1H); 8.35 (d, J = 9.2 Hz, 1H).

### 20.4. Chlorhydrate de la 7-méthoxy-4-méthoxyméthyl-N-[1-(naphthalèn-2-ylméthyl)pipéridin-4-yl]phthalazin-1-amine

Ce composé est obtenu selon le mode opératoire décrit en 1.4, par réaction de la 1-chloro-7-méthoxy-4-méthoxyméthyl-phthalazine avec la 4-amino-1-(naphthalèn-2-yl-méthyl)-pipéridine. Le chlorhydrate est obtenu après traitement avec une solution d'acide chlorhydrique dans l'isopropanol.

### Exemple 21: (composé n° 33)

### 4-Cyclopropyl-7-méthoxy-N-[1-(naphthalèn-2-ylméthyl)pipéridin-4-yl]phthalazin-1-amine

### 21.1 Acide 2-cyclopropanecarbonyl-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la cyclopropyl-N-méthoxy-N-méthyl-carboxamide. Par précipitation dans l'éther isopropylique, on obtient une poudre jaune.
PF = 117°C (Mettler FP62)
RMN ^{1H} δ en ppm (DMSO d 6) : 0.97-1.07 (m, 4H); 2.35-2.50 (m, 1H); 3.87(s, 3H); 7.15-7.20 (m, 2H); 7.71 (dd, J₁ = 8.8 Hz, J₂ = 2.5 Hz, 1H)(signal du COOH non visible).

### 21.2. 4-Cyclopropyl-7-méthoxy-2H-phthalazin-1-one

Ce composé est obtenu selon le mode opératoire décrit en 1.2. par réaction de l'acide 2-cyclopropanecarbonyl-5-méthoxy-benzoïque avec de l'hydrate d'hydrazine.
PF = 189°C (Mettler FP62)
RMN ^{1H} δ en ppm (DMSO d 6) : 0.85-1.02 (m, 4H); 2.38-2.45 (m, 1H); 3.96 (s, 3H); 7.53 (dd, J₁ = 9 Hz, J₂ = 2.7 Hz, 1H); 7.66 (d, J = 2.7 Hz, 1H); 8.22 (d, J = 9 Hz, 1H); 12.31 (s, 1H, NH).

### 21.3. 1-Chloro-4-cyclopropyl-7-méthoxy-phthalazine

Ce composé est obtenu selon le mode opératoire décrit en 1.3. par réaction de la 4-cyclopropyl-7-méthoxy-2H-phthalazin-1-one avec le chlorure de phosphoryle.
LC/MS: MH⁺= 235 (tR = 7.67 minutes)
RMN ^{1H} δ en ppm (DMSO d 6) : 1.13-1.26 (m, 4H); 2.80-2.95 (m, 1H); 4.05 (s, 3H); 7.51 (d, J = 2.5 Hz, 1H); 7.76 (dd, J₁ = 9.2 Hz, J₂ = 2.5 Hz, 1H); 8.57 (d, J = 9 Hz, 1H).

### 21.4. 4-Cyclopropyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-cyclopropyl-7-méthoxy-phthalazine avec la 4-amino-1-(naphthalèn-2-yl-méthyl)pipéridine.

### Exemple 22: (composé n°42)

### Dichlorhydrate de la 4-(1,3-benzodioxol-5-yl)-7-méthoxy-N-[1-(naphthalèn-2-ylméthyl)pipéridin-4-yl]phthalazin-1-amine

### 22.1 Acide 2-(1,3-Benzodioxol-5-yl)carbonyl-5-méthoxy-benzoïque

Ce composé est obtenu selon le mode opératoire décrit en 1.1. par réaction du chlorure de l'acide 1,3-benzodioxol-5-carboxylique et de l'acide 2-bromo-5-méthoxy-benzoïque après échange halogène-lithium. Il est utilisé brut dans la réaction suivante.

### 22.2. 4-(1,3-Benzodioxol-5-yl)-7-méthoxy-2H-phthalazin-1-one

Ce composé est obtenu selon le mode opératoire décrit en 1.2. par réaction de l'acide 2-(1,3-benzodioxol-5-yl)carbonyl-5-méthoxy-benzoïque non purifié avec de l'hydrate d'hydrazine.
PF= 271°C (Mettler FP62)
LC/MS: MH⁺= 297 (tR = 6.83 minutes)
RMN ^{1H} δ en ppm (DMSO d 6) : 3.95 (s, 3H); 6.12 (s, 2H); 7.01-7.12 (m, 3H); 7.46 (dd, J₁ = 9 Hz, J₂ = 2.7 Hz, 1H); 7.66 (d, J = 9 Hz, 1H); 7.71 (d, J = 2.7 Hz, 1H); 12.69 (s, 1H, NH).

### 22.3. 4-(1,3-Benzodioxol-5-yl)-1-chloro-7-méthoxy-phthalazine

Ce composé est obtenu selon le mode opératoire décrit en 1.3. par réaction de la 4-(1,3-benzodioxol-5-yl)-7-méthoxy-2H-phthalazin-1-one avec le chlorure de phosphoryle.
PF = 178°C (Mettler FP62)
LC/MS: MH⁺= 315 (tR = 7.81 minutes)
RMN ^{1H} δ en ppm (DMSO d 6) : 4.05 (s, 3H); 6.17 (s, 2H); 7.12-7.20 (m, 2H); 7.27 (d, J = 1.5 Hz, 1H); 7.58 (d, J = 2.7 Hz, 1H); 7.70 (dd, J₁ = 9.3 Hz, J₂ = 2.7 Hz, 1H); 8.01 (d, J = 9.3 Hz, 1H).

### 22.4. Dichlorhydrate de la 4-(1,3-benzodioxol-5-yl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 4-(1,3-benzodioxol-5-yl)-1-chloro-7-méthoxy-phthalazine avec la 4-amino-1-(naphthalèn-2-yl-méthyl)-pipéridine. Par traitement avec une solution d'acide chlorhydrique dans l'éther diéthylique, il se forme un précipité qui est filtré, lavé à l'éther diéthylique et séché sous pression réduite. Le chlorhydrate obtenu est sous la forme d'un solide blanc.

### Exemple 23: (composé n°43)

### 4-(4-Chlorophényl)-7-méthoxy-N-[1-(naphthalèn-2-ylméthyl)pipéridin-4-yl]phthalazin-1-amine

### 23.1. Acide 2-(4-chlorobenzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec 4-chloro-N-méthoxy-N-méthyl-benzamide. Il est utilisé brut dans la réaction suivante.

### 23.2. 4-(4-Chlorophényl)-7-méthoxy-2H-phthalazin-1-one

Ce composé est obtenu selon le mode opératoire décrit en 1.2. par réaction de l'acide 2-(4-chlorobenzoyl)-5-méthoxy-benzoïque non purifié avec de l'hydrate d'hydrazine.
PF= 274°C (Mettler FP62)
LC/MS: MH⁺= 287 (tR = 7.20 minutes)
RMN ^{1H} δ en ppm (DMSO d 6) : 3.97 (s, 3H); 7.48 (dd, J₁ = 8.5 Hz, J₂ = 2.5 Hz, 1H); 7.61-7.65 (m, 5H); 7.74 (d, J = 2.5 Hz, 1H); 12.82 (s, 1H, NH).

### 23.3. 1-Chloro-4-(4-chlorophényl)-7-méthoxy-phthalazine

Ce composé est obtenu selon le mode opératoire décrit en 1.3. par réaction de la 4-(4-chlorophényl)-7-méthoxy-2H-phthalazin-1-one avec le chlorure de phosphoryle.
PF = 199°C (Mettler FP62)
LC/MS: MH⁺= 305 (tR = 8.05 minutes)
RMN ^{1H} δ en ppm (DMSO d 6) : 4.07 (s, 3H); 7.62 (d, J = 2.5 Hz, 1H); 7.67-7.77 (m, 5H); 7.96 (d, J = 9 Hz, 1H).

### 23.4. 4-(4-Chlorophényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(4-chlorophényl)-7-méthoxy-phthalazine avec la 4-amino-1-(naphthalèn-2-yl-méthyl)-pipéridine.

### Exemple 24: (composé n°45)

### 7-Méthoxy-4)-N-[1-(naphthalèn-2-ylméthyl)pipéridin-4-yl]-(4-trifluorométhylphényl-phthalazin-1-amine

### 24.1. Acide 5-méthoxy-2-(4-trifluorométhyl-benzoyl)-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-4-trifluorométhyl-benzamide. Il est utilisé brut dans la réaction suivante.

### 24.2. 7-Méthoxy-4-(4-trifluorométhyl-phényl)-2H-phthalazin-1-one

Ce composé est obtenu selon le mode opératoire décrit en 1.2. par réaction de l'acide 5-méthoxy-2-(4-trifluorométhyl-benzoyl)-benzoïque non purifié avec de l'hydrate d'hydrazine.
PF= 264°C (Köffler)
LC/MS: MH⁺= 321 (tR = 8.14 minutes)
RMN ^{1H} δ en ppm (DMSO d 6) : 3.96 (s, 3H); 7.46 (dd, J₁ = 9 Hz, J₂ = 2.7 Hz, 1H); 7.62 (d, J = 9 Hz, 1H); 7.74 (d, J = 2.7 Hz, 1H); 7.82 (d, J = 8.2, 2H); 7.92 (d, J = 8.2, 2H); 12.87 (s, 1H, NH).

### 24.3. 1-Chloro-7-méthoxy-4-(4-trifluorométhyl-phényl)-phthalazine

Ce composé est obtenu selon le mode opératoire décrit en 1.3. par réaction de la 7-méthoxy-4-(4-trifluorométhyl-phényl)-2H-phthalazin-1-one avec le chlorure de phosphoryle.
LC/MS: MH⁺= 338 (tR = 8.68 minutes)
RMN ^{1H} δ en ppm (DMSO d 6) : 4.07 (s, 3H); 7.63 (d, J = 2.5 Hz, 1H); 7.71 (dd, J₁ = 9 Hz, J₂ = 2.5 Hz, 1H); 7.91-8.01 (m, 5H).

### 24.4 7-Méthoxy-4)-N-[1-(naphthalèn-2-ylméthyl)pipéridin-4-yl]-(4-trifluorométhylphényl-phthalazin-1-amine

Ce composé est obtenu selon le mode opératoire décrit en 1.4. par réaction de la 1-chloro-7-méthoxy-4-(4-trifluorométhyl-phényl)-phthalazine avec la 4-amino-1-(naphthalèn-2-yl-méthyl)-pipéridine.

### Exemple 25: (composé n° 51)

### Dichlorhydrate de la 4-cyclopentyl-7-méthoxy-N-[1-(naphthalèn-2-ylméthyl)pipéridin-4-yl]phthalazin-1-amine

### 25.1 Acide 2-cyclopentanecarbonyl-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la cyclopentyl-N-méthoxy-N-méthyl-carboxamide. Il est utilisé brut dans la réaction suivante.

### 25.2. 4-Cyclopentyl-7-méthoxy-2H-phthalazin-1-one

Ce composé est obtenu selon le mode opératoire décrit en 1.2. par réaction de l'acide 2-cyclopentanecarbonyl-5-méthoxy-benzoïque non purifié avec de l'hydrate d'hydrazine.
PF = 232°C (Mettler FP62)
RMN ^{1H} δ en ppm (DMSO d 6) : 1.65-1.95 (m, 6H); 1.95-2.10 (m, 2H); 3.55-3.70 (m, 1H); 3.95 (s, 3H); 7.50 (dd, J₁ = 9 Hz, J₂ = 2.7 Hz, 1H); 7.67 (d, J = 2.7 Hz, 1H); 8.03 (d, J = 9 Hz, 1H); 12.37 (s, 1H, NH).

### 25.3. 1-Chloro-4-cyclopentyl-7-méthoxy-phthalazine

Ce composé est obtenu selon le mode opératoire décrit en 1.3. par réaction de la 4-cyclopentyl-7-méthoxy-2H-phthalazin-1-one avec le chlorure de phosphoryle. Le produit est trop impur pour une description RMN. Il est engagé tel quel dans la réaction d'amination suivante.
LC/MS: MH⁺= 263 (tR = 9.04 minutes)

### 25.4. Dichlorhydrate de la 4-cyclopentyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-cyclopentyl-7-méthoxy-phthalazine impure avec la 4-amino-1-(naphthalèn-2-yl-méthyl)-pipéridine. Le dichlorhydrate est obtenu après traitement avec une solution d'acide chlorhydrique dans l'isopropanol, puis une cristallisation dans l'éther diisopropylique.

### Exemple 26: (composé n°66)

### 7-Méthoxy-4-(4-méthylphényl)-N-[1-(naphthalèn-2-ylméthyl)pipéridin-4-yl]phthalazin-1-amine

### 26.1. Acide 5-méthoxy-2-(4-méthyl-benzoyl)-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-4-méthylbenzamide. Il est utilisé brut dans la réaction suivante.

### 26.2. 7-Méthoxy-4-(4-méthyl-phényl)-2H-phthalazin-1-one

Ce composé est obtenu selon le mode opératoire décrit en 1.2. par réaction de l'acide 5-méthoxy-2-(4-méthyl-benzoyl)-benzoïque non purifié avec de l'hydrate d'hydrazine.
PF= 223°C (Mettler FP62)
LC/MS: MH⁺= 267 (tR = 8.07 minutes)
RMN ^{1H} δ en ppm (DMSO d 6) : 2.40 (s, 3H); 3.95 (s, 3H); 7.35 (d, J = 7.8 Hz, 2H); 7.44-7.48 (m, 3H); 7.63 (d, J = 9 Hz, 1H); 7.72 (d, J = 2.7 Hz, 1H); 12.71 (s, 1H, NH).

### 26.3. 1-Chloro-7-méthoxy-4-(4-méthyl-phényl)-phthalazine

Ce composé est obtenu selon le mode opératoire décrit en 1.3. par réaction de la 7-méthoxy-4-(4-méthyl-phényl)-2H-phthalazin-1-one avec le chlorure de phosphoryle.
PF= 183°C (Mettler FP62)
LC/MS: MH⁺= 285 (tR = 7.76 minutes)
RMN ^{1H} δ en ppm (DMSO d 6) : 2.46 (s, 3H); 4.06 (s, 3H); 7.43 (d, J = 8.5 Hz, 2H); 7.58-7.63 (m, 3H); 7.70 (dd, J₁ = 9 Hz, J₂ = 2.5 Hz, 1H); 7.97 (d, J = 9 Hz, 1H).

### 26.4. 7-Méthoxy-4-(4-méthylphényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-(4-méthyl-phényl)-phthalazine avec la 4-amino-1-(naphthalèn-2-yl-méthyl)-pipéridine.

### Exemple 27: (composé n° 67)

### 4-Butyl-7-méthoxy-N-[1-(naphthalèn-2-ylméthyl)pipéridin-4-yl]phthalazin-1-amine

### 27.1 Acide 5-méthoxy-2-pentanoyl-benzoïque

Ce composé est synthétisé selon la méthode décrite en 3.2. en faisant réagir de l'acide 2-bromo-5-méthoxybenzoïque préalablement traité avec du n-butyl-lithium avec la butyl-N-méthoxy-N-méthyl-carboxamide. Il est utilisé brut dans la réaction suivante.

### 27.2. 4-Butyl-7-méthoxy-2H-phthalazin-1-one

Ce composé est obtenu selon le mode opératoire décrit en 1.2. par réaction de l'acide 5-méthoxy-2-pentanoyl-benzoïque non purifié avec de l'hydrate d'hydrazine.
PF = 175°C (Mettler FP62)
LC/MS: MH⁺= 233 (tR = 7.75 minutes)
RMN ^{1H} δ en ppm (DMSO d 6) : 0.92 (t, J = 7.3 Hz, 3H); 1.32-1.47 (m, 2H); 1.59-1.72 (m, 2H); 2.85-2.92 (m, 2H); 3.94 (s, 3H); 7.49 (dd, J₁ = 9 Hz, J₂ = 2.7 Hz, 1H); 7.66 (d, J = 2.7 Hz, 1H); 7.93 (d, J = 9 Hz, 1H); 12.37 (s, 1H, NH).

### 27.3. 4-Butyl-1-chloro-7-méthoxy-phthalazine

Ce composé est obtenu selon le mode opératoire décrit en 1.3. par réaction de la 4-butyl-7-méthoxy-2H-phthalazin-1-one avec le chlorure de phosphoryle.
LC/MS: MH⁺= 251 (tR = 7.10 minutes)
RMN ^{1H} δ en ppm (DMSO d 6) : 0.94 (t, J = 7.3 Hz, 3H); 1.35-1.50 (m, 2H); 1.70-1.82 (m, 2H); 3.24-3.32 (m, 2H); 4.04 (s, 3H); 7.51 (d, J = 2.5 Hz, 1H); 7.72 (dd, J₁ = 9 Hz, J₂ = 2.5 Hz, 1H); 8.31 (d, J = 9 Hz, 1H).

### 27.4. 4-Butyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 4-butyl-1-chloro-7-méthoxy-phthalazine avec la 4-amino-1-(naphthalèn-2-yl-méthyl)-pipéridine.

### Exemple 28: (composé n° 71)

### 4-Cyclopropylméthyl-7-méthoxy-N-[1-(naphthalèn-2-ylméthyl)pipéridin-4-yl]phthalazin-1-amine

### 28.1 Acide 2-(2-cyclopropyl-acétyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 2-cyclopropyl-N-méthoxy-N-méthyl-acétamide. Il est utilisé brut dans la réaction suivante.

### 28.2. 4-Cyclopropylméthyl-7-méthoxy-2H-phthalazin-1-one

Ce composé est obtenu selon le mode opératoire décrit en 1.2. par réaction de l'acide 2-(2-cyclopropyl-acétyl)-5-méthoxy-benzoïque non purifié avec de l'hydrate d'hydrazine.
RMN ^{1H} δ en ppm (DMSO d 6) : 0.23-0.28 (m, 2H); 0.46-0.52 (m, 2H); 1.09-1.13 (m, 1H); 2.83 (d, J = 6.7 Hz, 2H); 3.96 (s, 3H); 7.52 (dd, J₁ = 8.7 Hz, J₂ = 2.7 Hz, 1H); 7.68 (d, J = 2.7 Hz, 1H); 7.99 (d, J = 8.7 Hz, 1H); 12.41 (s, 1H, NH).

### 28.3. 1-Chloro-4-cyclopropylméthyl-7-méthoxy-phthalazine

Ce composé est obtenu selon le mode opératoire décrit en 1.3. par réaction de la 4-cyclopropylméthyl-7-méthoxy-2H-phthalazin-1-one avec le chlorure de phosphoryle.
LC/MS: MH⁺= 249 (tR = 6.91 minutes)

### 28.4. 4-Cyclopropylméthyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-cyclopropylméthyl-7-méthoxy-phthalazine avec la 4-amino-1-(naphthalèn-2-yl-méthyl)-pipéridine.
LC/MS: MH⁺= 453 (tR = 5.45 minutes)
RMN ^{1H} δ en ppm (DMSO d 6) : 0.23-0.26 (m, 2H); 0.43-0.47 (m, 2H); 1.08-1.16 (m, 1H); 1.60-1.80 (m, 2H); 2.03-2.30 (m, 4H); 2.90-3.0 (m, 2H); 2.95 (d, J = 6.6 Hz, 2H); 3.74 (s, 2H); 3.96 (s, 3H); 4.15-4.25 (m, 1H); 6.88 (d, J = 7 Hz, 1H, NH); 7.42-7.58 (m, 4H); 7.73 (d, J = 2.5 Hz, 1H); 7.83-7.94 (m, 4H); 8.03 (d, J = 9 Hz, 1H).

### Exemple 29: (composé n°82).

### 4-(4-Fluorophényl)-7-méthoxy-N-[1-(naphthalèn-2-ylméthyl)pipéridin-4-yl]phthalazin-1-amine

### 29.1. Acide 2-(4-fluorobenzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 4-fluoro-N-méthoxy-N-méthyl-benzamide. Il est utilisé brut dans la réaction suivante.

### 29.2. 4-(4-Fluorophényl)-7-méthoxy-2H-phthalazin-1-one

Ce composé est obtenu selon le mode opératoire décrit en 1.2. par réaction de l'acide 2-(4-fluorobenzoyl)-5-méthoxy-benzoïque non purifié avec de l'hydrate d'hydrazine.
PF= 256°C (Mettler FP62)
LC/MS: MH⁺= 271 (tR = 6.51 minutes)
RMN ^{1H} δ en ppm (DMSO d 6) : 3.97 (s, 3H); 7.35-7.43 (m, 2H), 7.48 (dd, J₁ = 9 Hz, J₂ = 2.7 Hz, 1H); 7.59-7.67 (m, 3H); 7.74 (d, J = 2.7 Hz, 1H); 12.80 (s, 1H, NH).

### 29.3. 1-Chloro-4-(4-fluorophényl)-7-méthoxy-phthalazine

Ce composé est obtenu selon le mode opératoire décrit en 1.3. par réaction de la 4-(4-fluorophényl)-7-méthoxy-2H-phthalazin-1-one avec le chlorure de phosphoryle.
LC/MS: MH⁺= 289 (tR = 8.58 minutes)
RMN ^{1H} δ en ppm (DMSO d 6) : 4.06 (s, 3H); 7.42-7.51 (m, 2H), 7.60 (d, J = 2.5 Hz, 1H); 7.69-7.80 (m, 3H); 7.95 (d, J = 9 Hz, 1H).

### 29.4. 4-(4-Fluorophényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(4-fluorophényl)-7-méthoxy-phthalazine avec la 4-amino-1-(naphthalèn-2-yl-méthyl)-pipéridine.

### Exemple 30: (composé n°88)

### 7-Méthoxy-4-(4-méthoxyméthyl-phényl)-N-[1-(naphthalèn-2-ylméthyl)pipéridin-4-yl]phthalazin-1-amine

### 30.1. Acide 5-méthoxy-2-(4-méthoxyméthylbenzoyl)-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-4-méthoxyméthyl-N-méthyl-benzamide. Le produit est cristallisé dans l'éther diisopropylique.
LC/MS: MH⁺= 301 (tR = 6.60 minutes)
RMN ^{1H} δ en ppm (DMSO d 6) : 3.32 (s, 3H); 3.89 (s, 3H); 4.49 (s, 2H); 7.26 (dd, J₁ = 8.5 Hz, J₂ = 2.5 Hz, 1H); 7.40-7.45 (m, 4H); 7.63 (d, J = 8.2 Hz, 2H); 13.1 (s, 1H, COOH).

### 30.2. 7-Méthoxy-4-(4-méthoxyméthyl-phényl)-2H-phthalazin-1-one

Ce composé est obtenu selon le mode opératoire décrit en 1.2. par réaction de l'acide 5-méthoxy-2-(4-méthoxyméthylbenzoyl)-benzoïque avec de l'hydrate d'hydrazine.
PF= 201°C (Mettler FP62)
LC/MS: MH⁺= 297 (tR = 6.54 minutes)
RMN ^{1H} δ en ppm (DMSO d 6) : 3.36 (s, 3H); 3.96 (s, 3H); 4.52 (s, 2H); 7.45-7.58 (m, 5H); 7.64 (d, J = 9 Hz, 1H); 7.74 (d, J = 2.7 Hz, 1H); 12.78 (s, 1H, NH).

### 30.3. 1-Chloro-7-méthoxy-4-(4-méthoxyméthyl-phényl)-phthalazine

Ce composé est obtenu selon le mode opératoire décrit en 1.3. par réaction de la 7-méthoxy-4-(4-méthoxyméthyl-phényl)-2H-phthalazin-1-one avec le chlorure de phosphoryle.
LC/MS: MH⁺= 315 (tR = 8.26 minutes)
RMN ^{1H} δ en ppm (DMSO d 6) : 3.38 (s, 3H); 4.06 (s, 3H); 4.56 (s, 2H); 7.56 (d, J = 8.4 Hz, 2H); 7.62 (d, J = 2.7 Hz, 1H); 7.68-7.74 (m, 3H); 7.97 (d, J = 9 Hz, 1H).

Au cours de cette réaction, le produit majoritaire est le 1-chloro-4-(4-chlorométhyl-phényl-7-méthoxy-phthalazine.
LC/MS: MH⁺= 319 (tR = 9.06 minutes) dichloré
RMN ^{1H} δ en ppm (DMSO d 6) : 4.06 (s, 3H); 4.90 (s, 2H); 7.61 (d, J = 2.7 Hz, 1H); 7.71-7.75 (m, 5H); 7.95 (d, J = 9 Hz, 1H).

### 30.4. 7-Méthoxy-4-(4-méthoxyméthyl-phényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-(4-méthoxyméthyl-phényl)-phthalazine avec la 4-amino-1-(naphthalèn-2-yl-méthyl)-pipéridine.

### Exemple 31: (composé n°105)

### Trichlorhydrate de la 4-[(4-diéthylaminométhyl)phényl]-7-méthoxy-N-[1-(naphthalèn-2-ylméthyl)pipéridin-4-yl]phthalazin-1-amine

### 31.1. 1-Chloro-4-(4-diéthylaminométhyl-phényl)-7-méthoxy-phthalazine

Une solution de 319 mg (1 mmol) de 1-chloro-4-(4-chlorométhyl-phényl)-7-méthoxy-phthalazine (synthétisée en 30.3) dans 3 mL d'éthanol est additionnée de 280 mg (2 mmol) de carbonate de potassium et 0.3 mL de diéthylamine. Le mélange est agité à 85°C pendant 4 heures. L'éthanol est évaporé et le résidu est repris par de l'eau et extrait au dichlorométhane. L'extrait organique est lavée à l'eau salée, séchée sur sulfate de sodium anhydre, puis évaporée sous pression réduite. Le résidu obtenu est purifié par chromatographie sur colonne de gel de silice (solvant: dichlorométhane/méthanol 95/5 à 85/15 (v/v)). On obtient 287 mg de produit (rendement 80%).
LC/MS: MH⁺= 356 (tR = 5.38 minutes)

### 31.2 Trichlorhydrate de la 4-[(4-diéthylaminométhyl)phényl]-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-Chloro-4-(4-diéthylaminométhyl-phényl)-7-méthoxy-phthalazine avec la 4-amino-1-(naphthalèn-2-yl-méthyl)-pipéridine. Le trichlorhydrate est obtenu après traitement avec une solution d'acide chlorhydrique dans l'éther diéthylique, concentration puis cristallisation.

### Exemple 32: (composé n° 95)

### 7-Méthoxy-4-méthyl-N-[1-(naphthalèn-2-ylméthyl)pipéridin-4-yl]phthalazin-1-amine

### 32.1 Acide 2-acétyl-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-acétamide. Il est utilisé brut dans la réaction suivante.

### 32.2. 7-Méthoxy-4-méthyl-2H-phthalazin-1-one

Ce composé est obtenu selon le mode opératoire décrit en 1.2. par réaction de l'acide 2-acétyl-5-méthoxy-benzoïque non purifié avec de l'hydrate d'hydrazine.
PF = 202°C (Mettler FP62)
LC/MS: MH⁺= 191 (tR = 5.73 minutes)
RMN ^{1H} δ en ppm (DMSO d 6) : 2.50 (s, 3H); 3.96 (s, 3H); 7.52 (dd, J₁ = 8.8 Hz, J₂ = 2.7 Hz, 1H); 7.66 (d, J = 2.7 Hz, 1H); 7.90 (d, J = 8.8 Hz, 1H); 12.35 (s, 1H, NH).

### 32.3. 1-Chloro-7-méthoxy-4-méthyl-phthalazine

Ce composé est obtenu selon le mode opératoire décrit en 1.3. par réaction de la 7-méthoxy-4-méthyl-2H-phthalazin-1-one avec le chlorure de phosphoryle.
LC/MS: MH⁺= 209 (tR = 6.26 minutes)
RMN ^{1H} δ en ppm (DMSO d 6) : 2.92 (s, 3H); 4.06 (s, 3H); 7.54 (d, J = 2.5 Hz, 1H); 7.77 (dd, J₁ = 9.2 Hz, J₂ = 2.5 Hz, 1H); 8.31 (d, J = 9.2 Hz, 1H).

### 32.4. 7-Méthoxy-4-méthyl-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-méthyl-phthalazine avec la 4-amino-1-(naphthalèn-2-yl-méthyl)pipéridine.

### Exemple 33: (composé n°97)

### Dichlorhydrate de la 4-(2,5-diméthoxyphényl)-7-méthoxy-N-[1-(naphthalèn-2-ylméthyl)pipéridin-4-yl]phthalazin-1-amine

### 33.1. Acide 2-(2,5-diméthoxy-benzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 2,5-diméthoxy-N-méthoxy-N-méthyl-benzamide. Il est cristallisé dans l'éther diisopropylique.
PF= 122°C (Mettler FP62)
LC/MS: MNa⁺= 339 (tR = 7.40 minutes)
RMN ^{1H} δ en ppm (DMSO d 6) : 3.50 (s, 3H); 3.73 (s, 3H); 3.86 (s, 3H); 7.02-7.14 (m, 4H); 7.22 (d, J = 2.4 Hz, 1H); 7.29 (d, J = 8.4 Hz, 1H); 12.96 (s, 1H, COOH).

### 33.2. 4-(2,5-Diméthoxyphényl)-7-méthoxy-2H-phthalazin-1-one

Ce composé est obtenu selon le mode opératoire décrit en 1.2. par réaction de l'acide 2-(2,5-diméthoxy-benzoyl)-5-méthoxy-benzoïque avec de l'hydrate d'hydrazine.
PF= 208°C (Mettler FP62)
LC/MS: MH⁺= 313 (tR = 7.42 minutes)
RMN ^{1H} δ en ppm (DMSO d 6) : 3.62 (s, 3H); 3.74 (s, 3H); 3.94 (s, 3H); 6.91 (d, J = 3 Hz, 1H); 7.05-7.15 (m, 2H); 7.22 (d, J = 9 Hz, 1H); 7.40 (dd, J₁ = 9 Hz, J₂ = 2.7 Hz, 1H); 7.67 (d, J = 2.7 Hz, 1H); 12.67 (s, 1H, NH).

### 33.3. 1-Chloro-4-(2,5-diméthoxyphényl)-7-méthoxy-phthalazine

Ce composé est obtenu selon le mode opératoire décrit en 1.3. par réaction de la 4-(2,5-diméthoxyphényl)-7-méthoxy-2H-phthalazin-1-one avec le chlorure de phosphoryle.
PF= 140°C (Mettler FP62)
LC/MS: MH⁺= 331 (tR = 8.36 minutes)
RMN ^{1H} δ en ppm (DMSO d 6) : 3.61 (s, 3H); 3.77 (s, 3H); 4.05 (s, 3H); 7.0 (d, J = 2.7 Hz, 1H); 7.13-7.21 (m, 2H); 7.55-7.59 (m, 2H); 7.65 (dd, J₁ = 9 Hz, J₂ = 2.7 Hz, 1H).

### 33.4. Dichlorhydrate de la 4-(2,5-diméthoxyphényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(2,5-diméthoxyphényl)-7-méthoxy-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 34: (composé n°102)

### Dichlorhydrate de la 4-(2,4-diméthoxyphényl)-7-méthoxy-N-[1-(naphthalèn-2-ylméthyl)pipéridin-4-yl]phthalazin-1-amine

### 34.1. Acide 2-(2,4-diméthoxy-benzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 2,4-diméthoxy-N-méthoxy-N-méthyl-benzamide. Il est utilisé brut dans la réaction suivante.

### 34.2. 4-(2,4-Diméthoxyphényl)-7-méthoxy-2H-phthalazin-1-one

Ce composé est obtenu selon le mode opératoire décrit en 1.2. par réaction de l'acide 2-(2,4-diméthoxy-benzoyl)-5-méthoxy-benzoïque avec de l'hydrate d'hydrazine.
PF= 256°C (Mettler FP62)
LC/MS: MH⁺= 313 (tR = 7.49 minutes)
RMN ^{1H} δ en ppm (DMSO d 6) : 3.69 (s, 3H); 3.86 (s, 3H); 3.94 (s, 3H); 6.65-6.75 (m, 2H); 7.21-7.26 (m, 2H); 7.37-7.43 (m, 1H); 7.66-7.68 (m, 1H); 12.64 (s, 1H, NH).

### 34.3. 1-Chloro-4-(2,4-diméthoxyphényl)-7-méthoxy-phthalazine

Ce composé est obtenu selon le mode opératoire décrit en 1.3. par réaction de la 4-(2,4-diméthoxyphényl)-7-méthoxy-2H-phthalazin-1-one avec le chlorure de phosphoryle.
PF= 177°C (Mettler FP62)
LC/MS: MH⁺= 331 (tR = 8.31 minutes)
RMN ^{1H} δ en ppm (DMSO d 6) : 3.68 (s, 3H); 3.89 (s, 3H); 4.05 (s, 3H); 6.75 (dd, J₁ = 8.3 Hz, J₂ = 2.5 Hz, 1H); 6.80 (d, J = 2.5 Hz, 1H); 7.35 (d, J = 8.3 Hz, 1H); 7.54-7.64 (m, 3H).

### 34.4. Dichlorhydrate de la 4-(2,4-diméthoxyphényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(2,4-diméthoxyphényl)-7-méthoxy-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 35: (composé n°103)

### Dichlorhydrate de la 4-(3,5-diméthoxyphényl)-7-méthoxy-N-[1-(naphthalèn-2-ylméthyl)pipéridin-4-yl]phthalazin-1-amine

### 35.1. Acide 2-(3,5-diméthoxy-benzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 3,5-diméthoxy-N-méthoxy-N-méthyl-benzamide. Il est cristallisé dans l'éther diisopropylique.
PF= 171°C (Mettler FP62)
LC/MS: MH⁺= 317 (tR = 7.91 minutes)
RMN ^{1H} δ en ppm (DMSO d 6) : 3.76 (s, 6H); 3.89 (s, 3H); 6.71-6.77 (m, 3H); 7.25 (dd, J₁ = 8.5 Hz, J₂ = 2.5 Hz, 1H); 7.38-7.46 (m, 2H); 13.18 (s, 1H, COOH).

### 35.2. 4-(3,5-Diméthoxyphényl)-7-méthoxy-2H-phthalazin-1-one

Ce composé est obtenu selon le mode opératoire décrit en 1.2. par réaction de l'acide 2-(3,5-diméthoxy-benzoyl)-5-méthoxy-benzoïque avec de l'hydrate d'hydrazine.
PF= 237°C (Mettler FP62)
LC/MS: MH⁺= 313 (tR = 7.74 minutes)
RMN ^{1H} δ en ppm (DMSO d 6) : 3.81 (s, 6H); 3.97 (s, 3H); 6.65-6.70 (m, 3H); 7.47 (dd, J₁ = 8.8 Hz, J₂ = 2.7 Hz, 1H); 7.68-7.74 (m, 2H); 12.74 (s, 1H, NH).

### 35.3. 1-Chloro-4-(3,5-diméthoxyphényl)-7-méthoxy-phthalazine

Ce composé est obtenu selon le mode opératoire décrit en 1.3. par réaction de la 4-(3,5-diméthoxyphényl)-7-méthoxy-2H-phthalazin-1-one avec le chlorure de phosphoryle.
PF= 189°C (Mettler FP62)
LC/MS: MH⁺= 331 (tR = 8.73 minutes)
RMN ^{1H} δ en ppm (DMSO d 6) : 3.84 (s, 6H); 4.07 (s, 3H); 6.75 (m, 1H); 6.82 (m, 2H); 7.61 (d, J = 2.5 Hz, 1H); 7.72 (dd, J₁ = 9 Hz, J₂ = 2.5 Hz, 1H); 8.02 (d, J = 9 Hz, 1H).

### 35.4. Dichlorhydrate de la 4-(3,5-diméthoxyphényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(3,5-diméthoxyphényl)-7-méthoxy-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 36: (composé n° 104)

### Dichlorhydrate de la 7-méthoxy-4-N-[1-(naphthalèn-2-ylméthyl)pipéridin-4-yl]-(2-phényléthyl)-phthalazin-1-amine

### 36.1 Acide 5-méthoxy-2-(2-phénylpropionyl)-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-2-phénylpropionamide. Il est utilisé brut dans la réaction suivante.

### 36.2. 7-Méthoxy-4-(2-phényléthyl)-2H-phthalazin-1-one

Ce composé est obtenu selon le mode opératoire décrit en 1.2. par réaction de l'acide 5-méthoxy-2-(2-phénylpropionyl)-benzoïque non purifié avec de l'hydrate d'hydrazine.
PF= 189°C (Mettler FP62)
LC/MS: MH⁺= 281 (tR = 6.62 minutes)
RMN ^{1H} δ en ppm (DMSO d 6) : 2.98-3.04 (m, 2H); 3.16-3.22 (m, 2H); 3.94 (s, 3H); 7.14-7.30 (m, 5H); 7.48 (dd, J₁ = 9 Hz, J₂ = 2.7 Hz, 1H); 7.66 (d, J = 2.7 Hz, 1H); 7.99 (d, J = 9 Hz, 1H); 12.40 (s, 1H, NH).

### 36.3. 1-Chloro-7-méthoxy-4-(2-phényléthyl)-phthalazine

Ce composé est obtenu selon le mode opératoire décrit en 1.3. par réaction de la 7-méthoxy-4-(2-phényléthyl)-2H-phthalazin-1-one avec le chlorure de phosphoryle.
LC/MS: MH⁺= 299 (tR = 8.94 minutes)
RMN ^{1H} δ en ppm (DMSO d 6) : 3.10-3.15 (m, 2H); 3.54-3.60 (m, 2H); 4.03 (s, 3H); 7.17-7.31 (m, 5H); 7.49 (d, J = 2.5 Hz, 1H); 7.67 (dd, J₁ = 9 Hz, J₂ = 2.5 Hz, 1H); 8.31 (d, J = 9 Hz, 1H).

### 36.4. Dichlorhydrate de la 7-méthoxy-4-N-[1-(naphthalèn-2-ylméthyl)pipéridin-4-yl]-(2-phényléthyl)-phthalazin-1-amine

Ce composé est obtenu selon le mode opératoire décrit en 1.4. par réaction de la 1-chloro-7-méthoxy-4-(2-phényléthyl)-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 37: (composé n°106)

### 7-Fluoro-4-(4-fluorophényl)-N-[1-(naphthalèn-2-ylméthyl)pipéridin-4-yl]phthalazin-1-amine

### 37.1. 7-Fluoro-4-(4-fluorophényl)-2H-phthalazin-1-one

Ce composé est obtenu selon le mode opératoire décrit en 1.2. par réaction de l'acide 5-fluoro-2-(4-fluorobenzoyl)-benzoïque avec de l'hydrate d'hydrazine.
PF= 206°C (Mettler FP62)
LC/MS: MH⁺= 259 (tR = 7.64 minutes)

### 37.2. 1-Chloro-7-fluoro-4-(4-fluorophényl)-phthalazine

Ce composé est obtenu selon le mode opératoire décrit en 1.3. par réaction de la 7-fluoro-4-(4-Fluorophényl)-2H-phthalazin-1-one avec le chlorure de phosphoryle.
PF= 195°C (Mettler FP62)
LC/MS: MH⁺= 277 (tR = 7.90 minutes)
RMN ^{1H} δ en ppm (DMSO d 6) : 7.45-7.52 (m, 2H), 7.78-7.84 (m, 2H); 8.03-8.19 (m, 3H).

### 37.3. 7-Fluoro-4-(4-fluorophényl)-N-[1-(naphthalèn-2-ylméthyl)pipéridin-4-yl]phthalazin-1-amine

Ce composé est obtenu selon le mode opératoire décrit en 1.4. par réaction de la 1-chloro-7-fluoro-4-(4-fluorophényl)-phthalazine avec la 4-amino-1-(naphthalèn-2-yl-méthyl)-pipéridine.

### Exemple 38: (composé n° 109)

### Trichlorhydrate de la 7-méthoxy-N-[1-(naphthalèn-2-ylméthyl)pipéridin-4-yl]-4-(pyridin-3-yl)-phthalazin-1-amine

### 38.1 Acide 5-méthoxy-2-pyridin-3-yl-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-nicotinamide. Après précipitation en phase aqueuse à pH 4 puis filtration et séchage une poudre beige est obtenue.
PF= 178°C (Mettler FP62)
LC/MS: MH⁺= 258 (tR = 5.59 minutes)
RMN ^{1H} δ en ppm (DMSO d 6) : 3.90 (s, 3H); 7.29 (dd, J₁ = 9 Hz, J₂ = 2.7 Hz, 1H); 7.43-7.56 (m, 3H); 7.97 (m, 1H); 8.73-8.77 (m, 2H); 13.31 (s, 1H, COOH).

### 38.2. 7-Méthoxy-4-(pyridin-3-yl)-2H-phthalazin-1-one

Ce composé est obtenu selon le mode opératoire décrit en 1.2. par réaction de l'acide 5-méthoxy-2-pyridin-3-yl-benzoïque avec de l'hydrate d'hydrazine.
PF= 260°C (Köffler)
LC/MS: MH⁺= 254 (tR = 5.43 minutes)
RMN ^{1H} δ en ppm (DMSO d 6) : 3.97 (s, 3H); 7.49 (dd, J₁ = 9 Hz, J₂ = 2.7 Hz, 1H); 7.59-7.64 (m, 2H); 7.76 (d, J = 2.7 Hz, 1H); 8.02-8.06 (m, 1H); 8.72-8.80 (m, 2H);12.90 (s, 1H, NH).

### 38.3. 1-Chloro-7-méthoxy-4-(pyridin-3-yl)-phthalazine

Ce composé est obtenu selon le mode opératoire décrit en 1.3. par réaction de la 7-méthoxy-4-(pyridin-3-yl)-2H-phthalazin-1-one avec le chlorure de phosphoryle.
PF= 180°C (Mettler FP62)
LC/MS: MH⁺= 272 (tR = 6.26 minutes)
RMN ^{1H} δ en ppm (DMSO d 6) : 4.08 (s, 3H); 7.51-7.76 (m, 3H); 7.96 (d, J = 9 Hz, 1H); 8.15-8.20 (m, 1H); 8.80-9.0 (m, 2H).

### 38.4. Trichlorhydrate de la 7-méthoxy-N-[1-(naphthalèn-2-ylméthyl)pipéridin-4-yl]-4-(pyridin-3-yl)-phthalazin-1-amine

Ce composé est obtenu selon le mode opératoire décrit en 1.4. par réaction de la 1-chloro-7-méthoxy-4-(pyridin-3-yl)-phthalazine avec la 4-amino-1-(naphthalèn-2-yl-méthyl)-pipéridine. Le trichlorhydrate est obtenu après traitement avec une solution d'acide chlorhydrique dans l'alcool isopropylique.

### Exemple 39: (composé n°111)

### Dichlorhydrate de la 4-(biphényl-4-yl)-7-méthoxy-N-[1-(naphthalèn-2-ylméthyl)pipéridin-4-yl]phthalazin-1-amine

### 39.1. Acide 2-(biphényl-4-carbonyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 4-phényl-N-méthoxy-N-méthyl-benzamide.
PF= 200°C (Mettler FP62)
LC/MS: MH⁺= 333 (tR = 9.16 minutes)
RMN ^{1H} δ en ppm (DMSO d 6) : 3.90 (s, 3H); 7.28 (dd, J₁ = 8.5 Hz, J₂ = 2.5 Hz, 1H); 7.41-7.54 (m, 5H); 7.69-7.82 (m, 6H); 13.2 (s, 1H, COOH).

### 39.2. 4-(Biphényl-4-yl)-7-méthoxy-2H-phthalazin-1-one

Ce composé est obtenu selon le mode opératoire décrit en 1.2. par réaction de l'acide 2-(biphényl-4-carbonyl)-5-méthoxy-benzoïque avec de l'hydrate d'hydrazine.
LC/MS: MH⁺= 329 (tR = 9.31 minutes)
RMN ^{1H} δ en ppm (DMSO d 6) : 3.98 (s, 3H); 7.42-7.56 (m, 4H), 7.66-7.88 (m, 8H); 12.81 (s, 1H, NH).

### 39.3. 4-(Biphényl-4-yl)-1-chloro-7-méthoxy-phthalazine

Ce composé est obtenu selon le mode opératoire décrit en 1.3. par réaction de la 4-(biphényl-4-yl)-7-méthoxy-2H-phthalazin-1-one avec le chlorure de phosphoryle.
PF= 177°C (Mettler FP62)
LC/MS: MH⁺= 347 (tR = 10.25 minutes)
RMN ^{1H} δ en ppm (DMSO d 6) : 4.08 (s, 3H); 7.44-7.58 (m, 3H), 7.63 (d, J = 2.5 Hz, 1H); 7.73 (dd, J₁ = 9 Hz, J₂ = 2.5 Hz, 1H); 7.79-7.95 (m, 6H); 8.05 (d, J = 9 Hz, 1H).

### 39.4. Dichlorhydrate de la 4-(biphényl-4-yl)-7-méthoxy-N-[1-(naphthalèn-2-ylméthyl)pipéridin-4-yl]phthalazin-1-amine

Ce composé est obtenu selon le mode opératoire décrit en 1.4, par réaction de la 4-(biphényl-4-yl)-1-chloro-7-méthoxy-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 40: (composé n°117)

### Dichlorhydrate de la 4-(3,4-diméthyl-phényl)-7-méthoxy-N-[1-(naphthalèn-2-ylméthyl)pipéridin-4-yl]phthalazin-1-amine

### 40.1. Acide 2-(3,4-diméthyl-benzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 3,4-diméthyl-N-méthoxy-N-méthyl-benzamide.
PF= 173°C (Mettler FP62)
LC/MS: MH⁺= 285 (tR = 8.37 minutes)
RMN ^{1H} δ en ppm (DMSO d 6) : 2.24 (s, 3H); 2.28 (s, 3H); 3.88 (s, 3H); 7.18-7.55 (m, 6H); 13.07 (s, 1H, COOH).

### 40.2. 4-(3,4-Diméthyl-phényl)-7-méthoxy-2H-phthalazin-1-one

Ce composé est obtenu selon le mode opératoire décrit en 1.2. par réaction de l'acide 2-(3,4-diméthyl-benzoyl)-5-méthoxy-benzoïque avec de l'hydrate d'hydrazine.
PF= 241°C (Mettler FP62)
LC/MS: MH⁺= 281 (tR = 8.54 minutes)
RMN ^{1H} δ en ppm (DMSO d 6) : 2.32 (s, 3H); 2.33 (s, 3H); 3.97 (s, 3H); 7.26-7.35 (m, 3H); 7.47 (dd, J₁ = 9 Hz, J₂ = 2.7 Hz, 1H); 7.66 (d, J = 9 Hz, 1H); 7.74 (d, J = 2.7 Hz, 1H); 12.73 (s, 1H, NH).

### 40.3. 1-Chloro-4-(3,4-diméthyl-phényl)-7-méthoxy-phthalazine

Ce composé est obtenu selon le mode opératoire décrit en 1.3. par réaction de la 4-(3,4-diméthyl-phényl)-7-méthoxy-2H-phthalazin-1-one avec le chlorure de phosphoryle.
PF= 204°C (Mettler FP62)
LC/MS: MH⁺= 299 (tR = 9.47 minutes)
RMN ^{1H} δ en ppm (DMSO d 6) : 2.35 (s, 3H); 2.36 (s, 3H); 4.06 (s, 3H); 7.35-7.48 (m, 3H); 7.59 (d, J = 2.5 Hz, 1H); 7.70 (dd, J₁ = 9 Hz, J₂ = 2.5 Hz, 1H); 7.98 (d, J = 9 Hz, 1H).

### 40.4. Dichlorhydrate de la 4-(3,4-diméthyl-phényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 1-chloro-4-(3,4-diméthyl-phényl)-7-méthoxy-phthalazine avec la 4-amino-1-(naphthalèn-2-yl-méthyl)-pipéridine. Le dichlorhydrate est obtenu après traitement avec une solution d'acide chlorhydrique dans l'isopropanol puis cristallisation dans l'acétate d'éthyle.

### Exemple 41: (composé n°136)

### Dichlorhydrate de la 7-méthoxy-4-phénoxyméthyl-N-[1-(naphthalèn-2-ylméthyl)pipéridin-4-yl]phthalazin-1-amine

### 41.1. Acide 5-méthoxy-2-(2-phénoxy-acétyl)-benzoïque

Ce composé est synthétisé selon la méthode décrite en 3.2. en faisant réagir de l'acide 2-bromo-5-méthoxybenzoïque préalablement traité avec du n-butyl-lithium avec la N-méthoxy-N-méthyl-2-phénoxyacétamide. Le produit est utilisé brut dans la réaction suivante.

### 41.2. 7-Méthoxy-4-phénoxyméthyl-2H-phthalazin-1-one

Ce composé est obtenu selon le mode opératoire décrit en 1.2. par réaction de l'acide 5-méthoxy-2-(2-phénoxy-acétyl)-benzoïque avec de l'hydrate d'hydrazine.
PF= 192°C (Mettler FP62)
LC/MS: MH⁺= 283 (tR = 7.91 minutes)
RMN ^{1H} δ en ppm (DMSO d 6) : 3.96 (s, 3H); 5.31 (s, 2H); 6.96-7.10 (m, 3H); 7.29-7.36 (m, 2H); 7.55 (dd, J₁ = 9 Hz, J₂ = 2.7 Hz, 1H); 7.68 (d, J = 2.7 Hz, 1H); 7.98 (d, J = 9 Hz, 1H); 12.69 (s, 1H, NH).

### 41.3. 1-Chloro-7-méthoxy-4-phénoxyméthyl-phthalazine

Ce composé est obtenu selon le mode opératoire décrit en 1.3. par réaction de la 7-méthoxy-4-phénoxyméthyl-2H-phthalazin-1-one avec le chlorure de phosphoryle.
LC/MS: MH⁺= 301 (tR = 8.90 minutes)
RMN ^{1H} δ en ppm (DMSO d 6) : 4.06 (s, 3H); 5.73 (s, 2H); 6.96-7.03 (m, 1H); 7.09-7.13 (m, 2H); 7.29-7.36 (m, 2H); 7.58 (d, J = 2.5 Hz, 1H); 7.81 (dd, J₁ = 9 Hz, J₂ = 2.5 Hz, 1H); 8.37 (d, J = 9 Hz, 1H).

### 41.4. Dichlorhydrate de la 7-méthoxy-4-phénoxyméthyl-N-[1-(naphthalèn-2-ylméthyl)pipéridin-4-yl]phthalazin-1-amine

Ce composé est obtenu selon le mode opératoire décrit en 1.4. par réaction de la 1-chloro-7-méthoxy-4-phénoxyméthyl-phthalazine avec la 4-amino-1-(naphthalèn-2-yl-méthyl)-pipéridine. Le dichlorhydrate est obtenu après traitement avec une solution d'acide chlorhydrique dans l'éther diéthylique puis cristallisation dans l'éther diisopropylique.

### Exemple 42 : (composé n° 137)

### Dichlorhydrate de la 4,7-diphényl-N-[1-(naphthalèn-2-ylméthyl)pipéridin-4-yl]phthalazin-1-amine

### 42.1. 7-Hydroxy-N-[1-(naphthalèn-2-ylméthyl)pipéridin-4-yl]-4-phényl-phthalazin-1-amine

Un mélange de 597 mg (1.25 mmol) de 7-méthoxy-N-[1-(naphthalèn-2-ylméthyl)pipéridin-4-yl]-4-phényl-phthalazin-1-amine (composé n°1) et de chlorhydrate de pyridinium est chauffé à 210°C pendant 45 minutes. Le milieu réactionnel est refroidi puis additionné de soude 1N. Il se forme un précipité qui est lavé à l'eau, au dichlorométhane puis séché sous pression réduite. On obtient 500 mg de poudre beige.
LC/MS: MH⁺= 461 (tR = 5.30 minutes)
RMN ^{1H} δ en ppm (DMSO d 6) : 1.64-1.73 (m, 2H); 1.90-2.03 (m, 2H); 2.10-2.22 (m, 2H); 2.85-2.95 (m, 2H); 3.66 (s, 2H); 4.10-4.18 (m, 1H); 5.90-6.0 (m, 1H, OH); 6.60-6.64 (m, 2H); 7.20-7.24 (d, J = 9.7 Hz, 1H, NH); 7.39-7.57 (m, 9H); 7.82-7.94 (m, 4H).

### 42.2. 1-[1-(naphthalèn-2-ylméthyl)pipéridin-4-yl-amino]-4-phényl-7-trifluoro méthanesulfonyloxy-phthalazin-6-yl

Une solution de 480 mg (1.04 mmol) de 7-hydroxy-N-[1-(naphthalèn-2-ylméthyl)pipéridin-4-yl]-4-phényl-phthalazin-1-amine dans 6 mL de dichlorométhane est additionnée de 558 mg (1.5 mmol) de N-phényltrifluorométhanesulfonimide ((CF₃SO₂)₂NC₆H₅) et de 0.17 mL de triéthylamine. Le mélange est agité à température ambiante sous argon pendant 2 jours. Il est hydrolysé par une solution aqueuse de chlorure l'ammonium puis extrait au dichlorométhane. La phase organique est séchée sur sulfate de sodium anhydre, filtrée et évaporée sous pression réduite. Une huile marron est obtenue et utilisée telle quelle dans la réaction suivante.

### 42.3. Dichlorhydrate de la 4,7-diphényl-N-[1-(naphthalèn-2-ylméthyl)pipéridin-4-yl]phthalazin-1-amine

Une solution de 0.5 mmol de 1-[1-(naphthalèn-2-ylméthyl)pipéridin-4-yl-amino]-4-phényl-7-trifluorométhanesulfonyloxy-phthalazin-6-yl dans 4 ml de toluène est additionnée de 0.5 mL d'une solution aqueuse 2N de carbonate de sodium, 70 mg (0.57 mmol) d'acide phénylboronique et 18 mg de palladium tétrakis. Le mélange est agité à 100°C pendant 6 heures dans un tube bouché sous argon. Le milieu réactionnel refroidi est additionné de soude 1N est extrait à l'acétate d'éthyle. La phase organique est séchée sur sulfate de sodium anhydre, filtrée et évaporée sous pression réduite. Après purification par chromatographie sur colonne (dichlorométhane/méthanol 100/0 à 90/10 (v/v)) 150 mg de produit sont obtenus.
LC/MS: MH⁺= 521 (tR = 6.48 minutes)
RMN ^{1H} δ en ppm (DMSO d 6) : 1.70-1.90 (m, 2H); 2.08-2.30 (m, 4H); 2.90-3.05 (m, 2H); 3.72 (s, 2H); 4.30-4.40 (m, 1H); 7.40 (d, 1H, NH); 7.48-7.70 (m, 11H); 7.82-7.97 (m, 7H); 8.18 (d, J = 9 Hz,1H); 8.25 (s, 1H).

Le dichlorhydrate est obtenu après traitement avec une solution d'acide chlorhydrique dans l'éther diéthylique.

### Exemple 43: (composé n°142)

### 4-(4-méthoxy-phényl)-6-méthyl-N-[1 -(naphthalèn-2-ylméthyl)pipéridin-4-yl]phthalazin-1-amine

### 43.1. Acide 2-(4-méthoxy-benzoyl)-4-méthyl-benzoïque

Ce composé est synthétisé selon la méthode décrite en 3.2. en faisant réagir de l'acide 2-bromo-4-méthyl-benzoïque préalablement traité avec du n-butyl-lithium avec la 4-méthoxy-N-méthoxy-N-méthyl-4-benzamide. Il est utilisé brut dans la réaction suivante.

### 43.2. 4-(4-Méthoxy-phényl)-6-méthyl-2H-phthalazin-1-one

Ce composé est obtenu selon le mode opératoire décrit en 1.2. par réaction de l'acide 2-(4-méthoxy-benzoyl)-4-méthyl-benzoïque non purifié avec de l'hydrate d'hydrazine.
PF= 245°C (Mettler FP62)
LC/MS: MH⁺= 267 (tR = 7.63 minutes)
RMN ^{1H} δ en ppm (DMSO d 6) : 2.33 (s, 3H); 3.86 (s, 3H); 7.10-7.13 (m, 2H); 7.48-7.55 (m, 3H); 7.70 (d, J = 8 Hz, 1H); 8.24 (d, J = 8 Hz, 1H); (NH non détecté).

### 43.3. 1-Chloro-4-(4-méthoxy-phényl)-6-méthyl-phthalazine

Ce composé est obtenu selon le mode opératoire décrit en 1.3. par réaction de la 4-(4-méthoxy-phényl)-6-méthyl-2H-phthalazin-1-one avec le chlorure de phosphoryle.
LC/MS: MH⁺= 285 (tR = 8.70 minutes)
RMN ^{1H} δ en ppm (DMSO d 6) : 2.58 (s, 3H); 3.90 (s, 3H); 7.16-7.23 (m, 2H); 7.66-7.73 (m, 2H); 7.85 (d, J = 1.5 Hz, 1H); 8.03 (dd, J₁ = 8.5 Hz, J₂ = 1.5 Hz, 1H); 8.28 (d, J = 8.5 Hz, 1H).

### 43.4. 4-(4-méthoxy-phényl)-6-méthyl-N-[1-(naphthalèn-2-ylméthyl)pipéridin-4-yl]phthalazin-1-amine

Ce composé est obtenu selon le mode opératoire décrit en 1.4. par réaction de la 1-chloro-4-(4-méthoxy-phényl)-6-méthyl-phthalazine avec la 4-amino-1-(naphthalèn-2-yl-méthyl)-pipéridine.

### Exemple 44: (composé n°143)

### Dichlorhydrate de la 7-chloro-4-(4-méthoxy-phényl)-N-[1-(naphthalèn-2-ylméthyl)pipéridin-4-yl]phthalazin-1-amine

### 44.1. Acide 5-chloro-2-(4-méthoxy-benzoyl)-benzoïque

Ce composé est synthétisé selon la méthode décrite en 3.2. en faisant réagir de l'acide 2-bromo-5-chloro-benzoïque préalablement traité avec du n-butyl-lithium avec la 4-méthoxy-N-méthoxy-N-méthyl-benzamide. Il est utilisé brut dans la réaction suivante.

### 44.2. 7-Chloro-4-(4-Méthoxy-phényl)-2H-phthalazin-1-one

Ce composé est obtenu selon le mode opératoire décrit en 1.2. par réaction de l'acide 5-chloro-2-(4-méthoxy-benzoyl)-benzoïque non purifié avec de l'hydrate d'hydrazine.
PF= 220°C (Mettler FP62)
LC/MS: MH⁺= 286 (tR = 8.12 minutes)
RMN ^{1H} δ en ppm (DMSO d 6) : 3.86 (s, 3H); 7.09-7.15 (m, 2H); 7.50-7.57 (m, 2H); 7.74 (d, J = 8.7 Hz, 1H); 7.96 (dd, J₁ = 8.7 Hz, J₂ = 2.2 Hz, 1H); 8.28 (d, J = 2.2 Hz, 1H); 13.0 (s, 1H,NH).

### 44.3. 1,7-Dichloro-4-(4-méthoxy-phényl)-phthalazine

Ce composé est obtenu selon le mode opératoire décrit en 1.3. par réaction de la 7-chloro-4-(4-Méthoxy-phényl)-2H-phthalazin-1-one avec le chlorure de phosphoryle.
LC/MS: MH⁺= 305 (tR = 9.34 minutes)
RMN ^{1H} δ en ppm (DMSO d 6) : 3.90 (s, 3H); 7.17-7.23 (m, 2H); 7.68-7.74 (m, 2H); 8.10 (d, J = 9 Hz, 1H); 8.17 (dd, J₁ = 9 Hz, J₂ = 2 Hz, 1H); 8.39 (d, J = 2 Hz, 1H).

### 44.4. Dichlorhydrate de la 7-chloro-4-(4-méthoxy-phényl)-N-[1-(naphthalèn-2-ylméthyl)pipéridin-4-yl]phthalazin-1-amine

Ce composé est obtenu selon le mode opératoire décrit en 1.4. par réaction de la 1,7-dichloro-4-(4-méthoxy-phényl)-phthalazine avec 4-amino-1-(naphthalèn-2-yl-méthyl)pipéridine. Le dichlorhydrate est obtenu après traitement avec une solution d'acide chlorhydrique dans l'éther diéthylique, puis cristallisation dans l'éther diisopropylique.

### Exemple 45: (composé n°145)

### Dichlorhydrate de la 4-(4-bromophényl)-7-méthoxy-N-[1-(naphthalèn-2-ylméthyl)pipéridin-4-yl]phthalazin-1-amine

### 45.1. Acide 2-(4-bromobenzoyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 4-bromo-N-méthoxy-N-méthyl-benzamide. Il est utilisé brut dans la réaction suivante.

### 45.2. 4-(4-Bromophényl)-7-méthoxy-2H-phthalazin-1-one

Ce composé est obtenu selon le mode opératoire décrit en 1.2. par réaction de l'acide 2-(4-bromobenzoyl)-5-méthoxy-benzoïque non purifié avec de l'hydrate d'hydrazine.
PF= 275°C (Mettler FP62)
LC/MS: MH⁺= 333 (tR = 8.47 minutes)
RMN ^{1H} δ en ppm (DMSO d 6) : 3.97 (s, 3H); 7.48 (dd, J₁ = 9 Hz, J₂ = 2.7 Hz, 1H); 7.52-7.58 (m, 2H); 7.64 (d, J = 9 Hz, 1H); 7.74-7.79 (m, 3H);12.83 (s, 1H, NH).

### 45.3. 4-(4-Bromophényl)-1-chloro-7-méthoxy-phthalazine

Ce composé est obtenu selon le mode opératoire décrit en 1.3. par réaction de la 4-(4-bromophényl)-7-méthoxy-2H-phthalazin-1-one avec le chlorure de phosphoryle.
PF = 205°C (Mettler FP62)
LC/MS: MH⁺= 351 (tR = 9.38 minutes)
RMN ^{1H} δ en ppm (DMSO d 6) : 4.07 (s, 3H); 7.63 (d, J = 2.5 Hz, 1H); 7.65-7.69 (m, 2H); 7.72 (dd, J₁ = 9 Hz, J₂ = 2.5 Hz, 1H); 7.81-7.87 (m, 2H); 7.96 (d, J = 9 Hz, 1H).

### 45.4. Dichlorhydrate de la 4-(4-bromophényl)-7-méthoxy-N-[1-(naphthalèn-2-ylméthyl)pipéridin-4-yl]phthalazin-1-amine

Ce composé est obtenu selon le mode opératoire décrit en 1.4. par réaction de la 4-(4-bromophényl)-1-chloro-7-méthoxy-phthalazine avec la 4-amino-1-(naphthalèn-2-yl-méthyl)-pipéridine. Le dichlorhydrate est obtenu après traitement avec une solution d'acide chlorhydrique dans l'éther diéthylique.

### Exemple 46: (composé n°17)

### Dichlorhydrate de la 7-méthoxy-N-[1-(naphthalèn-2-ylméthyl)pipéridin-4-yl]-5-phényl-phthalazin-1-amine

### 46.1. 2-Formyl-3-hydroxy-5-méthoxy-benzoate de méthyle

Une solution de 5.1 g (.3 mmol) de 2-Formyl-3,5-méthoxy-benzoate de méthyle dans 50 mL de dichlorométhane est agitée à -10°C puis additionnée goutte à goutte de 23 mL d'une solution de tribromure de bore dans le dichlorométhane. Le mélange est agitée à-10°C pendant 1 heure puis est hydrolysé par 100 mL d'eau. La phase organique est lavée à l'eau, séchée sur sulfate de sodium anhydre, filtrée et évaporée sous pression réduite. Le résidu est purifié sur colonne de silice (éluant cyclohexane/acétate d'éthyle 7/3 v/v). 4 g de produit beige sont obtenus.
RMN ^{1H} δ en ppm (DMSO d 6) : 3.83 (s, 3H); 3.86 (s, 3H); 6.66 (d, J = 2.5 Hz, 1H); 6.73 (d, J = 2.5 Hz, 1H); 10.20 (s, 1H, CHO); 11.8 (s, 1H, OH).

### 46.2. 2-Formyl-5-méthoxy-3-trifluorométhanesulfonyloxybenzoate de méthyle

Une solution de 1 g (4.7 mmol) de 2-formyl-3-hydroxy-5-méthoxy-benzoate de méthyle dans 20 mL de tétrahydrofuranne est agitée à température ambiante sous azote puis additionnée de 200 mg d'une suspension huileuse d'hydrure de sodium à 60 %. Le mélange est agité pendant 30 minutes puis 1.7 g (4.8 mmol) de N-phényltrifluorométhanesulfonimide ((CF₃SO₂)₂NC₆H₅) sont ajouté par fractions. Le milieu réactionnel est agité pendant 24 heures puis hydrolysé avec une solution aqueuse de chlorure d'ammonium et extrait à l'acétate d'éthyle. La phase organique est lavée à l'eau, et à l'eau salée, séchée sur sulfate de sodium anhydre, filtrée et évaporée sous pression réduite. Le produit sous forme d'une huile marron est utilisé sans purification dans la réaction suivante.
LC/MS: MH⁺= 343 (tR = 9.07 minutes)

### 46.3. Acide 2-formyl-5-méthoxy-3-phényl-benzoique

Une solution de environ 2.3 mmol de 2-formyl-5-méthoxy-3-trifluorométhanesulfonyloxy-benzoate de méthyle dans 10 mL de toluène est agitée sous argon et additionnée de 330 mg (2.7 mmol) d'acide phénylboronique, 84 mg de palladium tétrakis et 2.5 mL d'une solution aqueuse 2N de carbonate de sodium. Le mélange est agité à 110°C pendant 6 heures dans un tube bouché sous argon. Le milieu réactionnel refroidi est additionné d'eau, lavé à l'acétate d'éthyle puis après acidification (HCl 1N) extraite par au dichlorométhane. La phase organique est séchée sur sulfate de sodium anhydre, filtrée et évaporée sous pression réduite. Le produit sous forme d'un solide brun est utilisé sans purification dans la réaction suivante.
LC/MS: MH⁺= 257 (tR = 7.76 minutes)

### 46.4. 7-Méthoxy-5-phényl-2H-phthalazin-1-one

Ce composé est obtenu selon le mode opératoire décrit en 1.2. par réaction de l'acide 2-formyl-5-méthoxy-3-phényl-benzoique avec de l'hydrate d'hydrazine.
PF= 139°C (Mettler FP62)
LC/MS: MH⁺= 253 (tR = 7.62 minutes)
RMN ^{1H} δ en ppm (DMSO d 6) : 3.99 (s, 3H); 7.41 (d, J = 2.5 Hz, 1H); 7.48-7.61 (m, 5H); 7.70 (d, J = 2.5 Hz, 1H); 7.96 (s, 1H); 12.69 (s, 1H, NH).

### 46.5. 1-Chloro-7-méthoxy-5-phényl-phthalazine

Ce composé est obtenu selon le mode opératoire décrit en 1.3. par réaction de la 7-méthoxy-5-phényl-2H-phthalazin-1-one avec le chlorure de phosphoryle.
PF= 146°C (Mettler FP62)
LC/MS: MH⁺= 271 (tR = 8.58 minutes)
RMN ^{1H} δ en ppm (DMSO d 6) : 4.08 (s, 3H); 7.54-7.62 (m, 6H); 7.67 (d, J = 2.7 Hz, 1H); 9.20 (s, 1H).

### 46.6. Dichlorhydrate de la 7-méthoxy-N-[1-(naphthalèn-2-ylméthyl)pipéridin-4-yl]-5-phényl-phthalazin-1-amine

Ce composé est obtenu selon le mode opératoire décrit en 2.4. par réaction de la 1-chloro-7-méthoxy-5-phényl-phthalazine avec la 4-amino-1-(naphthalèn-2-yl-méthyl)pipéridine.
LC/MS: MH⁺= 475 (tR = 5.69 minutes)
RMN ^{1H} δ en ppm (DMSO d 6) : 1.65-1.80 (m, 2H); 2.03-2.25 (m, 4H); 2.90-3.03 (m, 2H); 3.70 (s, 2H); 4.0 (s, 3H); 4.20-4.30 (m, 1H); 7.03 (d, 1H, NH); 7.36 (d, J = 2.5 Hz, 1H); 7.48-7.58 (m, 8H); 7.76 (d, J = 2.5 Hz, 1H); 7.84-7.92 (m, 4H); 8.52 (s, 1H).

Le dichlorhydrate est obtenu après traitement avec une solution d'acide chlorhydrique dans l'éther diéthylique.

### Exemple 47: (composé n°147)

### Dichlorhydrate de la 4-benzyloxyméthyl-7-méthoxy-N-[1-(naphthalèn-2-ylméthyl)pipéridin-4-yl]phthalazin-1-amine

### 47.1. Acide 2-(2-benzyloxy-acétyl)-5-méthoxy-benzoïque

Ce composé est synthétisé selon la méthode décrite en 3.2. en faisant réagir de l'acide 2-bromo-5-méthoxybenzoïque préalablement traité avec du n-butyl-lithium avec la 2-benzyloxy-N-méthoxy-N-méthyl-acétamide. Le produit est utilisé brut dans la réaction suivante.

### 47.2. 4-Benzyloxyméthyl-7-Méthoxy-2H-phthalazin-1-one

Ce composé est obtenu selon le mode opératoire décrit en 1.2. par réaction de l'acide 2-(2-benzyloxy-acétyl)-5-méthoxy-benzoïque avec de l'hydrate d'hydrazine.
PF= 140°C (Mettler FP62)
LC/MS: MH⁺= 297 (tR = 7.80 minutes)
RMN ^{1H} δ en ppm (DMSO d 6) : 3.95 (s, 3H); 4.58 (s, 2H); 4.73 (s, 2H); 7.25-7.37 (m, 5H); 7.53 (dd, J₁ = 9 Hz, J₂ = 2.7 Hz, 1H); 7.66 (d, J = 2.7 Hz, 1H); 8.04 (d, J = 9 Hz, 1H); 12.59 (s, 1H, NH).

### 47.3. 4-Benzyloxyméthyl-1-chloro-7-méthoxy-phthalazine

Ce composé est obtenu selon le mode opératoire décrit en 1.3. par réaction de la 4-benzyloxyméthyl-7-Méthoxy-2H-phthalazin-1-one avec le chlorure de phosphoryle.

Ce composé huileux est utilisé tel quel dans la réaction suivante.

### 47.4. Dichlorhydrate de la 4-benzyloxyméthyl-7-méthoxy-N-[1-(naphthalèn-2-ylméthyl)pipéridin-4-yl]phthalazin-1-amine

Ce composé est obtenu selon le mode opératoire décrit en 1.4. par réaction de la 4-benzyloxyméthyl-1-chloro-7-méthoxy-phthalazine avec la 4-amino-1-(naphthalèn-2-yl-méthyl)-pipéridine.
RMN ^{1H} δ en ppm (DMSO d 6) : 1.65-1.75 (m, 2H); 2.0-2.25 (m, 4H); 2.90-3.0 (m, 2H); 3.70 (s, 2H); 3.97 (s, 3H); 4.20-4.30 (m, 1H); 4.55 (s, 2H); 4.90 (s, 2H); 7.02 (d, 1H, NH); 7.30-7.36 (m, 5H); 7.45-7.56 (m, 4H); 7.73 (d, J = 2.2 Hz, 1H); 7.84-7.92 (m, 4H); 8.06 (d, J = 9 Hz, 1H).

Le dichlorhydrate est obtenu après traitement avec une solution d'acide chlorhydrique dans l'éther diéthylique.

### Exemple 48: (composé n°148)

### 4-Hydroxyméthyl-7-méthoxy-N-[1-(naphthalèn-2-ylméthyl)pipéridin-4-yl]phthalazin-1-amine

Une solution de 120 mg de 4-benzyloxyméthyl-7-méthoxy-N-[1-(naphthalèn-2-ylméthyl)pipéridin-4-yl]phthalazin-1-amine dans 4 mL de dichlorométhane est agitée sous azote à 0°C puis additionnée goutte à goutte de 2.3 mL d'une solution molaire de trichlorure de bore dans le dichlorométhane. Le milieu réactionnel est agité pendant 1 heure à 0°C puis 30 minutes à température ambiante. Il est hydrolysé par une solution aqueuse de bicarbonate de sodium et extrait au dichlorométhane. La phase organique est lavée à l'eau, séchée sur sulfate de sodium anhydre, filtrée et évaporée sous pression réduite. Le résidu est purifié sur colonne de silice (éluant dichlorométhane/méthanol 100/0 à 85/15 v/v). 58 mg de produit sont obtenus sous forme d'une poudre beige.
PF= 102°C (Köffler)
LC/MS: MH⁺= 429 (tR = 4.78 minutes)
RMN ^{1H} δ en ppm (DMSO d 6) : 1.60-1.80 (m, 2H); 2.0-2.30 (m, 4H); 2.90-3.0 (m, 2H); 3.72 (s, 2H); 3.97 (s, 3H); 4.18-4.30 (m, 1H); 4.84 (d, J = 5.5 Hz, 2H); 5.29 (t, J = 5.5 Hz, 1H); 6.95 (d, J = 7.5 Hz, 1H, NH); 7.45-7.60 (m, 4H); 7.71 (d, J = 2.5 Hz, 1H); 7.84-7.93 (m, 4H); 8.05 (d, J = 9.2 Hz, 1H).

Le dichlorhydrate est obtenu après traitement avec une solution d'acide chlorhydrique dans l'éther diéthylique.

### Exemple 49: (composé n°18)

### N-[1-(3-cyanophényl-méthyl)-pipéridin-4-yl]-7-méthoxy-4-(4-méthoxyphényl)phthalazin-1-amine

### 49.1. 7-Méthoxy-4-(4-méthoxyphényl)-N-(pipéridin-4-yl)-phthalazin-1-amine

La manipulation s'effectue sous atmosphère inerte (azote).

Dans un tricol de 500 mL sont introduits 1 g de palladium sur charbon à 10 % et 100 mL d'eau. A ce mélange agité à 85°C, est additionné goutte à goutte, pendant 1 heure une solution préalablement préparée de 13.3 g de N-(1-benzylpipéridin-4-yl)-7-méthoxy-4-(4-méthoxyphényl)phthalazin-1-amine (composé 7) dans 50 mL d'éthanol et 4.4 mL d'acide formique. Le mélange réactionnel est agité à reflux pendant 2 heures. Après retour à température ambiante, l'éthanol est évaporé sous pression réduite. Le mélange est ensuite alcalinisé à pH 12 par de la soude 2N, puis est extrait au dichlorométhane. La phase organique est lavée par de l'eau saturée en chlorure de sodium, séchée sur sulfate de sodium anhydre, puis évaporée sous pression réduite. L'huile jaune obtenue est concrétée dans l'éther diisopropylique pour obtenir un solide blanc. Après filtration et séchage sous vide en présence d'anhydride phosphorique, on obtient 10.29 g de 7-méthoxy-4-(4-méthoxyphényl)-N-(pipéridin-4-yl)-phthalazin-1-amine (rendement = 97 %).
PF= 143°C (Mettler FP62)
LC/MS: MH⁺= 365 (tR = 4.88 minutes)
RMN ^{1H} δ en ppm (DMSO d 6) : 1.5-1.65 (m, 2H); 2.0-2.1 (m, 2H); 2.6-2.7 (m, 2H); 3.0-3.1 (m, 2H); 3.3 (s, 1H, NH); 3.85 (s, 3H); 3.98 (s, 3H); 4.2-4.3 (m, 1H); 7.0 (d, J = 7.5 Hz, 1H); 7.10 (d, J = 8.5 Hz, 2H); 7.42 (dd, J₁ = 9.2 Hz, J₂ = 2.5 Hz, 1H); 7.52 (d, J = 8.5 Hz, 2H); 7.75 (d, J = 9.2 Hz, 1H); 7.78 (d, J = 2.5 Hz, 1H).

### 49.2. N-[1-(3-cyanobenzyl)-pipéridin-4-yl)-7-méthoxy-4-(4-méthoxyphényl)phthalazin-1-amine

Une solution de 500 mg (1.4 mmol) de 7-méthoxy-4-(4-méthoxyphényl)-N-(pipéridin-4-yl)phthalazin-1-amine dans 5 mL de dichloro-1,2-éthane est additionnée de 183 mg (1.4 mmol) de 3-cyanobenzaldéhyde. Le mélange est agité pendant 30 minutes sous azote, puis 378 mg (1.8 mmol) de triacétoxyborohydrure de sodium sont additionnés. Le milieu réactionnel est agité pendant 16 heures à température ambiante puis hydrolysé avec de l'eau et de la soude 1N. L'extrait au dichlorométhane est lavé par de l'eau saturée en chlorure de sodium, séché sur sulfate de sodium anhydre, puis évaporé sous pression réduite. Le résidu est purifié sur colonne de silice (éluant dichlorométhane/méthanol 95/5 (v/v)). Après cristallisation dans l'éther diisopropylique, 300 mg de poudre blanche sont obtenus.
PF= 115°C (Mettler FP62)
LC/MS: MH⁺= 480 (tR = 4.24 minutes)
RMN ^{1H} δ en ppm (DMSO d 6) : 1.64-1.80 (m, 2H); 2.03-2.28 (m, 4H); 2.85-2.95 (m, 2H); 3.62 (s, 2H); 3.86 (s, 3H); 3.99 (s, 3H); 4.20-4.33 (m, 1H); 7.0 (d, 1H, NH); 7.10 (d, J = 8.5 Hz, 2H); 7.44 (dd, J₁ = 9.2 Hz, J₂ = 2.5 Hz, 1H); 7.54 (d, J = 8.5 Hz, 2H); 7.60 (d, J = 9.2 Hz, 1H); 7.70-8.80 (m, 5H).

Cette méthode d'amination réductrice a été utilisée avec de nombreux aldéhydes commerciaux ou déjà décrits dans la littérature. Les composés synthétisés sont décrits dans le tableau.

### Exemple 50: (composés n°56, n°149, n°150)

### 7-méthoxy-4-(4-méthoxyphényl)-N-1 [1-(naphtalène-2-yl-éthyl)-pipéridin-4yl]phthalazin-1-amine (racémique et énantiomères)

Une solution de 500 mg (1:4 mmol) de 7-méthoxy-4-(4-méthoxyphényl)-N-(pipéridin-4-yl)phthalazin-1-amine dans 5 mL d'éthanol est additionnée de 232 mg (1.4 mmol) de 1-naphtalèn-2-yl-éthanone et de 0.5 mL de tétraisopropyloxyde de titane. Le mélange est agité pendant 18 heures sous azote à température ambiante, puis 58 mg (0.9 mmol) de cyanoborohydrure de sodium en solution dans 2 mL d'éthanol sont additionnés. Le milieu réactionnel est agité pendant 18 heures à température ambiante puis filtré. Le filtrat est évaporé à sec puis purifié par chromatographie sur colonne de silice (éluant dichlorométhane/méthanol 100/0 à 90/10 v/v). Après cristallisation dans l'éther diisopropylique, 480 mg de poudre blanche sont obtenus.

Le dichlorhydrate est obtenu après traitement avec une solution d'acide chlorhydrique dans l'éther diéthylique puis cristallisation dans l'éther diisopropylique.

Les énantiomères sont séparés par chromatographie liquide sur phase chirale Chiralpak AD (éluant isohexane/éthanol 60/40 (v/v). Leur configuration absolue n'ont pas été déterminées. Ces produits contiennent un peu d'isohexane résiduel.

### (-)-7-Méthoxy-4-(4-méthoxyphényl)-N-1 [1-(naphtalène-2-yl-éthyl)-pipéridin-4yl]-phthalazin-1-amine

[α]_{D}²⁰ = -24 (c=0.15, dichlorométhane)
PF= 136°C (Mettler FP62)
LC/MS: MH⁺= 519 (tR = 5.76 minutes)
RMN ^{1H} δ en ppm (CDCl₃): 1.45 (d, J = 6.7 Hz, 3H); 1.64-1.79 (m, 2H); 2.10-2.35 (m, 4H); 2.80-2.90 (m, 1H); 3.08-3.15 (m, 1H); 3.65-3.75 (m, 1H, CH); 3.84 (s, 3H); 3.94 (s, 3H); 4.30-4.40 (m, 1H); 5.13 (d, J = 7.2, 1H, NH); 6.98 (d, J = 8.7 Hz, 2H); 7.11 (d, J = 2.2 Hz, 1H, H-1 naphtyl); 7.27 (dd, J₁ = 9.2 Hz, J₂ = 2.5 Hz, 1H); 7.40-7.45 (m, 2H); 7.52-7.60 (m, 3H); 7.71-7.80 (m, 4H); 7.87 (d, J = 9.2 Hz, 1H).

### (+)-7-Méthoxy-4-(4-méthoxyphényl)-N-1[1-(naphtalène-2-yl-éthyl)-pipéridin-4yl]-phthalazin-1-amine

[α]_{D}²⁰ = +17 (c=0.18, dichlorométhane)
PF= 132°C (Mettler FP62)
LC/MS: MH⁺= 519 (tR = 5.76 minutes)
RMN ^{1H} δ en ppm (CDCl₃) : 1.53 (d, J = 6.7 Hz, 3H); 1.63-1.80 (m, 2H); 2.15-2.42 (m, 4H); 2.90-3.0 (m, 1H); 3.17-3.27 (m, 1H); 3.65-3.75 (m, 1H, CH); 3.90 (s, 3H); 4.02 (s, 3H); 4.34-4.44 (m, 1H); 4.76 (d, J = 7.2, 1H, NH); 7.01 (d, J = 2.2 Hz, 1H, H-1 naphtyl); 7.05 (d, J = 8.7 Hz, 2H); 7.34 (dd, J₁ = 9.2 Hz, J₂ = 2.5 Hz, 1H); 7.46-7.52 (m, 2H); 7.58-7.66 (m, 3H); 7.74-7.88 (m, 4H); 7.96 (d, J = 9.2 Hz, 1H).

### Exemple 51: (composés n°112)

### Dichlorhydrate de la N-[1-(4-hydroxyméthyl-benzyl)-pipéridin-4-yl]-7-méthoxy-4-(4-méthoxyphényl)phthalazin-1-amine

Ce produit est obtenu par réduction de l'ester méthylique correspondant (composé 107) qui a été préparé par la méthode d'amination réductrice décrite en 49.2 en utilisant le 4-formyl-benzoate de méthyle.

Une solution de 400 mg (0.78 mmol) de {4-[(4-{[7-méthoxy-4-(4-méthoxyphényl)phthalazin-1-yl]amino}pipéridin-1-yl)méthyl]phényl}carboxate de méthyle (composé 107) dans 6 mL de tétrahydrofurane est additionnée lentement sur une suspension de 59 mg (1.6 mmol) d'hydrure d'aluminium lithium dans 6 mL de tétrahydrofurane agitée à 0°C. Le milieu réactionnel est agité à 0°C pendant 1 heure puis hydrolysé par 0.1 mL de soude 1N, 2mL d'eau puis 0.1 mL de soude 1N. Il est extrait au dichlorométhane. La phase organique est lavée par de l'eau saturée en chlorure de sodium, séchée sur sulfate de sodium anhydre, puis évaporée sous pression réduite. Le résidu est purifié sur colonne de silice (éluant dichlorométhane/méthanol 85/15 (v/v)). Après cristallisation dans l'éther diisopropylique, 220 mg de poudre blanche sont obtenus.
PF= 139°C (Mettler FP62)
LC/MS: MH⁺= 485 (tR = 4.99 minutes)
RMN ^{1H} δ en ppm (DMSO d 6) : 1.60-1.80 (m, 2H); 2.03-2.20 (m, 4H); 2.85-2.96 (m, 2H); 3.51 (s, 2H); 3.85 (s, 3H); 3.97 (s, 3H); 4.20-4.30 (m, 1H); 4.49 (d, J = 5.7 Hz, 2H); 5.13 (t, J = 5.7 Hz, 1H); 6.98 (d, J = 7.5 Hz, 1H, NH); 7.10 (d, J = 8.7 Hz, 2H); 7.30 (s, 4H); 7.42 (dd, J₁ = 9.2 Hz, J₂ = 2.5 Hz, 1H); 7.50 (d, J = 8.7 Hz, 2H); 7.72-7.77 (m, 2H).

Le dichlorhydrate est obtenu après traitement avec une solution d'acide chlorhydrique dans l'éther diéthylique.

### Exemple 52: (composés n°113)

### Trichlorhydrate de la N-[1-(4-aminométhyl-benzyl)-pipéridin-4-yl]-7-méthoxy-4-(4-méthoxyphényl)phthalazin-1-amine

Ce produit est obtenu par réduction de la N-[1-(4-cyanobenzyl)-pipéridin-4-yl)-7-méthoxy-4-(4-méthoxyphényl)phthalazin-1-amine (composé 108) qui a été préparé par la méthode d'amination réductrice décrite en 49.2 en utilisant le 4-cyano-benzaldéhyde. 8 mL de tétrahydrofuranne sont agité à 10°C et additionné de 394 mg (10 mmol) de borohydrure de sodium. Ensuite, 0.8 mL (10 mmol) d'acide trifluoroacétique sont ajoutés goutte à goutte. Le milieu réactionnel est agité pendant 10 minutes puis une solution de 1 g (2 mmol) de N-[1-(4-cyanobenzyl)-pipéridin-4-yl)-7-méthoxy-4-(4-méthoxyphényl)phthalazin-1-amine dans 8 mL de tétrahydrofurane est introduite. Le milieu réactionnel est agité pendant une nuit à température ambiante puis est concenté sous pression réduite. 10 mL d'acide chlorhydrique à 10% sont ajouté et la solution est chauffée au reflux pendant 1 heure 30. Après refroidissement, le milieu réactionnel est lavé à l'éther diéthylique puis alcalinisé par de la soude 1N et extrait à l'acétate d'éthyle. La phase organique est lavée par de l'eau saturée en chlorure de sodium, séchée sur sulfate de sodium anhydre, puis évaporée sous pression réduite. Après cristallisation dans l'éther diisopropylique, 850 mg de poudre blanche sont obtenus.
PF= 203°C (Mettler FP62)
LC/MS: MH⁺= 484 (tR = 4.42 minutes)
RMN ^{1H} δ en ppm (DMSO d 6) : 1.60-1.80 (m, 2H); 2.0-2.20 (m, 4H); 2.85-2.96 (m, 2H); 3.20-3.40 (s, NH₂); 3.50 (s, 2H); 3.70 (s, 2H); 3.85 (s, 3H); 3.98 (s, 3H); 4.20-4.30 (m, 1H); 6.98 (d, J = 7.5 Hz, 1H, NH); 7.10 (d, J = 8.7 Hz, 2H); 7.20-7.35 (m, 4H); 7.42 (dd, J₁ = 9.2 Hz, J₂ = 2.5 Hz, 1H); 7.52 (d, J = 8.7 Hz, 2H); 7.74-7.77 (m, 2H).

Le dichlorhydrate est obtenu après traitement avec une solution d'acide chlorhydrique dans l'éther diéthylique.

### Exemple 53: (composés n°123)

### 53.1 Acide {4-[(4-{[7-méthoxy-4-(4-méthoxyphényl)phthalazin-1-yl]amino}pipéridin-1-yl)méthyl]phényl}carboxylique (dichlorhydrate)

Une solution de 1.6 g (3.1 mmol) de N-{4-[(4-{[7-méthoxy-4-(4-méthoxyphényl)phthalazin-1-yl]amino}pipéridin-1-yl)méthyl]phényl}carboxylate de méthyle dans 20 mL de méthanol est additionnée de 3.1 mL de soude 2N et 5 mL de dichlorométhane. Le mélange est agité à température ambiante pendant 20 heures puis acidifié par de l'acide chlorhydrique 1N. Il est évaporé sous pression réduite et le résidu est repris par du dichlorométhane et de l'éthanol. Il se forme un précipité blanc qui est lavé à l'éther diisopropylique puis séché. 1.27 g de poudre blanche sont obtenues (rendement 72 %).
PF= 295°C (Mettler FP62)
LC/MS: MH⁺= 499 (tR = 5.12 minutes)
RMN ^{1H} δ en ppm (DMSO d 6) : 2.15-2.50 (m, 4H); 3.0-3.20 (m, 2H); 3.40-3.60 (m, 2H); 3.89 (s, 3H); 4.09 (s, 3H); 4.20-4.30 (m, 1H); 4.40 (s, 2H); 7.21 (d, J = 8.5 Hz, 2H); 7.63 (d, J = 8.5 Hz, 2H); 7.72 (dd, J₁ = 9 Hz, J₂ = 2.2 Hz, 1H); 7.83 (d, J = 8.2 Hz, 2H); 7.93 (d, J = 9 Hz, 1H); 8.04 (d, J = 8.2 Hz, 2H); 8.46 (s, 1H); 11.16 (s, 1H, COOH); 13.17 (s, 1H, NH);.

### 53.2 Dichlorhydrate de la N-{4-[(4-{[7-méthoxy-4-(4-méthoxyphényl)phthalazin-1-yl]amino}pipéridin-1-yl)méthyl]phényl}carboamide

Une suspension de 400 mg (0.7 mmol d'acide {4-[(4-{[7-méthoxy-4-(4-méthoxyphényl)phthalazin-1-yl]amino}pipéridin-1-yl)méthyl]phényl}carboxylique (dichlorhydrate) dans 10 mL de tétrahydrofuranne est agitée à 0°C et additionnée de 0.29 mL (2.3 mmol) de N-éthyl-morpholine puis goutte à goutte de 0.074 mL (0.77 mmol) de chloroformate d'éthyle. Le mélange est agité pendant 1 heure à 0°C puis 0.6 mL (3.5 mmol) d'une solution aqueuse d'ammoniaque à 20% est additionnée lentement. Le milieu réactionnel est agité pendant 20 heures à température ambiante puis est concentré sous pression réduite. Le résidu est additionné de soude 1N puis extrait au dichlorométhane.

La phase organique est lavée par de l'eau saturée en chlorure de sodium, séchée sur sulfate de sodium anhydre, puis évaporée sous pression réduite. Le résidu est purifié sur colonne de silice (éluant dichlorométhane/méthanol 85/15 (v/v)). 150 mg d'une huile incolore sont obtenus.
LC/MS: MH⁺= 498 (tR = 4.88 minutes)
RMN ^{1H} δ en ppm (DMSO d 6) : 1.65-1.80 (m, 2H); 2.05-2.25 (m, 4H); 2.85-2.95 (m, 2H); 3.58 (s, 2H); 3.85 (s, 3H); 3.98 (s, 3H); 4.20-4.30 (m, 1H); 6.99 (d, J = 7.3 Hz, 1H, NH); 7.09 (d, J = 6.7 Hz, 2H); 7.30 (s, 1H); 7.40-7.45 (m, 3H); 7.51 (d, J = 6.7 Hz, 2H); 7.72-7.76 (m, 2H); 7.86 (d, J = 8.2 Hz, 2H); 7.93 (s, 1H).

Le dichlorhydrate est obtenu après traitement avec une solution d'acide chlorhydrique dans l'éther diéthylique.

### Exemple 54 (composés n°139)

### Dichlorhydrate du 1 -{4-[(4-{[7-méthoxy-4-(4-méthoxyphényl)phthalazin-1-yl]amino} pipéridin-1-yl)méthyl]phényl}éthanol

Une suspension de 300 mg (0.6 mmol) de 1-{4-[(4-{[7-méthoxy-4-(4-méthoxyphényl)phthalazin-1-yl]amino}pipéridin-1-yl)méthyl]phényl}éthanone (composé 121) dans 6 mL de méthanol est additionnée de 56 mg (1.2 mmol) de borohydrure de sodium. Le mélange est agité pendant 24 heure à température ambiante puis 30 minutes à reflux. Le milieu réactionnel refroidi à 10°C est additionné de 1 mL d'acétone puis évaporé sous pression réduite. Le résidu est purifié sur colonne de silice (éluant dichlorométhane/méthanol de 100/0 à 85/15 (v/v)). 300 mg de poudre blanche sont obtenus.
LC/MS: MH⁺= 499 (tR = 5.06 minutes)
RMN ^{1H} δ en ppm (DMSO d 6) : 1.07 (d, J = 7 Hz, 3H);1.65-1.80 (m, 2H); 2.05-2.25 (m, 4H); 2.85-2.95 (m, 2H); 3.51 (s, 2H); 3.85 (s, 3H); 3.98 (s, 3H); 4.20-4.30 (m, 1H); 4.68-4.77 (m, 1H); 5.10 (d, J = 4.2 Hz, 1H, OH); 6.99 (d, J = 7.5 Hz, 1H, NH); 7.09 (d, J = 8.7 Hz, 2H); 7.26-7.34 (m, 4H); 7.43 (dd, J₁ = 9 Hz, J₂ = 2.5 Hz, 1H); 7.52 (d, J = 8.7 Hz, 2H); 7.73-7.77 (m, 2H).

Le dichlorhydrate est obtenu après traitement avec une solution d'acide chlorhydrique dans l'éther diéthylique.

### Exemple 55 (composés n°140)

### Trichlorhydrate de la 7-méthoxy-4-(4-méthoxyphényl)-N-{1-[4-(pyrrolidin-1-yl-méthyl)-benzyl]-pipéridin-4-yl}-phthalazin-1-amine

Une solution de 360 mg (0.65 mmol) de 7-méthoxy-4-(4-méthoxyphényl)-N-{I-[4-(pyrrolidin-1-yl-carboxy)-benzyl]-pipéridin-4-yl}-phthalazin-1-amine dans 6 mL de tétrahydrofuranne est agitée à 0°C et additionnée de 50 mg (1.3 mmol) d'hydrure d'aluminium lithium. Le mélange est chauffé à reflux pendant 3 heures puis refroidi et hydrolysé successivement par 0.25 mL d'eau, 0.25 mL de soude 6N et 0.75 mL d'eau. La suspension obtenue est filtrée et évaporée sous pression réduite. Le résidu est purifié sur colonne de silice (éluant dichlorométhane/méthanol de 100/0 à 80/20 (v/v)). 40 mg de produit gommeux blanc sont obtenus.
LC/MS: MH⁺= 538 (tR = 4.50 minutes)

Le dichlorhydrate est obtenu après traitement avec une solution d'acide chlorhydrique dans l'éther diéthylique.

### Exemple 56 (composés n°151)

### Trichlorhydrate de la N-[1-(4-diméthylaminométhyl-benzyl)-pipéridin-4-yl]-7-méthoxy-4-(4-méthoxyphényl)phthalazin-1-amine

Un mélange de 400 mg (0.82 mmol) de N-[1-(4-aminométhyl-benzyl)-pipéridin-4-yl]-7-méthoxy-4-(4-méthoxyphényl)phthalazin-1-amine et 0.2 mL (5 mmol) d'acide formique est agité à 0°C et additionné de 0.32 mL (4.2 mmol) d'une solution aqueuse de formaldéhyde à 37% et de 0.5 mL d'eau. Le milieu réactionnel est agité à 90°C pendant 8 heures puis à température ambiante pendant 16 heures. Il est additionné de soude 1N puis extrait au dichlorométhane. La phase organique est lavée par de l'eau saturée en chlorure de sodium, séchée sur sulfate de sodium anhydre, puis évaporée sous pression réduite. Le résidu est purifié en utilisant une résine d'isocyanate de méthyl greffé sur polystyrène. 300 mg de produit sous forme d'une huile jaune sont obtenus.
LC/MS: MH⁺= 512 (tR = 4.43 minutes)
RMN ^{1H} δ en ppm (DMSO d 6) : 1.65-1.80 (m, 2H); 2.00-2.15 (m, 4H); 2.15 (s, 6H); 2.85-2.95 (m, 2H); 3.37 (s, 2H); 3.51 (s, 2H); 3.85 (s, 3H); 3.98 (s, 3H); 4.20-4.30 (m, 1H); 6.99 (d, J = 7.6 Hz, 1H, NH); 7.09 (d, J = 8.5 Hz, 2H); 7.20-7.34 (m, 4H); 7.43 (dd, J₁ = 9 Hz, J₂ = 2.3 Hz, 1H); 7.52 (d, J = 8.5 Hz, 2H); 7.73-7.77 (m, 2H).

Le trichlorhydrate est obtenu après traitement avec une solution d'acide chlorhydrique dans l'éther diéthylique.

### Exemple 57 (composés n°94)

### Dichlorhydrate de la 4-(4-méthoxyphényl)-7-méthoxy-N-[(3R)-1-(naphthalèn-2-ylméthyl)pyrrolidin-3-yl]phthalazin-1-amine

### 57.1 (3R)-1-(Naphthalèn-2-ylméthyl)-3-tert-butyloxycarbonylamino-pyrrolidine

Ce composé a été préparé par la méthode d'amination réductrice décrite en 49.2 par réaction de la (R)-3-tert-butyloxycarbonylamino-pyrrolidine avec le 2-naphtaldéhyde.

Le résidu est purifié sur colonne de silice (éluant dichlorométhane/méthanol de 100/0 à 90/10 (v/v)).
LC/MS: MH⁺= 327 (tR = 6.34 minutes)
RMN ^{1H} δ en ppm (DMSO d 6) : 1.37 (m, 10H); 1.50-1.65 (m, 1H);1.95-2.15 (m,1H); 2.25-2.35 (m, 1H); 2.52 (m, 1H); 2.70-2.80 (m, 1H); 3.71 (s, 2H); 3.85-3.95 (m, 1H); 6.95 (d, 1H,NH); 7.45-7.55 (m, 3H); 7.80 (s, 1H); 7.85-7.95 (m, 3H).

### 57.2. (3R)-1-(naphthalèn-2-ylméthyl)pyrrolidin-3-ylamine

Une solution de 2.5g (7.66mmol) du composé 57-1 dans 25 ml HCl-EtOAc 2.5M est chauffée à 80°C pendant 6 heures puis à température ambiante pendant 16 heures. Le milieu réactionnel est filtré.Le précipité est lavé à l'acétate d'éthyle puis séché à l'étuve .II est repris par un mélange dichlorométhane et soude 1N puis extrait au dichlorométhane.

La phase organique est lavée par de l'eau saturée en chlorure de sodium, séchée sur sulfate de sodium anhydre, puis évaporée sous pression réduite.
LC/MS: MH⁺= 227 (tR = 2.71 minutes)
RMN ^{1H} δ en ppm (DMSO d 6) : 1.35-1.45 (m, 1H); 1.50-1.85 (m, 1H);1.95-2.15 (m,1H); 2.15-2.20 (m, 1H); 2.45-2.75 (m, 4H); 3.30-3.40 (m, 1H); 3.65-3.8 (m, 2H); 7.4-7.55 (m, 3H); 7.80 (s, 1H); 7.85-7.95 (m, 3H).

### 57.3. 4-(4-méthoxyphényl)-7-méthoxy-N-[(3R)-1-(naphthalèn-2-ylméthyl)pyrrolidin-3-yl]phthalazin-1-amine

Ce composé est obtenu selon la méthode décrite à l'étape 1.4.de l'exemple 1 par réaction de la (3R)-1-(naphthalèn-2-ylméthyl)pyrrolidin-3-ylamine avec la 1-Chloro-7-méthoxy-4-(4-méthoxy-phényl)-phthalazine. Le résidu est purifié sur colonne de silice (éluant dichlorométhane/méthanol de 100/0 à 85/15 (v/v)).
LC/MS: MH⁺= 491 (tR = 5.93 minutes)

Le dichlorhydrate est obtenu après traitement avec une solution d'acide chlorhydrique dans l'éther diéthylique
[α]_{D}²⁰= - 50 (c=1, MeOH)

### Exemple 58 (composés n°96)

### Dichlorhydrate de la 4-(4-méthoxyphényl)-7-méthoxy-N-[(3S)-1-(naphthalèn-2-ylméthyl)pyrrolidin-3-yl]phthalazin-1-amine

### 58.1. (3S)-1-(Naphthatèn-2-ytméthyl)-3-tert-butyoxycarbonylamino-pyrrolidine

Ce composé a été obtenu par la méthode d'amination réductrice décrite en 49.2 par réaction de la (S)-3-tert-butyloxycarbonylamino-pyrrolidine avec le 2-naphtaldéhyde.

Le résidu est purifié sur colonne de silice (éluant dichlorométhane/méthanol de 100/0 à 90/10 (v/v)).
LC/MS: MH⁺= 327 (tR = 6.40 minutes)
RMN ^{1H} δ en ppm (DMSO d 6) : 1.37 (m, 10H); 1.50-1.65 (m, 1H);1.95-2.15 (m,1H); 2.25-2.35 (m, 1H); 2.52 (m, 1H); 2.70-2.80 (m, 1H); 3.71 (s, 2H); 3.85-3.95 (m, 1H); 6.95 (d, 1H,NH); 7.45-7.55 (m, 3H); 7.80 (s, 1H); 7.85-7.95 (m, 3H).

### 58.2. (3S)-1-(naphthalèn-2-ylméthyl)pyrrolidin-3-ylamine

La déprotection de l'amine a été faite selon la méthode décrite en 57.2.
LC/MS: MH⁺= 227 (tR = 2.89 minutes)
RMN ^{1H} δ en ppm (DMSO d 6) : 1.35-1.45 (m,1H); 1.50-1.85 (m, 1H);1.95-2.15 (m,1H); 2.15-2.20 (m, 1H); 2.45-2.75 (m, 4H); 3.30-3.40 (m, 1H); 3.65-3.8 (m, 2H); 7.40-7.55 (m, 3H); 7.80 (s, 1H); 7.85-7.95 (m, 3H).

### 58.3. 4-(4-méthoxyphényl)-7-méthoxy-N-[(3S)-1-(naphthalèn-2-ylméthyl)pyrrolidin-3-yl]phthalazin-1-amine

Ce composé est obtenu selon la méthode décrite à l'étape 1.4.de l'exemple 1. par réaction de la (3S)-1-(naphthalèn-2-ylméthyl)pyrrolidin-3-ylamine avec la 1-Chloro-7-méthoxy-4-(4-méthoxy-phényl)-phthalazine. Le résidu est purifié sur colonne de silice (éluant dichlorométhane/méthanol de 100/0 à 85/15 (v/v)).
LC/MS: MH⁺= 491 (tR = 5.93 minutes)

Le dichlorhydrate est obtenu après traitement avec une solution d'acide chlorhydrique dans l'éther diéthylique
[α]_{D}²⁰ = +47 (c=1, MeOH)

**Tableau I**

| | | | | | | | | | | |
|---|---|---|---|---|---|---|---|---|---|---|
| 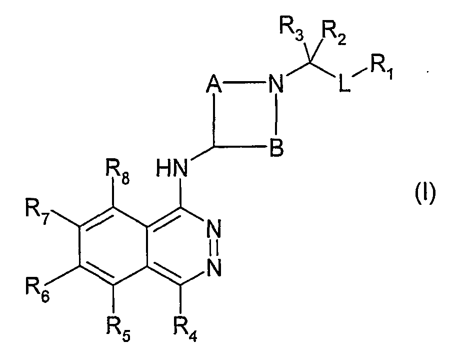 | | | | | | | | | | |

| N° | 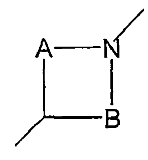 | 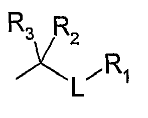 | R₄ | R₅ | R₆ | R₇ | R₈ | Sel et/ou Solvats | PF (°C) | LC/MS (M+H)+; tR (minute) |
|---|---|---|---|---|---|---|---|---|---|---|
| 1 | 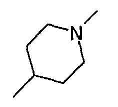 | 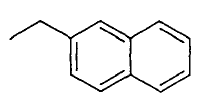 | 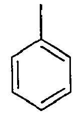 | H | H | OMe | H | H₂O (0.25/1) | 121 (K) | 475 ; tR = 5.15 |
| 2 | 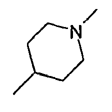 | 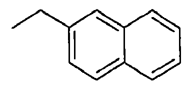 | 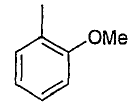 | H | H | OMe | H | H₂O (0.75/1) | 158 (M) | 505 ; tR = 5.16 |
| 3 | 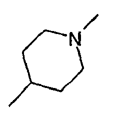 | 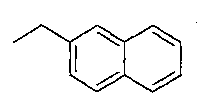 | 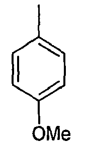 | H | H | OMe | H | H₂O (0.25/1) iPr₂O (0.2/1) | 195 (M) | 505 ; tR = 5.28 |
| 4 | 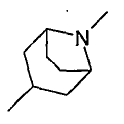 | 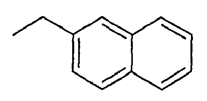 | 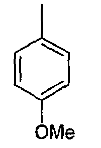 | H | H | OMe | H | H₂O (0.25/1) | 176 (M) | 531 ; tR = 5.43 |
| 5 | 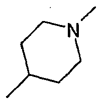 | 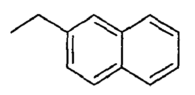 | 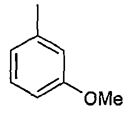 | H | H | OMe | H | H₂O (0.5/1) | 154 (M) | 505 ; tR = 5.28 |
| 6 | 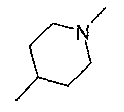 | 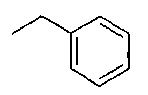 | 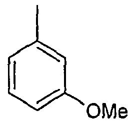 | H | H | OMe | H | H₂O (0.25/1) | 176 (M) | 455 ; tR = 4.80 |
| 7 | 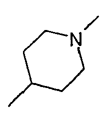 | 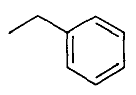 | 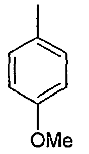 | H | H | OMe | H | H₂O (0.25/1) iPr₂O (0.15/1) | 150 (M) | 455 ; tR = 4.71 |
| 8 | 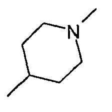 | 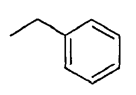 | 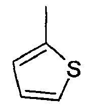 | H | H | OMe | H | H₂O (0.3/1) iPr₂O (0.2/1) | 115 (M) | 431 ; tR = 4.72 |
| 9 | 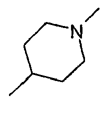 | 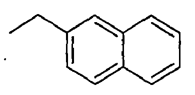 | 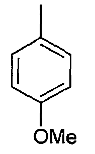 | H | H | Me | H | H₂O (0.3/1) | 198 (M) | 489 ; tR = 5.27 |
| 10 | 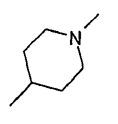 | 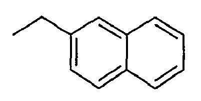 | 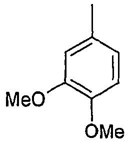 | H | H | OMe | H | H₂O (0.5/1) | 131 (K) | 535 ; tR = 5.13 |
| 11 | 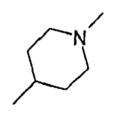 | 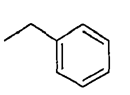 | 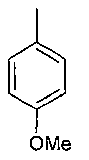 | OMe | H | OMe | H | - | 168 (K) | 485 ; tR = 4.82 |
| 12 | 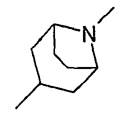 | 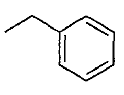 | 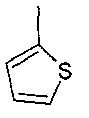 | H | H | OMe | H | H₂O (0.5/1) | 209 (M) | 457 ; tR = 7.93 |
| 13 | 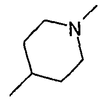 | 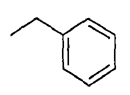 | H | OMe | H | OMe | H | H₂O (0.5/1) | 169 (M) | 379 ; tR = 4.05 |
| 14 | 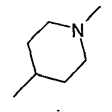 | 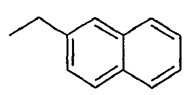 | H | OMe | H | OMe | H | H₂O (0.5/1) | 206 (M) | 429 ; tR = 4.60 |
| 15 | 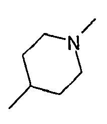 | 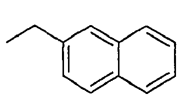 | 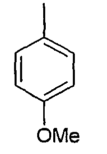 | OMe | H | OMe | H | HCl (2/1) H₂O (1.5/1) | 212 (K) | 535 ; tR = 5.26 |
| 16 | 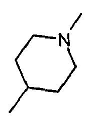 | 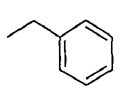 | 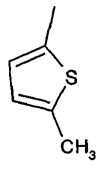 | H | H | OMe | H | H₂O (0.7/1) iPr₂O (0.1/1) | 155 (M) | 445 ; tR = 5.02 |
| 17 | 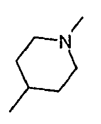 | 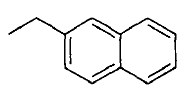 | H | 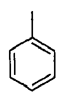 | H | OMe | H | H₂O (2/1) Et₂O (0.23/1) | 199 (K) | 475 ; tR = 5.70 |
| 18 | 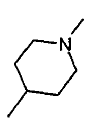 | 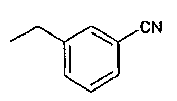 | 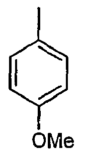 | H | H | OMe | H | H₂O (0.5/1) iPr₂O (0.15/1) | 115 (M) | 480 ; tR = 4.24 |
| 19 | 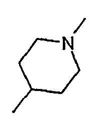 | 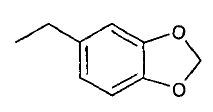 | 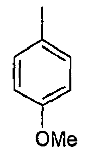 | H | H | OMe | H | HCl (2/1) H₂O (1/1) | 235 (B) | 499 ; tR = 5.31 |
| 20 | 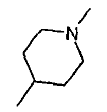 | 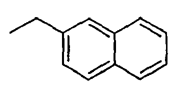 | Et | H | H | OMe | H | H₂O (0.85/1) | 135 (M) | 427 ; tR = 5.16 |
| 21 | 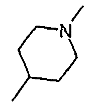 | 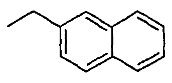 | 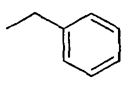 | H | H | OMe | H | H₂O (0.7/1) | 104 (M) | 489 ; tR = 5.71 |
| 22 | 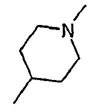 | 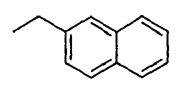 | 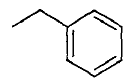 | OMe | H | OMe | H | - | 163 (M) | 519 ; tR = 5.92 |
| 23 | 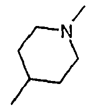 | 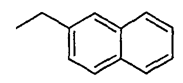 | Et | OMe | H | OMe | H | H₂O (0.6/1) | 250 (M) | 457 ; tR = 4.48 |
| 24 | 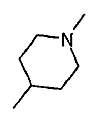 | 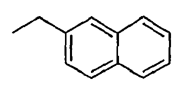 | -CH₂-O-CH₃ | H | H | OMe | H | HCl (1/1) H₂O (1.2/1) | 176 (M) | 443 ; tR = 4.01 |
| 25 | 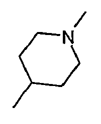 | 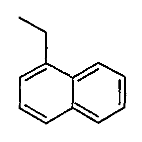 | 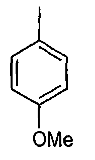 | H | H | OMe | H | H₂O (0.25/1) | 223 (M) | 505 ; tR = 4.77 |
| 26 | 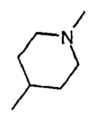 | 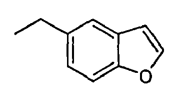 | 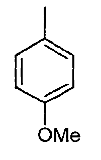 | H | H | OMe | H | H₂O (0.8/1) | 160 (M) | 495 ; tR = 4.53 |
| 27 | 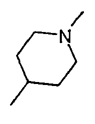 | 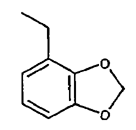 | 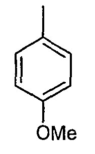 | H | H | OMe | H | H₂O (0.5/1) | 180 (M) | 499 ; tR = 4.39 |
| 28 | 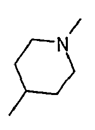 | 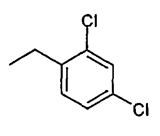 | 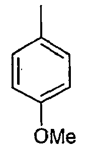 | H | H | OMe | H | H₂O (0.5/1) | 212 (K) | 523 ; tR = 4.56 |
| 29 | 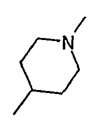 | 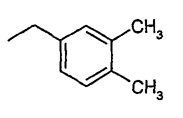 | 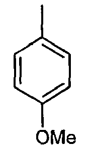 | H | H | OMe | H | H₂O (0.5/1) | 130 (K) | 483 ; tR = 4.53 |
| 30 | 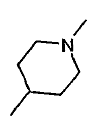 | 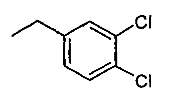 | 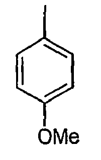 | H | H | OMe | H | H₂O (0.5/1) | 208 (K) | 523 ; tR = 4.83 |
| 31 | 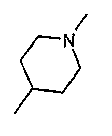 | 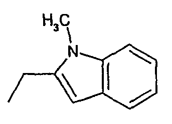 | 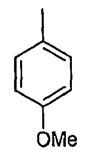 | H | H | OMe | H | H₂O (0.75/1) | 122 (K) | 508 ; tR = 4.91 |
| 32 | 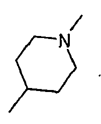 | 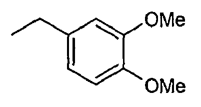 | 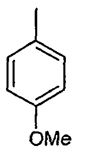 | H | H | OMe | H | H₂O (0.75/1) | 110 (K) | 515 ; tR = 4.44 |
| 33 | 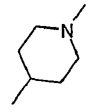 | 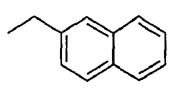 | 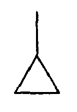 | H | H | OMe | H | H₂O (0.1/1) | 154 (K) | 439 ; tR = 4.52 |
| 34 | 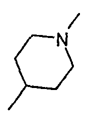 | 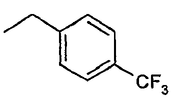 | 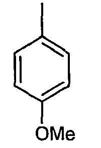 | H | H | OMe | H | H₂O (0.8/1) | 214 (K) | 523 ; tR = 10.08 |
| 35 | 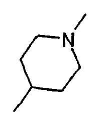 | 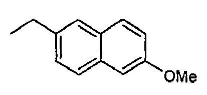 | 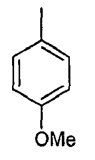 | H | H | OMe | H | H₂O (1/1) iPr₂O (0.3/1) | 119 (K) | 535 ; tR = 4.90 |
| 36 | 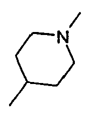 | 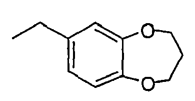 | 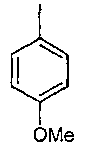 | H | H | OMe | H | H₂O (1.1/1) | 130 (K) | 527 ; tR = 4.71 |
| 37 | 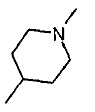 | 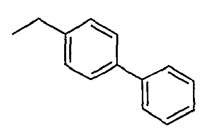 | 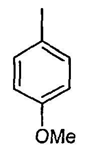 | H | H | OMe | H | iPr₂O (0.15/1) | 228 (K) | 531 ; tR = 5.17 |
| 38 | 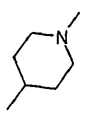 | 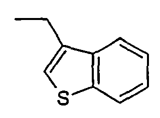 | 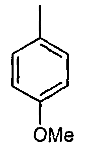 | H | H | OMe | H | - | 236 (K) | 511 ; tR = 4.51 |
| 39 | 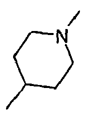 | 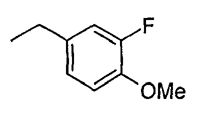 | 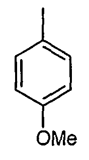 | H | H | OMe | H | H₂O (0.5/1) | 280 (M) | 503 ; tR = 4.68 |
| 40 | 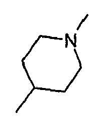 | 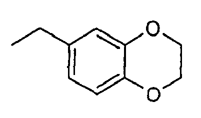 | 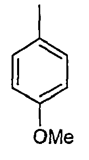 | H | H | OMe | H | H₂O (0.6/1) | 228 (K) | 513 ; tR = 4.22 |
| 41 | 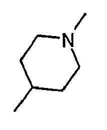 | 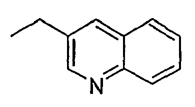 | 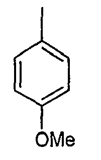 | H | H | OMe | H | (0.6/1) | 190 (K) | 506 ; tR = 4.20 |
| 42 | 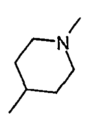 | 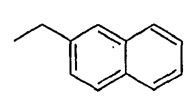 | 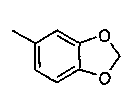 | H | H | OMe | H | HCl (2/1) H₂O (3.5/1) | 272 (M) | 519 ; tR = 4.66 |
| 43 | 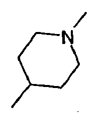 | 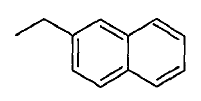 | 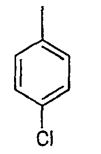 | H | H | OMe | H | (H₂O (1.5/1) | 181 (M) | 509 ; tR = 5.22 |
| 44 | 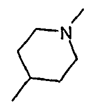 | 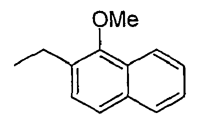 | 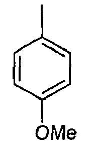 | H | H | OMe | H | H₂O (0.25/1) | 265 (M) | 535 ; tR = 4.98 |
| 45 | 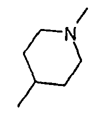 | 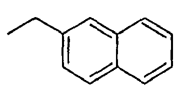 | 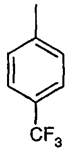 | H | H | OMe | H | H₂O (0.75/1) | 230 (K) | 543 ; tR = 5.27 |
| 46 | 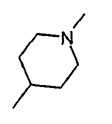 | 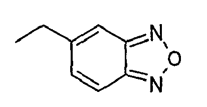 | 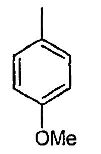 | H | H | OMe | H | H₂O (0.5/1) | 201 (M) | 497 ; tR = 4.64 |
| 47 | 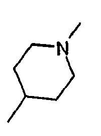 | 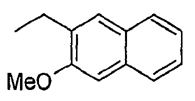 | 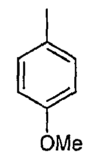 | H | H | OMe | H | H₂O (1/1) | 200 (M) | 535 ; tR = 4.97 |
| 48 | 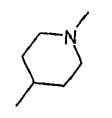 | 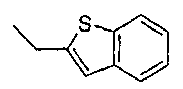 | 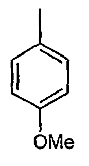 | H | H | OMe | H | H₂O (0.25/1) | 196 (M) | 511 ; tR = 4.84 |
| 49 | 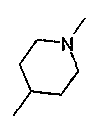 | 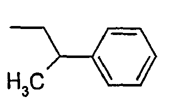 | 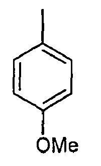 | H | H | OMe | H | H₂O (0.35/1) iPr₂O (0.2/1) | 149 (M) | 483 ; tR = 4.69 |
| 50 | 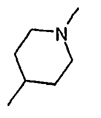 | 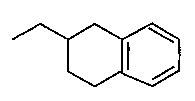 | 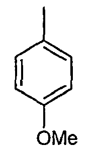 | H | H | OMe | H | H₂O (0.55/1) | 266 (M) | 509 ; tR = 4.82 |
| 51 | 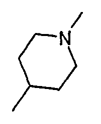 | 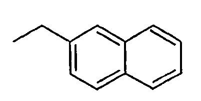 | 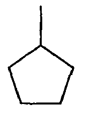 | H | H | OMe | H | HCl (2/1) H₂O (2.2/1) | 244 (K) | 467 ; tR = 4.84 |
| 52 | 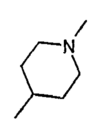 | 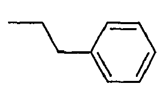 | 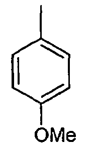 | H | H | OMe | H | H₂O (0.5/1) iPr₂O (0.15/1) | 152 (M) | 469 ; tR = 4.23 |
| 53 | 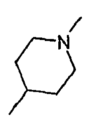 | 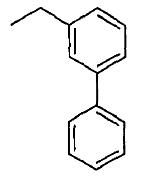 | 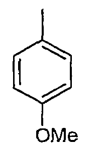 | H | H | OMe | H | H₂O (1.5/1) | 212 (M) | 531 ; tR = 6.14 |
| 54 | 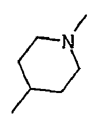 | 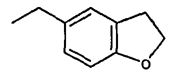 | 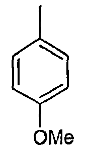 | H | H | OMe | H | H₂O (0.9/1) | 142 (M) | 497 ; tR = 5.41 |
| 55 | 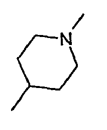 | 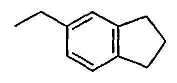 | 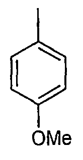 | H | H | OMe | H | H₂O (0.3/1) | 162 (M) | 495 ; tR = 5.71 |
| 56 | 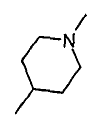 | 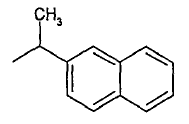 | 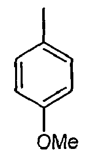 | H | H | OMe | H | HCl (2/1) H₂O (3.6/1) | 274 (M) | 519 ; tR = 5.87 |
| 57 | 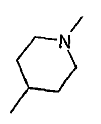 | 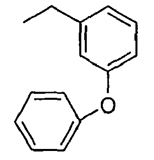 | 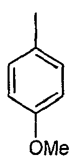 | H | H | OMe | H | H₂O (0.4/1) iPr₂O (0.2/1) | 98 (M) | 547 ; tR = 5.34 |
| 58 | 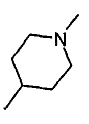 | 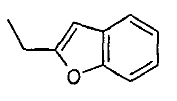 | 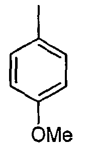 | H | H | OMe | H | H₂O (0.4/1) iPr₂O (0.04/1) | 186 (M) | 495 ; tR = 4.74 |
| 59 | 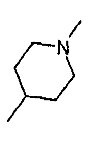 | 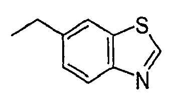 | 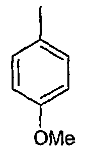 | H | H | OMe | H | H₂O (0.6/1) iPr₂O (0.02/1) | 180 (K) | 512 ; tR = 5.24 |
| 60 | 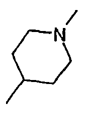 | 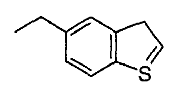 | 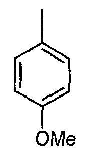 | H | H | OMe | H | H₂O (0.25/1) iPr₂O (0.04/1) | 176 (K) | 511 ; tR = 5.69 |
| 61 | 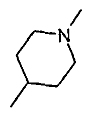 | 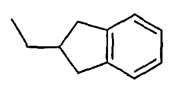 | 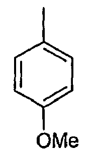 | H | H | OMe | H | H₂O (1.8/1) iPr₂O (0.1/1) | 118 (K) | 495 ; tR = 4.40 |
| 62 | 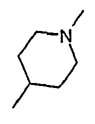 | 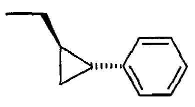 | 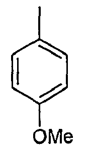 | H | H | OMe | H | H₂O (1.3/1) iPr₂O (0.13/1) | 114 (K) | 495 ; tR = 4.45 |
| 63 | 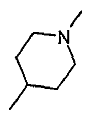 | 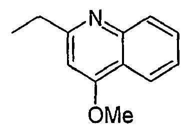 | 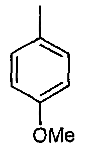 | H | H | OMe | H | H₂O (0.7/1) iPr₂O (0.03/1) | 172 (M) | 536 ; tR = 5.71 |
| 64 | 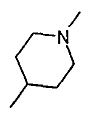 | 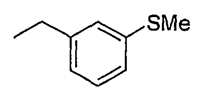 | 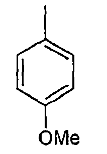 | H | H | OMe | H | H₂O (0.2/1) iPr₂O (0.05/1) | 176 (M) | 501 ; tR = 5.53 |
| 65 | 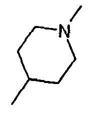 | 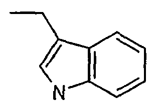 | 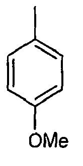 | H | H | OMe | H | H₂O (0.5/1) iPr₂O (0.07/1) | 257 (M) | 494 ; tR = 4.82 |
| 66 | 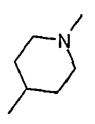 | 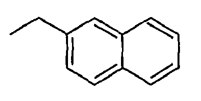 | 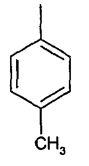 | H | H | OMe | H | H₂O (0.5/1) iPr₂O (0.01/1) | 135 (M) | 489 ; tR = 5.16 |
| 67 | 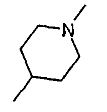 | 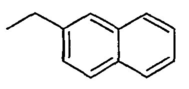 | nBut | H | H | OMe | H | H₂O (0.5/1) | 101 (M) | 455 ; tR = 5.59 |
| 68 | 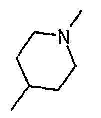 | 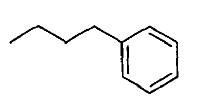 | 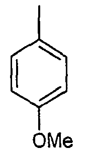 | H | H | OMe | H | H₂O (0.5/1) | 196 (M) | 483 ; tR = 5.50 |
| 69 | 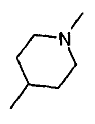 | 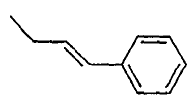 | 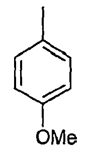 | H | H | OMe | H | HCl (2/1) H₂O (2.8/1) | 210 (K) | 481 ; tR = 5.67 |
| 70 | 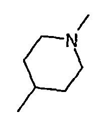 | 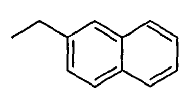 | H | H | H | OMe | H | HCl (0.75/1) H₂O (0.2/1) | 189 (M) | 399 ; tR = 3.78 |
| 71 | 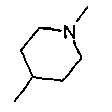 | 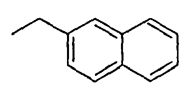 | 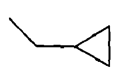 | H | H | OMe | H | - | n.d. | 453 ; tR = 4.45 |
| 72 | 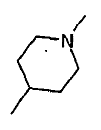 | 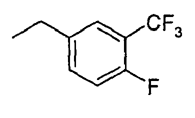 | 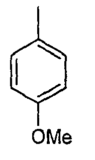 | H | H | OMe | H | - | 172 (M) | 541 ; tR = 5.79 |
| 73 | 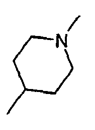 | 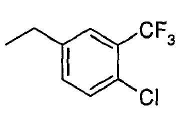 | 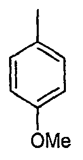 | H | H | OMe | H | - | 190 (K) | 557 ; tR = 5.96 |
| 74 | 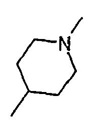 | 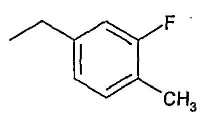 | 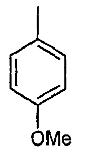 | H | H | OMe | H | - | 210 (M) | 487 ; tR = 5.53 |
| 75 | 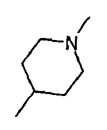 | 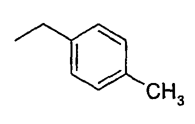 | 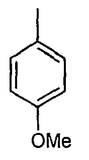 | H | H | OMe | H | HCl (0.75/1) iPr₂O (0.3/1) | 148 (M) | 469 ; tR = 5.44 |
| 76 | 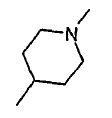 | 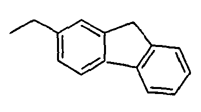 | 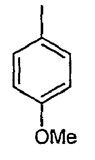 | H | H | OMe | H | H₂O (0.5/1) | 210 (M) | 543 ; tR = 6.07 |
| 77 | 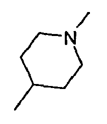 | 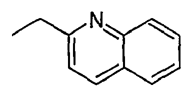 | 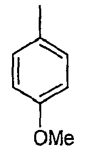 | H | H | OMe | H | H₂O (0.65/1) iPr₂O (0.01/1) | 124 (K) | 506 ; tR = 5.50 |
| 78 | 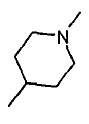 | 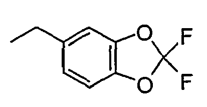 | 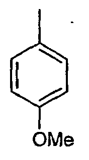 | H | H | OMe | H | - | 192 (M) | 535 ; tR = 5.72 |
| 79 | 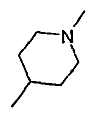 | 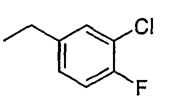 | 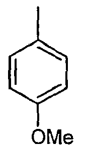 | H | H | OMe | H | - | 168 (M) | 507 ; tR = 5.55 |
| 80 | 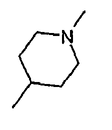 | 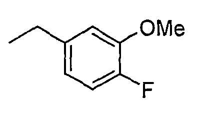 | 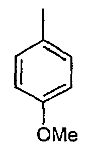 | H | H | OMe | H | H₂O (0.4/1) | 144 (M) | 503 ; tR = 5.41 |
| 81 | 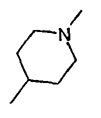 | 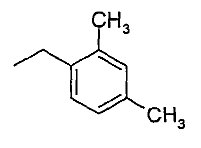 | 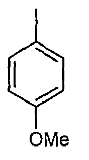 | H | H | OMe | H | HCl (2/1) H₂O (2.3/1) | 290 (M) | 483 ; tR = 5.55 |
| 82 | 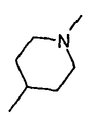 | 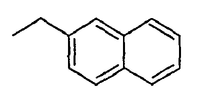 | 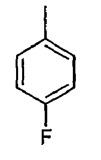 | H | H | OMe | H | H₂O (0.65/1) | 175 (M) | 493 ; tR = 5.77 |
| 83 | 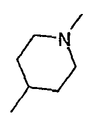 | 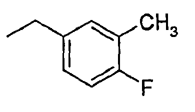 | 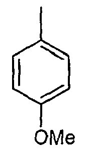 | H | H | OMe | H | - | 157 (M) | 487 ; tR = 5.51 |
| 84 | 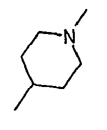 | 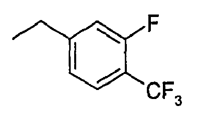 | 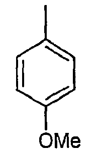 | H | H | OMe | H | - | 186 (M) | 541 ; tR = 5.85 |
| 85 | 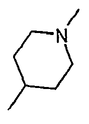 | 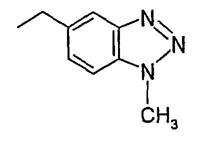 | 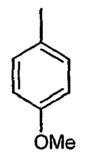 | H | H | OMe | H | H₂O (1/1) | 163 (M) | 510 ; tR = 5.07 |
| 86 | 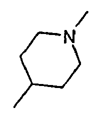 | 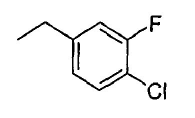 | 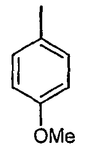 | H | H | OMe | H | - | 194 (M) | 507 ; tR = 5.58 |
| 87 | 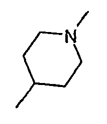 | 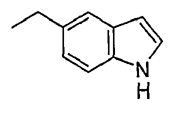 | 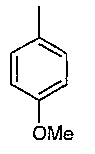 | H | H | OMe | H | H₂O (1.4/1) iPr₂O (0.3/1) | 281 (M) | 494 ; tR = 5.32 |
| 88 | 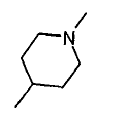 | 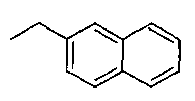 | 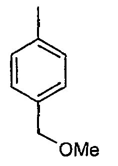 | H | H | OMe | H | H₂O (0.8/1) iPr₂O (0.3/1) | 155 (M) | 519 ; tR = 5.68 |
| 89 | 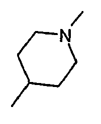 | 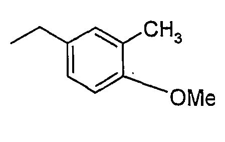 | 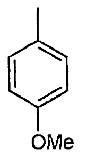 | H | H | OMe | H | H₂O (2/1) | 270 (M) | 499 ; tR = 5.55 |
| 90 | 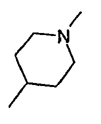 | 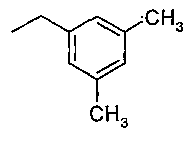 | 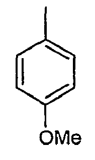 | H | H | OMe | H | H₂O (1.3/1) iPr₂O (0.2/1) | 277 (M) | 483 ; tR = 5.63 |
| 91 | 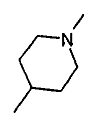 | 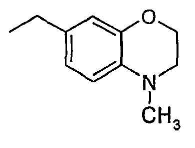 | 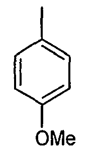 | H | H | OMe | H | H₂O (1.5/1) iPr₂O (0.2/1) | 251 (M) | 526 ; tR = 5.41 |
| 92 | 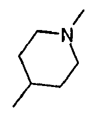 | 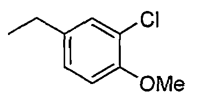 | 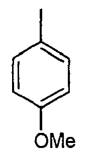 | H | H | OMe | H | H₂O (0.15/1) | 277 (M) | 518 ; tR = 5.54 |
| 93 | 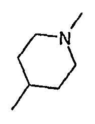 | 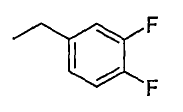 | 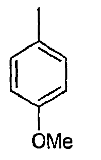 | H | H | OMe | H | H₂O (0.2/1) | 175 (M) | 491 ; tR = 5.40 |
| 94 (R) | 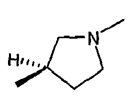 | 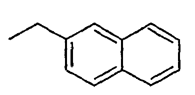 | 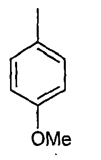 | H | H | OMe | H | HCl (2/1) H₂O (3/1) | 254 (M) | 491 ; tR = 5.96 |
| 95 | 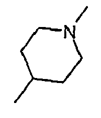 | 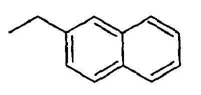 | Me | H | H | OMe | H | - | 188 (M) | 413 ; tR = 4.96 |
| 96 (S) | 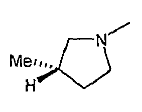 | 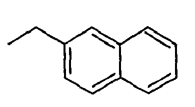 | 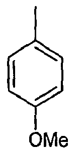 | H | H | OMe | H | HCl (2/1) H₂O (2.5/1) | 280 (M) | 491 ; tR = 5.96 |
| 97 | 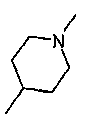 | 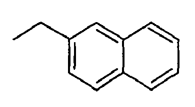 | 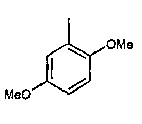 | H | H | OMe | H | HCl (2/1) H₂O (2.7/1) Et₂O (0.08/1) | 240 (B) | 535 ; tR = 5.77 |
| 98 | 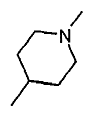 | 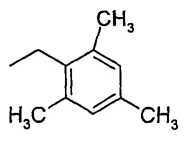 | 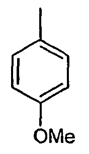 | H | H | OMe | H | H₂O (0.5/1) | 145 (M) | 497 ; tR = 5.66 |
| 99 | 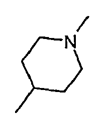 | 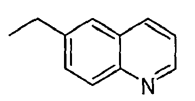 | 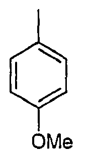 | H | H | OMe | H | H₂O (0.5/1) | 249 (M) | 506 ; tR = 4.00 |
| 100 | 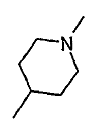 | 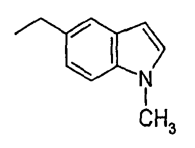 | 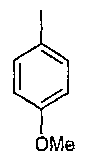 | H | H | OMe | H | H₂O (1.5/1) | 161 (M) | 508 ; tR = 4.47 |
| 101 | 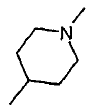 | 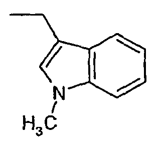 | 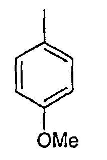 | H | H | OMe | H | HCl (2/1) H₂O (3.5/1) | 280 (M) | 508 ; tR = 5.64 |
| 102 | 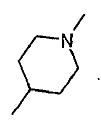 | 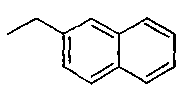 | 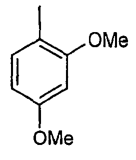 | H | H | OMe | H | HCl (2/1) H₂O (3/1) | 228 (K) | 535 ; tR = 5.80 |
| 103 | 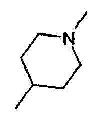 | 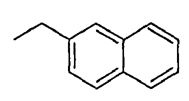 | 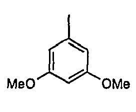 | H | H | OMe | H | HCl (2/1) H₂O (2.7/1) Et ₂O (0.06/1) | 240 (B) | 535 ; tR = 5.93 |
| 104 | 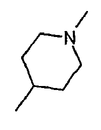 | 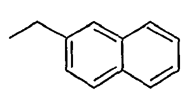 | 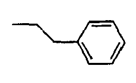 | H | H | OMe | H | HCl (2/1) H₂O (2.6/1) | 264 (M) | 503 ; tR = 5.87 |
| 105 | 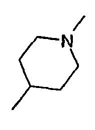 | 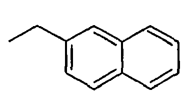 | 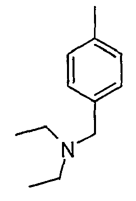 | H | H | OMe | H | HCl (3/1) H₂O (2.5/1) | 260 (K) | 560 ; tR = 4.87 |
| 106 | 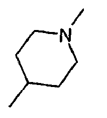 | 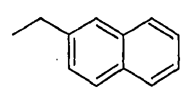 | 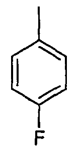 | H | H | F | H | - | 207 (M) | 481 ; tR = 6.04 |
| 107 | 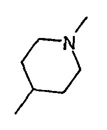 | 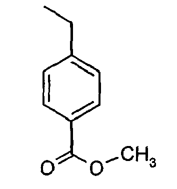 | 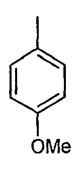 | H | H | OMe | H | HCl (2/1) H₂O (1.6/1) | 241 (M) | 513 ; tR = 5.36 |
| 108 | 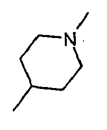 | 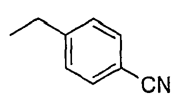 | 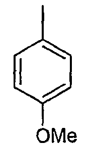 | H | H | OMe | H | HCl (2/1) H₂O (2.5/1) | 270 (M) | 480 ; tR = 5.23 |
| 109 | 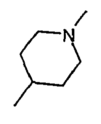 | 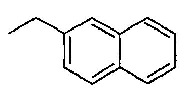 | 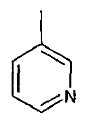 | H | H | OMe | H | HCl (2.6/1) H₂O (2.1/1) | 225 (B) | 476 ; tR = 5.25 |
| 110 | 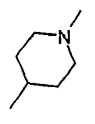 | 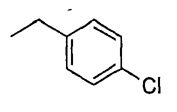 | 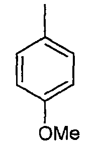 | H | H | OMe | H | HCl (2/1) H₂O (2/1) | 277 (M) | 489 ; tR = 5.51 |
| 111 | 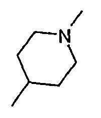 | 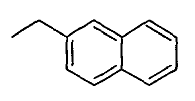 | 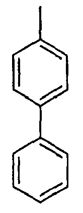 | H | H | OMe | H | HCl (2/1) H₂O (2.3/1) | 292 (M) | 551 ; tR = 6.53 |
| 112 | 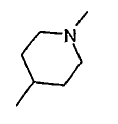 | 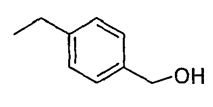 | 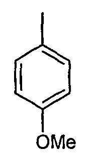 | H | H | OMe | H | HCl (2/1) H₂O (2.5/1) Et₂O (0.25/1) | 294 (M) | 485 ; tR = 4.98 |
| 113 | 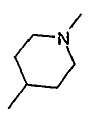 | 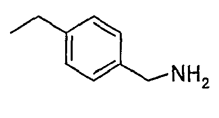 | 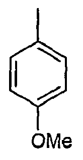 | H | H | OMe | H | HCl (3/1) H₂O (3.5/1) | 284 (B) | 484 ; tR = 4.38 |
| 114 | 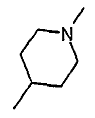 | 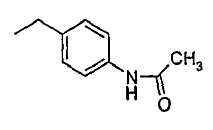 | 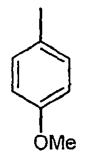 | H | H | OMe | H | HCl (2/1) H₂O (2.5/1) | 255 (B) | 512 ; tR = 5.04 |
| 115 | 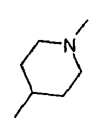 | 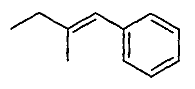 | 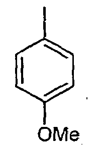 | H | H | OMe | H | H₂O (2/1) | 104 (M) | 495 ; tR = 5.74 |
| 116 | 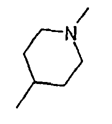 | 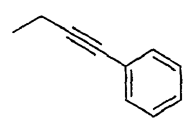 | 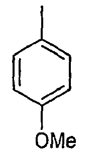 | H | H | OMe | H | H₂O (0.35/1) | 151 (M) | 479 ; tR = 5.68 |
| 117 | 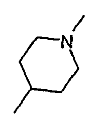 | 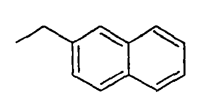 | 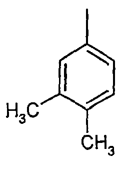 | H | H | OMe | H | HCl (2/1) H₂O (2/1) | 210 (B) | 503 ; tR = 6.04 |
| 118 | 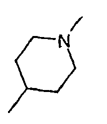 | 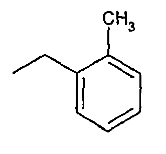 | 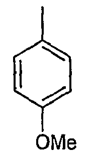 | H | H | OMe | H | HCl (2/1) H₂O (1.7/1) | 195 (M) | 469 ; tR = 5.44 |
| 119 | 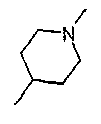 | 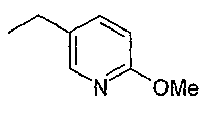 | 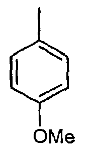 | H | H | OMe | H | HCl (0.6/1) iPr₂O (0.1/1) | 228 (M) | 486 ; tR = 5.14 |
| 120 | 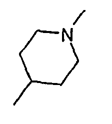 | 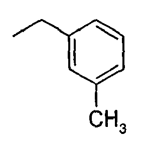 | 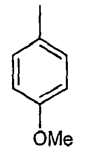 | H | H | OMe | H | HCl (2/1) H₂O (1.6/1) | 210 (B) | 469 ; tR = 5.44 |
| 121 | 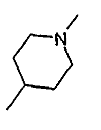 | 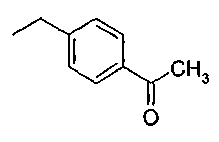 | 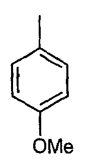 | H | H | OMe | H | HCl (2/1) H₂O (2.4/1) Et ₂O (0.15/1) | 241 (M) | 497 ; tR = 5.21 |
| 122 | 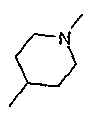 | 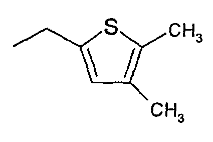 | 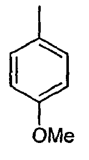 | H | H | OMe | H | HCl (2/1) H₂O (1.2/1) | 296 (M) | 489 ; tR = 5.56 |
| 123 | 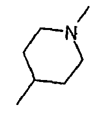 | 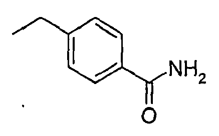 | 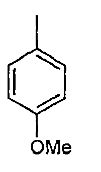 | H | H | OMe | H | HCl (2/1) H₂O (4/1) Et₂O (0.3/1) | 293 (M) | 498 ; tR = 4.88 |
| 124 | 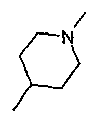 | 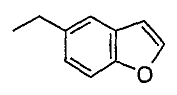 | 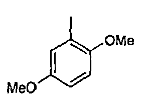 | H | H | OMe | H | HCl (2/1) H₂O (2/1) | 250 (M) | 525 ; tR = 5.50 |
| 125 | 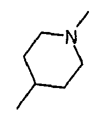 | 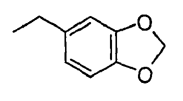 | 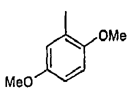 | H | H | OMe | H | HCl (2/1) H₂O (2.3/1) iPr₂O (0.2/1) | 272 (M) | 529 ; tR = 5.33 |
| 126 | 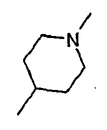 | 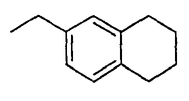 | 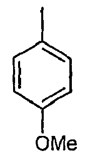 | H | H | OMe | H | HCl (2/1) H₂O (2.3/1) | 224 (M) | 509 ; tR = 5.91 |
| 127 | 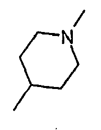 | 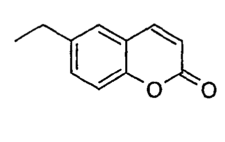 | 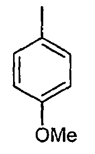 | H | H | OMe | H | HCl (2/1) H₂O (2.5/1) | 293 (M) | 523 ; tR = 5.21 |
| 128 | 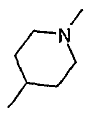 | 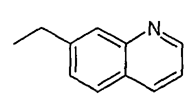 | 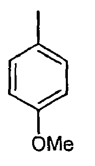 | H | H | OMe | H | HCl (3/1) H₂O (4.3/1) Et₂O (0.4/1) | 272 (M) | 506 ; tR = 5.17 |
| 129 | 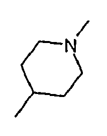 | 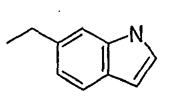 | 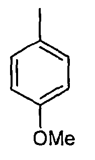 | H | H | OMe | H | H₂O (0.25/1) | 278 (M) | 494 ; tR = 5.49 |
| 130 | 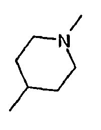 | 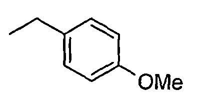 | 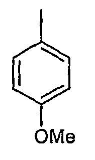 | H | H | OMe | H | HCl (2/1) H₂O (2.5/1) Et₂O (0.15/1) | 216 (K) | 485 ; tR = 5.34 |
| 131 | 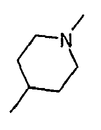 | 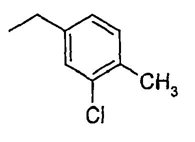 | 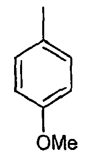 | H | H | OMe | H | HCl (2/1) H₂O (2/1) | 291 (M) | 503 ; tR = 5.71 |
| 132 | 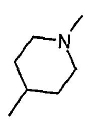 | 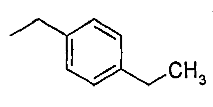 | 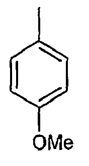 | H | H | OMe | H | HCl (1.7/1) H₂O (1/1) | 245 (M) | 483 ; tR = 5.64 |
| 133 | 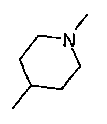 | 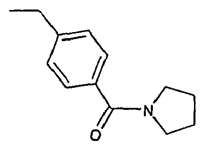 | 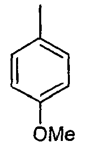 | H | H | OMe | H | HCl (2/1) H₂O (2.7/1) | 290 (M) | 552 ; tR = 5.21 |
| 134 | 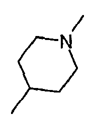 | 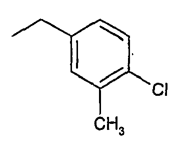 | 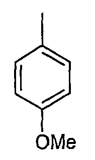 | H | H | OMe | H | HCl (2/1) H₂O (1.5/1) iPr₂O (0.4/1) | 280 (M) | 503 ; tR = 5.71 |
| 135 | 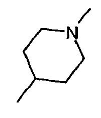 | 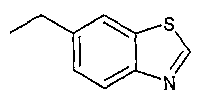 | 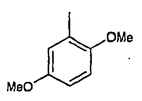 | H | H | OMe | H | H₂O (1/1) | 193 (M) | 542 ; tR = 5.21 |
| 136 | 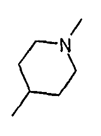 | 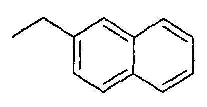 | 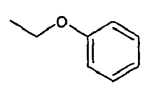 | H | H | OMe | H | HCl (2/1) H₂O (1.3/1) | 239 (M) | 505 ; tR = 6.06 |
| 137 | 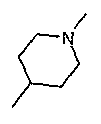 | 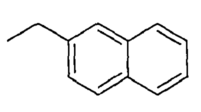 | 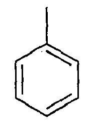 | H | H | 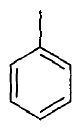 | H | HCl (2/1) H₂O (1.8/1) Et₂O (0.25/1) | 224 (M) | 521 ; tR = 6.48 |
| 138 | 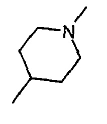 | 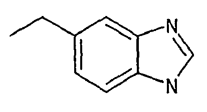 | 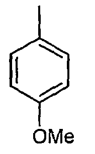 | H | H | OMe | H | - | 267 (M) | 495 ; tR = 4.62 |
| 139 | 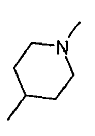 | 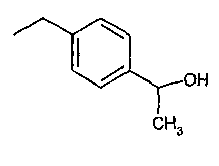 | 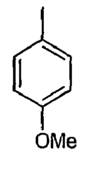 | H | H | OMe | H | HCl (2/1) H₂O (1.8/1) Et₂O (0.2/1) | 208 (K) | 499 ; tR = 5.07 |
| 140 | 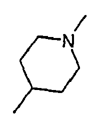 | 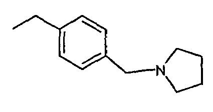 | 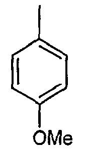 | H | H | OMe | H | HCl (3/1) H₂O (4.5/1) | 222 (K) | 538 ; tR = 4.49 |
| 141 | 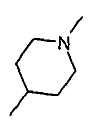 | 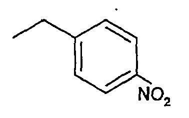 | 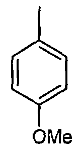 | H | H | OMe | H | H₂O (0.3/1) | 212 (M) | 500 ; tR = 5.32 |
| 142 | 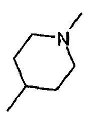 | 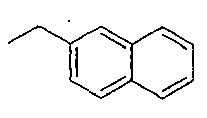 | 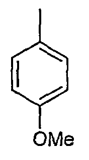 | H | Me | H | H | H₂O (0.2/1) | 134 (M) | 489 ; tR = 5.72 |
| 143 | 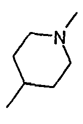 | 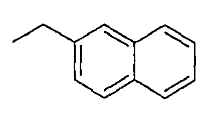 | 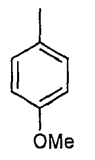 | H | H | Cl | H | HCl (2/1) H₂O (1.15/1) | 230 (K) | 509 ; tR = 6.13 |
| 144 | 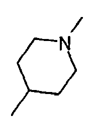 | 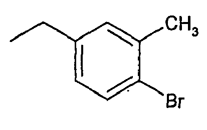 | 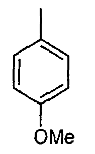 | H | H | OMe | H | HCl (2/1) H₂O (1.3/1) | 289 (M) | 549 ; tR = 5.76 |
| 145 | 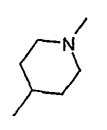 | 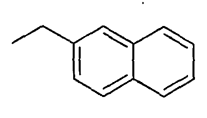 | 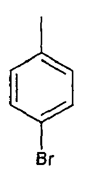 | H | H | OMe | H | HCl (2/1) H₂O (0.6/1) Et₂O (0.14/1) | 246 (M) | 555 ; tR = 6.13 |
| 146 | 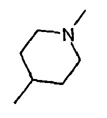 | 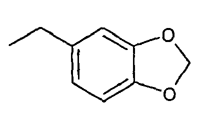 | -CH₂-O-CH₃ | H | H | OMe | H | HCl (2/1) H₂O (1/1) | 284 (M) | 437 ; tR = 4.48 |
| 147 | 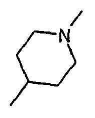 | 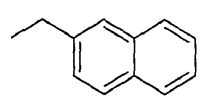 | 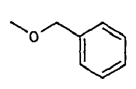 | H | H | OMe | H | HCl (2/1) Et₂O (0.23/1) | 284 (M) | 519 ; tR = 5.87 |
| 148 | 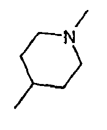 | 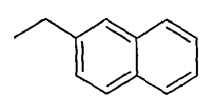 | -CH₂-OH | H | H | OMe | H | HCl (2/1) H₂O (2.8/1) | 186 (K) | 429 ; tR = 4.76 |
| 149 (-) | 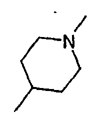 | 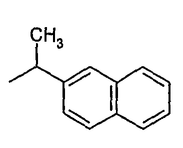 | 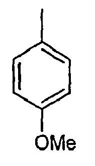 | H | H | OMe | H | Iso-hexane (0.5/1) H₂O (1.7/1) | 136 (M) | 519 ; tR = 5.76 |
| 150 (+) | 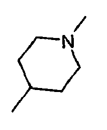 | 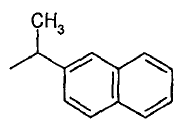 | 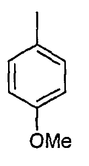 | H | H | OMe | H | Iso-hexane (0.4/1) H₂O (2.2/1) | 132 (M) | 519 ; tR = 5.76 |
| 151 | 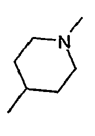 | 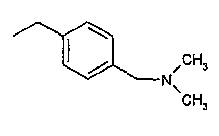 | 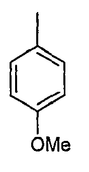 | H | H | OMe | H | HCl (3/1) H₂O (4.5/1) Et₂O (0.3/1) | 218 (K) | 512 ; tR = 4.40 |

Les composés selon l'invention ont fait l'objet d'essais pharmacologiques. Leur affinité vis-à-vis du récepteur 1 de la Melanin-Concentrating Hormone (MCH), MCH₁, a été ainsi déterminée.

Des essais ont consisté à mesurer l'activité *in vitro* des composés de l'invention sur les récepteurs MCH₁ de la MCH.

### Etudes de liaison :

La mesure de l'affinité des composés de l'invention pour les récepteurs de la MCH a été réalisée par l'étude du déplacement de la liaison d'un dérivé radio-marqué de la MCH aux récepteurs MCH₁. Cette étude est effectuée sur des préparations membranaires de cerveau de rat et/ou souris selon le protocole décrit ci-après.

En prévision des études de liaison, les cerveaux sont dilués dans du tampon HEPES 25 mM (pH : 7.4) contenant du MgCl₂ 5 mM, du CaCl₂ 1 mM, homogénéisés à l'aide d'un Polytron 3 fois 20 secondes (vitesse 25), puis subissent pendant 30 minutes une ultracentrifugation à 22 000 RPM à +4°C. Le culot est repris par le même tampon et les membranes sont aliquotées et conservées congelées à -80°C jusqu'à leur utilisation.

Les membranes sont ramenées à température ambiante puis sont incubées en présence des composés à tester, et de 50 pM d'une molécule radiomarquée dérivée de la MCH, le [¹²⁵I]-Tyr-S36057 (8-amino-3,6-dioxyoctanoyl MCH 6-17 commercialisé par Perkin-Elmer), dans du tampon HEPES 25 mM (pH : 7.4) contenant du MgCl₂ 5 mM, du CaCl₂ 1 mM, de la bacitracine 140 mg/L, de la phénantroline 1 mM, et 0.2 % d'albumine bovine sérique. L'incubation est réalisée à température ambiante pendant 30 minutes, puis arrêtée par addition rapide de tampon HEPES 25 mM (pH : 7.4) glacé, supplémenté par 0.2 % d'albumine bovine sérique, et par filtration sur filtres en fibre de verre GF/B pré-incubés pendant 2 heures dans une solution aqueuse de polyéthylèneimine à 0.1 %. La radioactivité retenue sur les filtres est mesurée à l'aide d'un compteur à scintillation Gamma. La liaison non spécifique est déterminée en présence de 1 µM de S36057 non-radiomarqué. La liaison spécifique est obtenue par différence entre la liaison totale et la liaison non spécifique. L'activité inhibitrice des composés de l'invention est exprimée par la concentration qui inhibe 50% de la liaison spécifique (CI₅₀).

Dans le cadre de l'invention, les CI₅₀ des composés sont généralement inférieures à 10µM.

Les composés de formule (I) présentent avantageusement des CI₅₀ inférieures à 1µM, plus avantageusement inférieures ou égales à 100nM et encore plus avantageusement inférieures ou égales à 10nM.

A titre d'exemple :
- le composé selon l'exemple 42 présente une CI₅₀ de 360 nM ;
- le composé selon l'exemple 2 présente une CI₅₀ de 72 nM ;
- le composé selon l'exemple 4 présente une CI₅₀ de 3 nM ;

Les composés selon l'invention peuvent être utilisés pour la préparation de médicaments, en particulier de médicaments antagonistes du récepteur MCH₁ de la MCH.

## Revendications

1. Composé répondant à la formule générale (I) 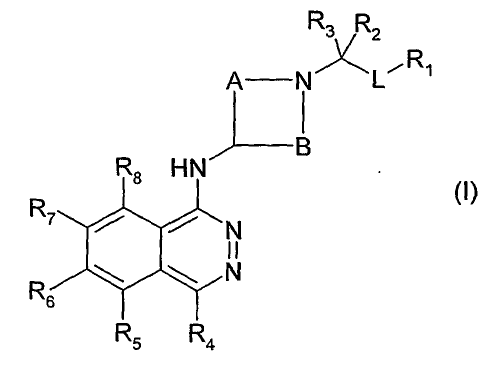
• A représente un groupe C₁₋₄-alkylène éventuellement substitué par un ou plusieurs groupes R₉ identiques ou différents l'un de l'autre ;
• B représente un groupe C₁₋₄-alkylène éventuellement substitué par un ou plusieurs groupes R₁₀ identiques ou différents l'un de l'autre ;
• R₉ et R₁₀ représentent chacun, indépendamment l'un de l'autre, un atome d'hydrogène ou un groupe C₁₋₅-alkyle, ou bien R₉ et R₁₀ forment ensemble une liaison simple ou un groupe C₁₋₄-alkylène;
• L représente une liaison simple ou un groupe C₁₋₂-alkylène, -CH=CH- ou -C≡C- ; les groupes C₁₋₂-alkylène et -CH=CH- étant éventuellement substitués par un ou plusieurs substituants C₁₋₂-alkyle ; ou bien L représente un groupe cycloprop-1,2-diyle ;
• R₁ représente un aryle ou un hétéroaryle ; les groupes aryle et hétéroaryle étant éventuellement substitués par un ou plusieurs radicaux Z identiques ou différents l'un de l'autre ;
• R₂ et R₃ représentent indépendamment l'un de l'autre, un atome d'hydrogène ou un groupe C₁₋₃-alkyle ou C₁₋₃-fluoroalkyle,
. ou bien R₂ et R₃ forment ensemble, avec l'atome de carbone qui les porte, un groupe cycloprop-1,1-diyle ;
• R₄ représente un atome d'hydrogène ou un groupe C₁₋₅-alkyle, C₁₋₃-fluoroalkyle, C₃₋₆-cycloalkyle, C₃₋₆-cycloalkyle-C₁₋₃-alkylène, C₁₋₃-alkyle-O-C₁₋₃-alkylène, HO-C₁₋₃-alkylène, C₁₋₃-alkyle-X-C₁₋₃-alkylène où X représente S, SO ou SO₂,
. ou bien R₄ représente un groupe RₐR_{b}N-C₁₋₃-alkylène, aryle, aryle-C₁₋₃-alkylène, aryle-O-, aryle-O-C₁₋₃-alkylène, aryle-C₁₋₃-alkylène-O-C₁₋₃-alkylène, hétéroaryle ou hétéroaryle-C₁₋₃-alkylène ; les groupes aryle, aryle-C₁₋₃-alkylène, aryle-O-, aryle-O-C₁₋₃-alkylène, hétéroaryle et hétéroaryle-C₁₋₃-alkylène étant éventuellement substitués par un ou plusieurs radicaux Z identiques ou différents l'un de l'autre ;
• R₅ représente un atome d'hydrogène ou d'halogène ou un groupe C₁₋₅-alkyle, C₁₋₃-fluoroalkyle, C₁₋₅-alcoxy, C₁₋₃-fluoroalcoxy, HO-C₁₋₃-alkylène, -CN, C₁₋₃-alkyle-X- où X représente S, SO ou SO₂,
. ou bien R₅ représente un groupe RₐR_{b}N-, RₐR_{b}N-C₁-₃-alkylène, aryle, aryle-C₁₋₃-alkylène, aryle-O- ou hétéroaryle ; les groupes aryle, aryle-C₁₋₃-alkylène, aryle-O- et hétéroaryle étant éventuellement substitués par un ou plusieurs radicaux Z identiques ou différents l'un de l'autre ;
• R₆ représente un atome d'hydrogène ou d'halogène ou un groupe C₁₋₅-alkyle, C₁₋₃-fluoroalkyle, C₁₋₅-alcoxy, C₁₋₃-fluoroalcoxy, -CN, RₐR_{b}N-, RₐR_{b}N-C₁₋₃-alkylène, aryle ou hétéroaryle ; les groupes aryle et hétéroaryle étant éventuellement substitués par un ou plusieurs radicaux Z identiques ou différents l'un de l'autre ;
• R₇ représente un atome d'hydrogène ou d'halogène ou un groupe C₁₋₅-alkyle, C₁₋₃-fluoroalkyle, C₁₋₅-alcoxy, C₁₋₃-fluoroalcoxy, HO-C₁₋₃-alkylène, -CN, C₁₋₃-alkyle-X- où X représente S, SO ou SO₂; ou bien R₇ représente un groupe RₐR_{b}N-, RₐR_{b}N-C₁₋₃-alkylène, RₐR_{b}NC(O)-, C₁₋₃-alkyle-C(O)-, aryle, aryle-O- ou hétéroaryle ; les groupes aryle, aryle-O-et hétéroaryle étant éventuellement substitués par un ou plusieurs radicaux Z identiques ou différents l'un de l'autre ;
R₈ représente un atome d'hydrogène ou d'halogène ou un groupe C₁₋₅-alkyle, C₁₋₅-alcoxy, C₁₋₃-fluoroalcoxy ;
• Z représente un atome d'hydrogène ou d'halogène ou un groupe C₁₋₅-alkyle, C₁₋₃-fluoroalkyle, C₃₋₆-cycloalkyle, C₃₋₆-cycloalkyle-C₁₋₃-alkylène, un phényle, C₁₋₅-alcoxy, C₁₋₃-fluoroalcoxy, C₁₋₃-alkyle-O-C₁₋₃-alkylène, HO-C₁₋₃-alkylène, NO₂ -CN, C₁₋₃-alkyle-X-, C₁₋₃-alkyle-X-C₁₋₃-alkylène où X représente S, SO ou SO₂
. ou bien Z représente un groupe RₐR_{b}N-, RₐR_{b}N-C₁₋₃-alkylène, RₐR_{b}NC(O)-, C₁₋₃-alkyle-C(O)-, C₁₋₄-alkyle-CO₂-, C₃₋₆-cycloalkyle-C(O)-,
. ou bien Z représente un radical oxo,
. ou bien deux radicaux Z adjacents forment ensemble un groupe C₁₋₃-alkylènedioxy ;
• Rₐ et R_{b} représentent chacun, indépendamment de l'autre, un atome d'hydrogène ou un groupe C₁₋₃-alkyle ou C₁₋₃-alkyle-C(O)- ; ou bien Rₐ et R_{b} forment ensemble, avec l'atome d'azote qui les porte, un hétérocycle éventuellement substitué par un ou plusieurs groupes C₁₋₃-alkyle ou oxo ;
étant entendu que lorsque R₂, R₃, R₅, R₆, R₇ et R₈ représentent tous un atome d'hydrogène, A et B représentent tous les deux un groupe éthylènyle (-CH₂CH₂-) et L est une liaison simple, R₁ et R₄ ne peuvent pas représenter tous les deux un groupe phényle non substitué ;
à l'état de base ou de sel d'addition à un acide, ainsi qu'à l'état d'hydrate ou de solvat, ainsi que ses énantiomères, diastéréoisomères et leurs mélanges.

2. Composé de formule (I) selon la revendication 1, **caractérisé en ce que** :
• A représente un groupe C₁₋₄-alkylène éventuellement substitué par un ou plusieurs groupes R₉ identiques ou différents l'un de l'autre ;
• B représente un groupe C₁₋₄-alkylène éventuellement substitué par un ou plusieurs groupes R₁₀ identiques ou différents l'un de l'autre ;
• R₉ et R₁₀ représentent chacun un atome d'hydrogène, ou bien R₉ et R₁₀ forment ensemble un groupe C₁₋₄-alkylène ;
• L représente une liaison simple ou -CH=CH- ;
• R₁ représente un aryle ou un hétéroaryle ; les groupes aryle et hétéroaryle étant éventuellement substitués par un ou plusieurs radicaux Z identiques ou différents l'un de l'autre ;
• R₂ et R₃ représentent indépendamment l'un de l'autre un atome d'hydrogène ou un groupe C₁₋₃-alkyle ;
• R₄ représente un atome d'hydrogène ou un groupe C₁₋₅-alkyle, C₃₋₆-cycloalkyle, C₃₋₆-cycloalkyle-C₁₋₃-alkylène, C₁₋₃-alkyle-O-C₁₋₃-alkylène, aryle, aryle-C₁₋₃-alkylène, aryle-C₁₋₃-alkylène-O-C₁₋₃-alkylène, hétéroaryle ou hétéroaryle-C₁₋₃-alkylène ; les groupes aryle, aryle-C₁₋₃-alkylène, hétéroaryle et hétéroaryle-C₁₋₃-alkylène étant éventuellement substitués par un ou plusieurs radicaux Z identiques ou différents l'un de l'autre ;
• R₅ représente un atome d'hydrogène ou un groupe C₁₋₅-alkyle, C₁₋₅-alcoxy ou aryle ; le groupe aryle étant éventuellement substitué par un ou plusieurs radicaux Z identiques ou différents l'un de l'autre ;
• R₆ représente un atome d'hydrogène ;
• R₇ représente un atome d'halogène ou un groupe C₁₋₅-alkyle, C₁₋₅-alcoxy ou un aryle ;
• R₈ représente un atome d'hydrogène ;
• Z représente un atome d'hydrogène ou d'halogène ou un groupe C₁₋₅-alkyle, C₁₋₃-fluoroalkyle, C₃₋₆-cycloalkyle, C₃₋₆-cycloalkyle-C₁₋₃-alkylène, un phényle, C₁₋₅-alcoxy, C₁₋₃-fluoroalcoxy, C₁₋₃-alkyle-O-C₁₋₃-alkylène, HO-C₁₋₃-alkylène, NO₂, -CN, C₁₋₃-alkyle-X-, C₁₋₃-alkyle-X-C₁₋₃-alkylène où X représente S, SO ou SO₂,
. ou bien Z représente un groupe RₐR_{b}N-, RₐR_{b}N-C₁₋₃-alkylène, RₐR_{b}NC(O)-, C₁₋₃-alkyle-C(O)-, C₃₋₆-cycloalkyle-C(O)-,
. ou bien Z représente un radical oxo,
. ou bien deux radicaux Z adjacents forment ensemble un groupe C₁₋₃-alkylènedioxy ;
• Rₐ et R_{b} représentent chacun, indépendamment de l'autre, un atome d'hydrogène ou un groupe C₁₋₃-alkyle ou C₁₋₃-alkyle-C(O)-,
ou bien Rₐ et R_{b} forment ensemble, avec l'atome d'azote qui les porte, un hétérocycle éventuellement substitué par un ou plusieurs groupes C₁₋₃-alkyle ou oxo ; à l'état de base ou de sel d'addition à un acide, ainsi qu'à l'état d'hydrate ou de solvat, ainsi que ses énantiomères, diastéréoisomères et leurs mélanges.

3. Composé de formule (I) selon l'une quelconque des revendications 1 ou 2, **caractérisé en ce que** :
• A représente un groupe C₁₋₄-alkylène éventuellement substitué par un groupe R₉ ;
• B représente un groupe C₁₋₄-alkylène éventuellement substitué par un groupe R₁₀ ;
• R₉ et R₁₀ représentent chacun un atome d'hydrogène ou R₉ et R₁₀ forment ensemble un groupe C₁₋₄-alkylène ;
• L représente une liaison simple ou -CH=CH- ;
• R₁ représente un aryle éventuellement substitué par un ou plusieurs radicaux Z identiques ou différents l'un de l'autre ;
• R₂ et R₃ représentent indépendamment l'un de l'autre, un atome d'hydrogène ou un groupe C₁₋₃-alkyle ;
• R₄ représente un atome d'hydrogène ou un groupe C₁₋₅-alkyle, C₃₋₆-cycloalkyle, C₃₋₆-cycloalkyle-C₁₋₃-alkylène, C₁₋₃-alkyle-O-C₁₋₃-alkylène, aryle ou hétéroaryle ; les groupes aryle et hétéroaryle étant éventuellement substitués par un ou plusieurs radicaux Z identiques ou différents l'un de l'autre ;
• R₅ représente un atome d'hydrogène ;
• R₆ représente un atome d'hydrogène ;
• R₇ représente un atome d'halogène, un méthyle, ou un méthoxy ;
• R₈ représente un atome d'hydrogène ;
• Z représente un atome d'hydrogène, un atome d'halogène, un groupe C₁₋₅-alkyle, C₁₋₅-alcoxy, C₁₋₃-alkyle-O-C₁₋₃-alkylène, un phényle, HO-C₁₋₃-alkylène, -CN, C₁₋₃-alkyle-X-où X représente un atome de soufre,
. ou bien Z représente un groupe RₐR_{b}N-, RₐR_{b}N-C₁₋₃-alkylène, RₐR_{b}NC(O)-, C₁₋₃-alkyle-C(O)-,
ou bien Z représente un radical oxo,
. ou bien deux radicaux Z adjacents forment ensemble un groupe C₁₋₃-alkylènedioxy ;
• Rₐ et R_{b} représentent chacun, indépendamment de l'autre, un atome d'hydrogène ou un groupe C₁₋₃-alkyle ou C₁₋₃-alkyle-C(O)- ; à l'état de base ou de sel d'addition à un acide, ainsi qu'à l'état d'hydrate ou de solvat, ainsi que ses énantiomères, diastéréoisomères et leurs mélanges.

4. Composés de formule (1) selon la revendication 1 choisis parmi :
• 7-méthoxy-4-(4-méthoxyphényl)-N-[8-(2-naphthylméthyl)-8-azabicyclo[3.2.1]oct-3-yl]phthalazin-1-amine ;
• 7-méthoxy-4-(4-méthoxyphényl)-N-[1-(2-naphthylméthyl)pipéridin-4-yl]phthalazin-1-amine ;
• 7-méthoxy-4-(3-méthoxyphényl)-N-[1-(2-naphthylméthyl)pipéridin-4-yl]phthalazin-1-amine ;
• 7-méthoxy-4-(2-méthoxyphényl)-N-[1-(2-naphthylméthyl)pipéridin-4-yl]phthalazin-1-amine ;
• 7-méthoxy-N-[1-(2-naphthylméthyl)pipéridin-4-yl]-4-phénylphthalazin-1-amine ;
• 4-(4-méthoxyphényl)-7-méthyl-N-[1-(2-naphthylméthyl)pipéridin-4-yl]phthalazin-1-amine;
• 4-(3,4-diméthoxyphényl)-7-méthoxy-N-[1-(2-naphthylméthyl)pipéridin-4-yl]phthalazin-1-amine ;
• Dichlorhydrate de N-[1-(1,3-benzodioxol-5-ylméthyl)pipéridin-4-yl]-7-méthoxy-4-(4-méthoxyphényl)phthalazin-1-amine ;
• 4-éthyl-7-méthoxy-N-[1-(2-naphthylméthyl)pipéridin-4-yl]phthalazin-1-amine ;
• 4-benzyl-7-méthoxy-N-[1-(2-naphthylméthyl)pipéridin-4-yl]phthalazin-1-amine ;
• Chlorhydrate de 7-méthoxy-4-(méthoxyméthyl)-N-[1-(2-naphthylméthyl)pipéridin-4-yl]phthalazin-1-amine ;
• N-[1-(1-benzofuran-5-ylméthyl)pipéridin-4-yl]-7-méthoxy-4-(4-méthoxyphényl)phthalazin-1-amine ;
• N-[1-(3,4-diméthylbenzyl)pipéridin-4-yl]-7-méthoxy-4-(4-méthoxyphényl)phthalazin-1-amine ;
• 7-méthoxy-4-(4-méthoxyphényl)-N-{1-[(1-méthyl-1H-indol-2-yl)méthyl]pipéridin-4-yl}phthalazin-1-amine ;
• 4-cyclopropyl-7-méthoxy-N-[1-(2-naphthylméthyl)pipéridin-4-yl]phthalazin-1-amine ;
• N-[1-(3-fluoro-4-méthoxybenzyl)pipéridin-4-yl]-7-méthoxy-4-(4-méthoxyphényl)phthalazin-1-amine ;
• N-[1-(2,3-dihydro-1,4-benzodioxin-6-ylméthyl)pipéridin-4-yl]-7-méthoxy-4-(4-méthoxyphényl)phthalazin-1-amine ;
• Dichlorhydrate de 4-(1,3-benzodioxol-5-yl)-7-méthoxy-N-[1-(2-naphthylméthyl)pipéridin-4-yl]phthalazin-1-amine ;
• 4-(4-chlorophényl)-7-méthoxy-N-[1-(2-naphthylméthyl)pipéridin-4-yl]phthalazin-1-amine ;
• 7-méthoxy-N-[1-(2-naphthylméthyl)pipéridin-4-yl]-4-[4-(trifluorométhyl)phényl] phthalazin-1-amine ;
• N-[1-(1-benzothien-2-ylméthyl)pipéridin-4-yl]-7-méthoxy-4-(4-méthoxyphényl) phthalazin-1-amine ;
• Dichlorhydrate de 4-cyclopentyl-7-méthoxy-N-[1-(2-naphthylméthyl)pipéridin-4-yl] phthalazin-1-amine ;
• N-[1-(2,3-dihydro-1-benzofuran-5-ylméthyl)pipéridin-4-yl]-7-méthoxy-4-(4-méthoxyphényl)phthalazin-1-amine ;
• N-[1-(2,3-dihydro-1H-indèn-5-ylméthyl)pipéridin-4-yl]-7-méthoxy-4-(4-méthoxyphényl)phthalazin-1-amine ;
• Dichlorhydrate de 7-méthoxy-4-(4-méthoxyphényl)-N-{1-[1-(2-naphthyl)éthyl]pipéridin-4-yl}phthalazin-1-amine;
• N-[1-(1,3-benzothiazol-6-ylméthyl)pipéridin-4-yl]-7-méthoxy-4-(4-méthoxyphényl)phthalazin-1-amine ;
• N-[1-(1-benzothièn-5-ylméthyl)pipéridin-4-yl]-7-méthoxy-4-(4-méthoxyphényl) phthalazin-1-amine ;
• N-[1-(1H-indol-3-ylméthyl)pipéridin-4-yl]-7-méthoxy-4-(4-méthoxyphényl) phthalazin-1-amine ;
• 7-méthoxy-4-(4-méthylphényl)-N-[1-(2-naphthylméthyl)pipéridin-4-yl] phthalazin-1-amine ;
• 4-butyl-7-méthoxy-N-[1-(2-naphthylméthyl)pipéridin-4-yl]phthalazin-1-amine ;
• Dichlorhydrate de 7-méthoxy-4-(4-méthoxyphényl)-N-{1-[(2E)-3-phénylprop-2-èn-1-yl]pipéridin-4-yl}phthalazin-1-amine;
• 7-méthoxy-N-[1-(2-naphthylméthyl)pipéridin-4-yl]phthalazin-1-amine ;
• 4-(cyclopropylméthyl)-7-méthoxy-N-[1-(2-naphthylméthyl)pipéridin-4-yl]phthalazin-1-amine ;
• N-[1-(3-fluoro-4-méthylbenzyl)pipéridin-4-yl]-7-méthoxy-4-(4-méthoxyphényl) phthalazin-1-amine ;
• 7-méthoxy-4-(4-méthoxyphényl)-N-[1-(4-méthylbenzyl)pipéridin-4-yl] phthalazin-1-amine ;
• 4-(4-fluorophényl)-7-méthoxy-N-[1-(2-naphthylméthyl)pipéridin-4-yl]phthalazin-1-amine (composé n° 82) ;
• N-[1-(1H-indol-5-ylméthyl)pipéridin-4-yl]-7-méthoxy-4-(4-méthoxyphényl)phthalazin-1-amine ;
• 7-méthoxy-4-[4-(méthoxyméthyl)phényl]-N-[1-(2-naphthylméthyl)pipéridin-4-yl]phthalazin-1-amine ;
• 7-méthoxy-N-[1-(4-méthoxy-3-méthylbenzyl)pipéridin-4-yl]-4-(4-méthoxyphényl)phthalazin-1-amine ;
• 7 -méthoxy-4-méthyl-N-[1-(2-naphthylméthyl)pipéridin-4-yl]phthalazin-1-amine ;
• Dichlorhydrate de 4-(2,5-diméthoxyphényl)-7-méthoxy-N-[1-(2-naphthylméthyl)pipéridin-4-yl]phthalazin-1-amine ;
• 7-méthoxy-4-(4-méthoxyphényl)-N-[1-(quinolin-6-ylméthyl)pipéridin-4-yl]phthalazin-1-amine ;
• 7-méthoxy-4-(4-méthoxyphényl)-N-{1-[(1-méthyl-1H-indol-5-yl)méthyl]pipéridin-4-yl}phthalazin-1-amine ;
• Dichlorhydrate de 4-(2,4-diméthoxyphényl)-7-méthoxy-N-[1-(2-naphthylméthyl) pipéridin-4-yl]phthalazin-1-amine ;
• Dichlorhydrate de 4-(3,5-diméthoxyphényl)-7-méthoxy-N-[1-(2-naphthylméthyl)pipéridin-4-yl]phthalazin-1-amine ;
• Dichlorhydrate de 7-méthoxy-N-[1-(2-naphthylméthyl)pipéridin-4-yl]-4-(2-phényléthyl)phthalazin-1-amine ;
• Trichlorhydrate de 4-{4-[(diéthylamino)méthyl]phényl}-7-méthoxy-N-[1-(2-naphthylméthyl)pipéridin-4-yl]phthalazin-1-amine ;
• 7-fluoro-4-(4-fluorophényl)-N-[1-(2-naphthylméthyl)pipéridin-4-yl]phthalazin-1-amine ;
• Trichlorhydrate de 7-méthoxy-N-[1-(2-naphthylméthyl)pipéridin-4-yl]-4-pyridin-3-ylphthalazin-1-amine ;
• Dichlorhydrate de N-[1-(4-chlorobenzyl)pipéridin-4-yl]-7-méthoxy-4-(4-méthoxyphényl)phthalazin-1-amine ;
• Dichlorhydrate de {4-[(4-{[7-méthoxy-4-(4-méthoxyphényl)phthalazin-1-yl]amino}pipéridin-1-yl)méthyl]phényl}méthanol ;
• Trichlorhydrate de N-{1-[4-(aminométhyl)benzyl]pipéridin-4-yl}-7-méthoxy-4-(4-méthoxyphényl)phthalazin-1-amine ;
• Dichlorhydrate de N-{4-[(4-{[7-méthoxy-4-(4-méthoxyphényl)phthalazin-1-yl]amino}pipéridin-1-yl)méthyl]phényl}acétamide ;
• Dichlorhydrate de 1-{4-[(4-{[7-méthoxy-4-(4-méthoxyphényl)phthalazin-1-yl]amino}pipéridin-1-yl)méthyl]phényl}éthanone ;
• Dichlorhydrate de N-[1-(1-benzofuran-5-ylméthyl)pipéridin-4-yl]-4-(2,5-diméthoxyphényl)-7-méthoxyphthalazin-1-amine ;
• Dichlorhydrate de N-[1-(1,3-benzodioxol-5-ylméthyl)pipéridin-4-yl]-4-(2,5-diméthoxyphényl)-7-méthoxyphthalazin-1-amine ;
• Dichlorhydrate de 6-[(4-{[7-méthoxy-4-(4-méthoxyphényl)phthalazin-1-yl]amino}pipéridin-1-yl)méthyl]-2H-chromèn-2-one(dichlorhydrate) ;
• N-[1-(1H-indol-6-ylméthyl)pipéridin-4-yl]-7-méthoxy-4-(4-méthoxyphenyl)phthalazin-1-amine ;
• Dichlorhydrate de 7-méthoxy-N-[1-(4-méthoxybenzyl)pipéridin-4-yl]-4-(4-méthoxyphényl)phthalazin-1-amine ;
• Dichlorhydrate de N-[1-(4-éthylbenzyl)pipéridin-4-yl]-7-méthoxy-4-(4-méthoxyphényl)phthalazin-1-amine ;
• N-[1-(1,3-benzothiazol-6-ylméthyl)pipéridin-4-yl]-4-(2,5-diméthoxyphényl)-7-méthoxyphthalazin-1-amine ;
• Dichlorhydrate de 7-méthoxy-N-[1-(2-naphthylméthyl)pipéridin-4-yl]-4-(phénoxyméthyl)phthalazin-1-amine ;
• N-[1-(1H-benzimidazol-5-ylméthyl)pipéridin-4-yl]-7-méthoxy-4-(4-méthoxyphényl)phthalazin-1-amine ;
• Dichlorhydrate de 7-chloro-4-(4-méthoxyphényl)-N-[1-(2-naphthylméthyl)pipéridin-4-yl]phthalazin-1-amine ;
• Dichlorhydrate de 4-(4-bromophényl)-7-méthoxy-N-[1-(2-naphthylméthyl)pipéridin-4-yl]phthalazin-1-amine ;
• Dichlorhydrate de N-[1-(1,3-benzodioxol-5-ylmethyl)pipéridin-4-yl]-7-méthoxy-4-(méthoxyméthyl)phthalazin-1-amine ;
• Dichlorhydrate de 4-[(benzyloxy)méthyl]-7-méthoxy-N-[1-(2-naphthylméthyl)pipéridin-4-yl]phthalazin-1-amine ;
• Dichlorhydrate de 7-méthoxy-5-phényl-N-[1-(2-naphthylméthyl)pipéridin-4-yl]phthalazin-1-amine;
• 4-(hydroxyméthyl)-7-méthoxy-N-[1-(2-naphthylméthyl)pipéridin-4-yl]-4-(phénoxyméthyl)phthalazin-1-amine ;
• (-)7-méthoxy-4-(4-méthoxyphényl)-N-{1-[1-(2-naphthyl)éthyl]pipéridin-4-yl}phthalazin-1-amine ;
• (+)7-méthoxy-4-(4-méthoxyphényl)-N-{1-[1-(2-naphthyl)éthyl]pipéridin-4-yl}phthalazin-1-amine ;
• Trichlorhydrate de N-{1-[4-(diméthylaminométhyl)benzyl]pipéridin-4-yl}-7-méthoxy-4-(4-méthoxyphényl)phthalazin-1-amine.

5. Procédé de préparation d'un composé de formule (I) selon l'une quelconque des revendications 1 à 4, **caractérisé en ce que** l'on fait réagir un composé de formule générale (III) 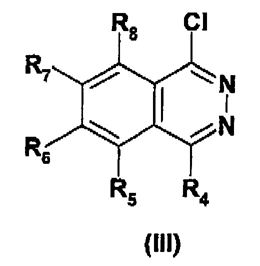 dans laquelle R₄, R₅, R₆, R₇ et R₈ sont tels que définis dans la formule générale (I) selon la revendication 1,
avec un composé de formule générale (II) 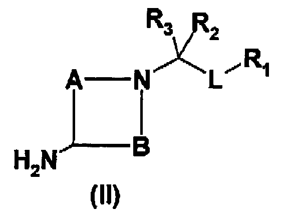 dans laquelle R₁, R₂, R₃, L, A et B sont tels que définis dans la formule générale (I) selon la revendication 1.

6. Médicament, **caractérisé en ce qu'**il comprend un composé de formule (I) selon l'une quelconque des revendications 1 à 4, ou un sel d'addition de ce composé à un acide pharmaceutiquement acceptable, ou encore un hydrate ou un solvat du composé de formule (I).

7. Composition pharmaceutique, **caractérisée en ce qu'**elle comprend au moins un composé de formule (I) selon l'une quelconque des revendications 1 à 4, ou un sel pharmaceutiquement acceptable, un hydrate ou un solvat de ce composé, ainsi qu'au moins un excipient pharmaceutiquement acceptable.

8. Utilisation d'un composé de formule (I) selon l'une quelconque des revendications 1 à 4, pour la préparation d'un médicament destiné au traitement de toutes maladies impliquant un dysfonctionnement lié au récepteur MCH₁.

9. Utilisation d'un composé de formule (I) selon l'une quelconque des revendications 1 à 4, pour la préparation d'un médicament destiné au traitement de l'obésité, de la cellulite, de troubles métaboliques et de leurs pathologies associées telles que le diabète, les troubles cardiovasculaires, le syndrome X, dans le traitement de pathologies liées au stress telles que l'anxiété et la dépression.

## Claims

1. Compound corresponding to general formula (I) 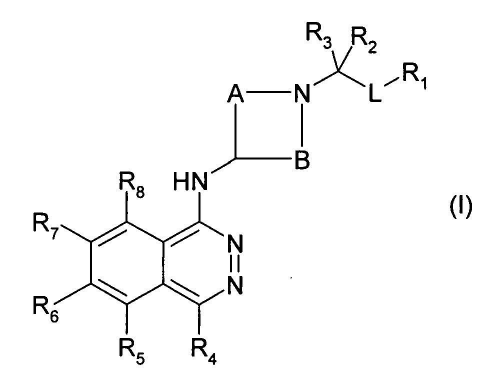
• A represents a C₁₋₄-alkylene group optionally substituted with one or more groups R₉, which may be identical or different;
• B represents a C₁₋₄-alkylene group optionally substituted with one or more groups R₁₀, which may be identical or different;
• R₉ and R₁₀ each represent, independently of each other, a hydrogen atom or a C₁₋₅-alkyl group, or alternatively R₉ and R₁₀ together form a single bond or a C₁₋₄-alkylene group;
• L represents a single bond or a C₁₋₂-alkylene, -CH=CH- or -C≡C- group; the C₁₋₂-alkylene and -CH=CH- groups being optionally substituted with one or more C₁₋₂-alkyl substituents; or alternatively L represents a cycloprop-1,2-diyl group;
• R₁ represents an aryl or a heteroaryl; the aryl and heteroaryl groups being optionally substituted with one or more radicals Z, which may be identical or different;
• R₂ and R₃ represent, independently of each other, a hydrogen atom or a C₁₋₃-alkyl or C₁₋₃-fluoroalkyl group,
. or alternatively R₂ or R₃ form, together with the carbon atom that bears them, a cycloprop-1,1-diyl group;
• R₄ represents a hydrogen atom or a C₁₋₅-alkyl, C₁₋₃-fluoroalkyl, C₃₋₆-cycloalkyl, C₃₋₆-cycloalkyl-C₁₋₃-alkylene, C₁₋₃-alkyl-O-C₁₋₃-alkylene, HO-C₁₋₃-alkylene or C₁₋₃-alkyl-X-C₁₋₃-alkylene group in which X represents S, SO or SO₂,
. or alternatively R₄ represents an RₐR_{b}N-C₁₋₃-alkylene, aryl, aryl-C₁₋₃-alkylene, aryl-O-, aryl-O-C₁₋₃-alkylene, aryl-C₁₋₃-alkylene-O-C₁₋₃-alkylene, heteroaryl or heteroaryl-C₁₋₃-alkylene group; the aryl, aryl-C₁₋₃-alkylene, aryl-O-, aryl-O-C₁₋₃-alkylene, heteroaryl and heteroaryl-C₁₋₃-alkylene groups being optionally substituted with one or more radicals Z, which may be identical or different;
• R₅ represents a hydrogen or halogen atom or a C₁₋₅-alkyl, C₁₋₃-fluoroalkyl, C₁₋₅-alkoxy, C₁₋₃-fluoroalkoxy, HO-C₁₋₃-alkylene, -CN or C₁₋₃-alkyl-X- group in which X represents S, SO or SO₂,
. or alternatively R₅ represents an RₐR_{b}N-, RₐR_{b}N-C₁₋₃-alkylene, aryl, aryl-C₁₋₃-alkylene, aryl-0- or heteroaryl group; the aryl, aryl-C₁₋₃-alkylene, aryl-O- and heteroaryl groups being optionally substituted with one or more radicals Z, which may be identical or different;
• R₆ represents a hydrogen or halogen atom or a C₁₋₅-alkyl, C₁₋₃-fluoroalkyl, C₁₋₅-alkoxy, C₁₋₃-fluoroalkoxy, -CN, RₐR_{b}N-, RₐR_{b}N-C₁₋₃-alkylene, aryl or heteroaryl group; the aryl and heteroaryl groups being optionally substituted with one or more radicals Z, which may be identical or different;
• R₇ represents a hydrogen or halogen atom or a C₁₋₅-alkyl, C₁₋₃-fluoroalkyl, C₁₋₅-alkoxy, C₁₋₃-fluoroalkoxy, HO-C₁₋₃-alkylene, -CN or C₁₋₃-alkyl-X- group in which X represents S, SO or SO₂; or alternatively R₇ represents an RₐR_{b}N-, RₐR_{b}N-C₁₋₃-alkylene, RₐR_{b}NC(O)-, C₁₋₃-alkyl-C(O)-, aryl, aryl-O- or heteroaryl group; the aryl, aryl-0- and heteroaryl groups being optionally substituted with one or more radicals Z, which may be identical or different;
• R₈ represents a hydrogen or halogen atom or a C₁₋₅-alkyl, C₁₋₅-alkoxy or C₁₋₃-fluoroalkoxy group;
• Z represents a hydrogen or halogen atom or a C₁₋₅-alkyl, C₁₋₃-fluoroalkyl, C₃₋₆-cycloalkyl, C₃₋₆-cycloalkyl-C₁₋₃-alkylene, phenyl, C₁₋₅-alkoxy, C₁₋₃-fluoroalkoxy, C₁₋₃-alkyl-O-C₁₋₃-alkylene, HO-C₁₋₃-alkylene, NO₂, -CN, C₁₋₃-alkyl-X- or C₁₋₃-alkyl-X-C₁₋₃-alkylene group in which X represents S, SO or SO₂,
. or alternatively Z represents an RₐR_{b}N-, RₐR_{b}N-C₁₃-alkylene, RₐR_{b}NC(O)-, C₁₋₃-alkyl-C(O)-, C₁₋₄-alkyl-CO₂-, or C₃₋₆-cycloalkyl-C(O)- group,
. or alternatively Z represents an oxo radical,
. or alternatively two adjacent radicals Z together form a C₁₋₃-alkylenedioxy group;
• Rₐ and R_{b} each represent, independently of each other, a hydrogen atom or a C₁₋₃-alkyl or C₁₋₃-alkyl-C(O)- group; or alternatively Rₐ and R_{b} form, together with the nitrogen atom that bears them, a heterocycle optionally substituted with one or more C₁₋₃-alkyl or oxo groups;
it being understood that when R₂, R₃, R₅, R₆, R₇ and R₈ all represent a hydrogen atom, A and B both represent an ethylenyl group (-CH₂CH₂-) and L is a single bond, R₁ and R₄ cannot both represent an unsubstituted phenyl group;
in the form of base or of acid-addition salt, and also in the form of hydrate or solvate,
and also the enantiomers and diastereoisomers thereof, and mixtures thereof.

2. Compound of formula (I) according to Claim 1, **characterized in that**:
• A represents a C₁₋₄-alkylene group optionally substituted with one or more groups R₉, which may be identical or different;
• B represents a C₁₋₄-alkylene group optionally substituted with one or more groups R₁₀, which may be identical or different;
• R₉ and R₁₀ each represent a hydrogen atom, or alternatively R₉ and R₁₀ together form a C₁₋₄-alkylene group;
• L represents a single bond or -CH=CH-;
• R₁ represents an aryl or a heteroaryl; the aryl and heteroaryl groups being optionally substituted with one or more radicals Z, which may be identical or different;
• R₂ and R₃ represent, independently of each other, a hydrogen atom or a C₁₋₃-alkyl group;
• R₄ represents a hydrogen atom or a C₁₋₅-alkyl, C₃₋₆-cycloalkyl, C₃₋₆-cycloalkyl-C₁₋₃-alkylene, C₁₋₃-alkyl-O-C₁₋₃-alkylene, aryl, aryl-C₁₋₃-alkylene, aryl-C₁₋₃-alkylene-O-C₁₋₃-alkylene, heteroaryl or heteroaryl-C₁₋₃-alkylene group; the aryl, aryl-C₁₋₃-alkylene, heteroaryl and heteroaryl-C₁₋₃-alkylene groups being optionally substituted with one or more radicals Z, which may be identical or different;
• R₅ represents a hydrogen atom or a C₁₋₅-alkyl, C₁₋₅-alkoxy or aryl group; the aryl group being optionally substituted with one or more radicals Z, which may be identical or different;
• R₆ represents a hydrogen atom;
• R₇ represents a halogen atom or a C₁₋₅-alkyl group, C₁₋₅-alkoxy or an aryl;
• R₈ represents a hydrogen atom;
• Z represents a hydrogen or halogen atom or a C₁₋₅-alkyl, C₁₋₃-fluoroalkyl, C₃₋₆-cycloalkyl, C₃₋₆-cycloalkyl-C₁₋₃-alkylene, phenyl, C₁₋₅-alkoxy, C₁₋₃-fluoroalkoxy, C₁₋₃-alkyl-O-C₁₋₃-alkylene, HO-C₁₋₃-alkylene, NO₂, -CN, C₁₋₃-alkyl-X- or C₁₋₃-alkyl-X-C₁₋₃-alkylene group in which X represents S, SO or SO₂,
. or alternatively Z represents an RₐR_{b}N-, RₐR_{b}N-C₁₋₃-alkylene, RₐR_{b}NC(O)-, C₁₋₃-alkyl-C(O)- or C₃₋₆-cycloalkyl-C(O)- group,
. or alternatively Z represents an oxo radical,
. or alternatively two adjacent radicals Z together form a C₁₋₃-alkylenedioxy group;
• Rₐ and R_{b} each represent, independently of each other, a hydrogen atom or a C₁₋₃-alkyl or C₁₋₃-alkyl-C(O)-group,
. or alternatively Rₐ and R_{b} form, together with the nitrogen atom that bears them, a heterocycle optionally substituted with one or more C₁₋₃-alkyl or oxo groups;
in the form of base or of acid-addition salt, and also in the form of hydrate or solvate, and also the enantiomers and diastereoisomers thereof, and mixtures thereof.

3. Compound of formula (I) according to either of Claims 1 and 2, **characterized in that**:
• A represents a C₁₋₄-alkylene group optionally substituted with a group R₉;
• B represents a C₁₋₄-alkylene group optionally substituted with a group R₁₀;
• R₉ and R₁₀ each represent a hydrogen atom or R₉ and R₁₀ together form a C₁₋₄-alkylene group;
• L represents a single bond or -CH=CH-;
• R₁ represents an aryl optionally substituted with one or more radicals Z, which may be identical or different;
• R₂ and R₃ represent, independently of each other, a hydrogen atom or a C₁₋₃-alkyl group;
• R₄ represents a hydrogen atom or a C₁₋₅-alkyl, C₃₋₆-cycloalkyl, C₃₋₆-cycloalkyl-C₁₋₃-alkylene, C₁₋₃-alkyl-O-C₁₋₃-alkylene, aryl or heteroaryl group; the aryl and heteroaryl groups being optionally substituted with one or more radicals Z, which may be identical or different;
• R₅ represents a hydrogen atom;
• R₆ represents a hydrogen atom;
• R₇ represents a halogen atom, a methyl or a methoxy;
• R₈ represents a hydrogen atom;
• Z represents a hydrogen atom, a halogen atom or a C₁₋₅-alkyl, C₁₋₅-alkoxy, C₁₋₃-alkyl-O-C₁₋₃-alkylene, phenyl, HO-C₁₋₃-alkylene, -CN or C₁₋₃-alkyl-X- group in which X represents a sulfur atom,
. or alternatively Z represents an RₐR_{b}N-, RₐR_{b}N-C₁₃-alkylene, RₐR_{b}NC(O)- or C₁₋₃-alkyl-C(O)- group,
. or alternatively Z represents an oxo radical,
. or alternatively two adjacent radicals Z together form a C₁₋₃-alkylenedioxy group;
• Rₐ and R_{b} each represent, independently of each other, a hydrogen atom or a C₁₋₃-alkyl or C₁₋₃-alkyl-C(O)- group;
in the form of base or of acid-addition salt, and also in the form of hydrate or solvate, and also the enantiomers and diastereoisomers thereof, and mixtures thereof.

4. Compounds of formula (I) according to Claim 1, chosen from:
• 7-methoxy-4-(4-methoxyphenyl)-N-[8-(2-naphthylmethyl)-8-azabicyclo[3.2.1]oct-3-yl]phthalazin-1-amine;
• 7-methoxy-4-(4-methoxyphenyl)-N-[1-(2-naphthylmethyl)piperidin-4-yl]phthalazin-1-amine;
• 7-methoxy-4-(3-methoxyphenyl)-N-[1-(2-naphthylmethyl)piperidin-4-yl]phthalazin-1-amine;
• 7-methoxy-4-(2-methoxyphenyl)-N-[1-(2-naphthylmethyl)piperidin-4-yl]phthalazin-1-amine;
• 7-methoxy-N-[1-(2-naphthylmethyl)piperidin-4-yl]-4-phenylphthalazin-1-amine;
• 4-(4-methoxyphenyl)-7-methyl-N-[1-(2-naphthylmethyl)piperidin-4-yl]phthalazin-1-amine;
• 4-(3,4-dimethoxyphenyl)-7-methoxy-N-[1-(2-naphthylmethyl)piperidin-4-yl]phthalazin-1-amine;
• N-[1-(1,3-benzodioxol-5-ylmethyl)piperidin-4-yl]-7-methoxy-4-(4-methoxyphenyl)phthalazin-1-amine dihydrochloride;
• 4-ethyl-7-methoxy-N-[1-(2-naphthylmethyl)piperidin-4-yl]phthalazin-1-amine;
• 4-benzyl-7-methoxy-N-[1-(2-naphthylmethyl)piperidin-4-yl]phthalazin-1-amine;
• 7-methoxy-4-(methoxymethyl)-N-[1-(2-naphthylmethyl)-piperidin-4-yl]phthalazin-1-amine hydrochloride;
• N-[1-(1-benzofuran-5-ylmethyl)piperidin-4-yl]-7-methoxy-4-(4-methoxyphenyl)phthalazin-1-amine;
• N-[1-(3,4-dimethylbenzyl)piperidin-4-yl]-7-methoxy-4-(4-methoxyphenyl)phthalazin-1-amine;
• 7-methoxy-4-(4-methoxyphenyl)-N-{1-[(1-methyl-1H-indol-2-yl)methyl]piperidin-4-yl}phthalazin-1-amine;
• 4-cyclopropyl-7-methoxy-N-[1-(2-naphthylmethyl)-piperidin-4-yl]phthalazin-1-amine;
• N-[1-(3-fluoro-4-methoxybenzyl)piperidin-4-yl]-7-methoxy-4-(4-methoxyphenyl)phthalazin-1-amine;
• N-[1-(2,3-dihydro-1,4-benzodioxin-6-ylmethyl)-piperidin-4-yl]-7-methoxy-4-(4-methoxyphenyl)-phthalazin-1-amine;
• 4-(1,3-benzodioxol-5-yl)-7-methoxy-N-[1-(2-naphthylmethyl)piperidin-4-yl]phthalazin-1-amine dihydrochloride;
• 4-(4-chlorophenyl)-7-methoxy-N-[1-(2-naphthylmethyl)-piperidin-4-yl]phthalazin-1-amine;
• 7-methoxy-N-[1-(2-naphthylmethyl)piperidin-4-yl]-4-[4-(trifluoromethyl)phenyl]phthalazin-1-amine;
• N-[1-(1-benzothien-2-ylmethyl)piperidin-4-yl]-7-methoxy-4-(4-methoxyphenyl)phthalazin-1-amine;
• 4-cyclopentyl-7-methoxy-N-[1-(2-naphthylmethyl)-piperidin-4-yl]phthalazin-1-amine dihydrochloride;
• N-[1-(2,3-dihydro-1-benzofuran-5-ylmethyl)piperidin-4-yl]-7-methoxy-4-(4-methoxyphenyl)phthalazin-1-amine;
• N-[1-(2,3-dihydro-1H-inden-5-ylmethyl)piperidin-4-yl]-7-methoxy-4-(4-methoxyphenyl)phthalazin-1-amine;
• 7-methoxy-4-(4-methoxyphenyl)-N-{1-[1-(2-naphthyl)-ethyl]piperidin-4-yl}phthalazin-1-amine dihydrochloride;
• N-[1-(1,3-benzothiazol-6-ylmethyl)piperidin-4-yl]-7-methoxy-4-(4-methoxyphenyl)phthalazin-1-amine;
• N-[1-(1-benzothien-5-ylmethyl)piperidin-4-yl]-7-methoxy-4-(4-methoxyphenyl)phthalazin-1-amine;
• N-[1-(1H-indol-3-ylmethyl)piperidin-4-yl]-7-methoxy-4-(4-methoxyphenyl)phthalazin-1-amine;
• 7-methoxy-4-(4-methylphenyl)-N-[1-(2-naphthylmethyl)piperidin-4-yl]phthalazin-1-amine;
• 4-butyl-7-methoxy-N-[1-(2-naphthylmethyl)piperidin-4-yl]phthalazin-l-amine;
• 7-methoxy-4-(4-methoxyphenyl)-N-{1-[(2E)-3-phenylprop-2-en-l-yl]piperidin-4-yl}phthalazin-1-amine dihydrochloride;
• 7-methoxy-N-[1-(2-naphthylmethyl)piperidin-4-yl]phthalazin-1-amine;
• 4-(cyclopropylmethyl)-7-methoxy-N-[1-(2-naphthylmethyl)piperidin-4-yl]phthalazin-1-amine;
• N-[1-(3-fluoro-4-methylbenzyl)piperidin-4-yl]-7-methoxy-4-(4-methoxyphenyl)phthalazin-1-amine;
• 7-methoxy-4-(4-methoxyphenyl)-N-[1-(4-methylbenzyl)piperidin-4-yl]phthalazin-1-amine;
• 4-(4-fluorophenyl)-7-methoxy-N-[1-(2-naphthylmethyl)piperidin-4-yl]phthalazin-1-amine;
• N-[1-(1H-indol-5-ylmethyl)piperidin-4-yl]-7-methoxy-4-(4-methoxyphenyl)phthalazin-1-amine;
• 7-methoxy-4-[4-(methoxymethyl)phenyl]-N-[1-(2-naphthylmethyl)piperidin-4-yl]phthalazin-1-amine;
• 7-methoxy-N-[1-(4-methoxy-3-methylbenzyl)piperidin-4-yl]-4-(4-methoxyphenyl)phthalazin-1-amine;
• 7-methoxy-4-methyl-N-[1-(2-naphthylmethyl)piperidin-4-yl]phthalazin-1-amine;
• 4-(2,5-dimethoxyphenyl)-7-methoxy-N-[1-(2-naphthylmethyl)piperidin-4-yl]phthalazin-1-amine dihydrochloride;
• 7-methoxy-4-(4-methoxyphenyl)-N-[1-(quinolin-6-ylmethyl)piperidin-4-yl]phthalazin-1-amine;
• 7-methoxy-4-(4-methoxyphenyl)-N-{1-[(1-methyl-1H-indol-5-yl)methyl]piperidin-4-yl}phthalazin-1-amine;
• 4-(2,4-dimethoxyphenyl)-7-methoxy-N-[1-(2-naphthylmethyl)piperidin-4-yl]phthalazin-1-amine dihydrochloride;
• 4-(3,5-dimethoxyphenyl)-7-methoxy-N-[1-(2-naphthylmethyl)piperidin-4-yl]phthalazin-1-amine dihydrochloride;
• 7-methoxy-N-[1-(2-naphthylmethyl)piperidin-4-yl]-4-(2-phenylethyl)phthalazin-1-amine dihydrochloride;
• 4-{4-[(diethylamino)methyl]phenyl}-7-methoxy-N-[1-(2-naphthylmethyl)piperidin-4-yl]phthalazin-1-amine trihydrochloride;
• 7-fluoro-4-(4-fluorophenyl)-N-[1-(2-naphthylmethyl)piperidin-4-yl]phthalazin-1-amine;
• 7-methoxy-N-[1-(2-naphthylmethyl)piperidin-4-yl]-4-pyridin-3-ylphthalazin-1-amine trihydrochloride;
• N-[1-(4-chlorobenzyl)piperidin-4-yl]-7-methoxy-4-(4-methoxyphenyl)phthalazin-1-amine dihydrochloride;
• {4-[(4-{[7-methoxy-4-(4-methoxyphenyl)phthalazin-1-yl]amino}piperidin-1-yl)methyl]phenyl}methanol dihydrochloride;
• N-{1-[4-(aminomethyl)benzyl]piperidin-4-yl}-7-methoxy-4-(4-methoxyphenyl)phthalazin-1-amine trihydrochloride;
• N-{4-[(4-{[7-methoxy-4-(4-methoxyphenyl)phthalazin-1-yl]amino}piperidin-1-yl)methyl]phenyl}acetamide dihydrochloride;
• 1-{4-[(4-{[7-methoxy-4-(4-methoxyphenyl)phthalazin-1-yl]amino}piperidin-1-yl)methyl]phenyl}ethanone dihydrochloride;
• N-[1-(1-benzofuran-5-ylmethyl)piperidin-4-yl]-4-(2,5-dimethoxyphenyl)-7-methoxyphthalazin-1-amine dihydrochloride;
• N-[1-(1,3-benzodioxol-5-ylmethyl)piperidin-4-yl]-4-(2,5-dimethoxyphenyl)-7-methoxyphthalazin-1-amine dihydrochloride;
• 6-[(4-{[7-methoxy-4-(4-methoxyphenyl)phthalazin-1-yl]amino}piperidin-1-yl)methyl]-2H-chromen-2-one dihydrochloride;
• N-[1-(1H-indol-6-ylmethyl)piperidin-4-yl]-7-methoxy-4-(4-methoxyphenyl)phthalazin-1-amine;
• 7-methoxy-N-[1-(4-methoxybenzyl)piperidin-4-yl]-4-(4-methoxyphenyl)phthalazin-1-amine dihydrochloride;
• N-[1-(4-ethylbenzyl)piperidin-4-yl]-7-methoxy-4-(4-methoxyphenyl)phthalazin-1-amine dihydrochloride;
• N-[1-(1,3-benzothiazol-6-ylmethyl)piperidin-4-yl]-4-(2,5-dimethoxyphenyl)-7-methoxyphthalazin-1-amine;
• 7-methoxy-N-[1-(2-naphthylmethyl)piperidin-4-yl]-4-(phenoxymethyl)phthalazin-1-amine dihydrochloride;
• N-[1-(1H-benzimidazol-5-ylmethyl)piperidin-4-yl]-7-methoxy-4-(4-methoxyphenyl)phthalazin-1-amine;
• 7-chloro-4-(4-methoxyphenyl)-N-[1-(2-naphthylmethyl)-piperidin-4-yl]phthalazin-1-amine dihydrochloride;
• 4-(4-bromophenyl)-7-methoxy-N-[1-(2-naphthylmethyl)-piperidin-4-yl]phthalazin-1-amine dihydrochloride;
• N-[1-(1,3-benzodioxol-5-ylmethyl)piperidin-4-yl]-7-methoxy-4-(methoxymethyl)phthalazin-1-amine dihydrochloride;
• 4-[(benzyloxy)methyl]-7-methoxy-N-[1-(2-naphthylmethyl)piperidin-4-yl]phthalazin-1-amine dihydrochloride;
• 7-methoxy-5-phenyl-N-[1-(2-naphthylmethyl)piperidin-4-yl]phthalazin-1-amine dihydrochloride;
• 4-(hydroxymethyl)-7-methoxy-N-[1-(2-naphthylmethyl)-piperidin-4-yl]-4-(phenoxymethyl)phthalazin-1-amine;
• (-)-7-methoxy-4-(4-methoxyphenyl)-N-{1-[1-(2-naphthyl)ethyl]piperidin-4-yl}phthalazin-1-amine;
• (+)-7-methoxy-4-(4-methoxyphenyl)-N-{1-[1-(2-naphthyl)ethyl]piperidin-4-yl}phthalazin-1-amine;
• N-{1-[4-(dimethylaminomethyl)benzyl]piperidin-4-yl}-7-methoxy-4-(4-methoxyphenyl)phthalazin-1-amine.

5. Process for preparing a compound of formula (I) according to any one of Claims 1 to 4, **characterized in that** the compound of general formula (III) 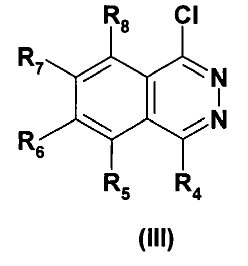 in which R₄, R₅, R₆, R₇ and R₈ are as defined in the general formula (I) according to Claim 1, is reacted with the compound of general formula (II) 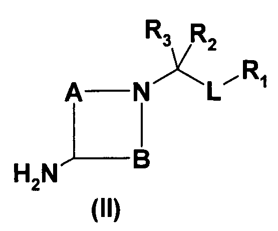 in which R₁, R₂, R₃, L, A and B are as defined in the general formula (I) according to Claim 1.

6. Medicament, **characterized in that** it comprises a compound of formula (I) according to any one of Claims 1 to 4, or a pharmaceutically acceptable acid-addition salt of this compound, or alternatively a hydrate or a solvate of the compound of formula (I).

7. Pharmaceutical composition, **characterized in that** it comprises at least one compound of formula (I) according to any one of Claims 1 to 4, or a pharmaceutically acceptable salt, a hydrate or a solvate of this compound, and also at least one pharmaceutically acceptable excipient.

8. Use of a compound of formula (I) according to any one of Claims 1 to 4, for the preparation of a medicament for treating any disease involving dysfunction associated with the MCH₁ receptor.

9. Use of a compound of formula (I) according to any one of Claims 1 to 4, for the preparation of a medicament for treating obesity, cellulite, metabolic disorders and associated pathologies thereof such as diabetes, cardiovascular disorders and syndrome X, and in the treatment of stress-related pathologies such as anxiety and depression.

## Patentansprüche

1. Verbindung der allgemeinen Formel (I) 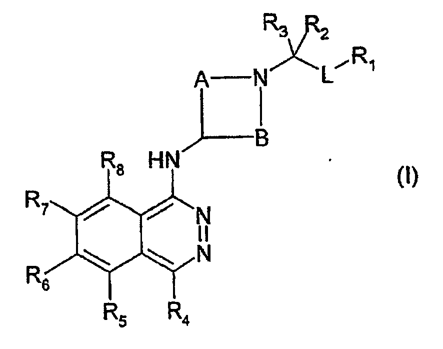 worin:
• A für eine gegebenenfalls durch eine oder mehrere Gruppen R₉, die gleich oder voneinander verschieden sein können, substituierte C₁₋₄-Alkylengruppe steht;
• B für eine gegebenenfalls durch eine oder mehrere Gruppen R₁₀, die gleich oder voneinander verschieden sein können, substituierte C₁₋₄-Alkylengruppe steht;
• R₉ und R₁₀ jeweils unabhängig voneinander für ein Wasserstoffatom oder eine C₁₋₅-Alkylgruppe stehen oder gemeinsam eine Einfachbindung oder eine C₁₋₄-Alkylengruppe bilden;
• L für eine Einfachbindung oder eine C₁₋₂-Alkylen-, -CH=CH- oder -C≡C-Gruppe, die gegebenenfalls durch einen oder mehrere C₁₋₂-Alkylsubstituenten substituiert ist, oder eine Cycloprop-1,2-diylgruppe steht;
• R₁ für Aryl oder Heteroaryl steht, wobei die Aryl- und Heteroarylgruppen gegebenenfalls durch einen oder mehrere Reste Z, die gleich oder voneinander verschieden sein können, substituiert sind;
• R₂ und R₃ unabhängig voneinander für ein Wasserstoffatom oder eine C₁₋₃-Alkyl- oder C₁₋₃-Fluoralkylgruppe stehen
oder gemeinsam mit dem Kohlenstoffatom, an das sie gebunden sind, eine Cycloprop-1,1-diylgruppe bilden;
• R₄ für ein Wasserstoffatom oder eine C₁₋₅-Alkyl-, C₁₋₃-Fluoralkyl-, C₃₋₆-Cycloalkyl-, C₃₋₆-Cycloalkyl-C₁₋₃-alkylen-, C₁₋₃-Alkyl-O-C₁₋₃-alkylen-, HO-C₁₋₃-Alkylen- oder C₁₋₃-Alkyl-X-C₁₋₃-alkylengruppe, worin X für S, SO oder SO₂ steht,
oder für eine RₐR_{b}N-C₁₋₃-Alkylen-, Aryl-, Aryl-C₁₋₃-alkylen-, Aryl-0-, Aryl-O-C₁₋₃-alkylen-, Aryl-C₁₋₃-alkylen-O-C₁₋₃-alkylen-, Heteroaryl- oder Heteroaryl-C₁₋₃-alkylengruppe, wobei die Aryl-, Aryl-C₁₋₃-alkylen-, Aryl-O-, Aryl-O-C₁₋₃-alkylen-, Heteroaryl- und Heteroaryl-C₁₋₃-alkylengruppen gegebenenfalls durch einen oder mehrere Reste Z, die gleich oder voneinander verschieden sein können, substituiert sind, steht;
• R₅ für ein Wasserstoff- oder Halogenatom oder eine C₁₋₅-Alkyl-, C₁₋₃-Fluoralkyl-, C₁₋₅-Alkoxy-, C₁₋₃-Fluoralkoxy-, HO-C₁₋₃-Alkylen-, -CN- oder C₁₋₃-Alkyl-X-Gruppe, worin X für S, SO oder SO₂ steht,
oder für eine RₐR_{b}N-, RₐR_{b}N-C₁₋₃-Alkylen-, Aryl-, Aryl-C₁₋₃-alkylen-, Aryl-O- oder Heteroarylgruppe, wobei die Aryl-, Aryl-C₁₋₃-alkylen-, Aryl-O- und Heteroarylgruppen gegebenenfalls durch einen oder mehrere Reste Z, die gleich oder voneinander verschieden sein können, substituiert sind, steht;
• R₆ für ein Wasserstoff- oder Halogenatom oder eine C₁₋₅-Alkyl-, C₁₋₃-Fluoralkyl-, C₁₋₅-Alkoxy-, C₁₋₃-Fluoralkoxy-, -CN-, RₐR_{b}N-, RₐR_{b}N-C₁₋₃-Alkylen-, Aryl- oder Heteroarylgruppe, wobei die Aryl- und Heteroarylgruppen gegebenenfalls durch einen oder mehrere Reste Z, die gleich oder voneinander verschieden sein können, substituiert sind, steht;
• R₇ für ein Wasserstoff- oder Halogenatom oder eine C₁₋₅-Alkyl-, C₁₋₃-Fluoralkyl-, C₁₋₅-Alkoxy-, C₁₋₃-Fluoralkoxy-, HO-C₁₋₃-Alkylen-, -CN- oder C₁₋₃-Alkyl-X-Gruppe, worin X für S, SO oder SO₂ steht, oder für eine RₐR_{b}N-, RₐR_{b}N-C₁₋₃-Alkylen-, RₐR_{b}NC(O)-, C₁₋₃-Alkyl-C(O)-, Aryl-, Aryl-O- oder Heteroarylgruppe, wobei die Aryl-, Aryl-O- und Heteroarylgruppen gegebenenfalls durch einen oder mehrere Reste Z, die gleich oder voneinander verschieden sein können, substituiert sind, steht;
• R₈ für ein Wasserstoff- oder Halogenatom oder eine C₁₋₅-Alkyl-, C₁₋₅-Alkoxy- oder C₁₋₃-Fluoralkoxygruppe steht;
• Z für ein Wasserstoff- oder Halogenatom oder eine C₁₋₅-Alkyl-, C₁₋₃-Fluoralkyl-, C₃₋₆-Cycloalkyl-, C₃₋₆-Cycloalkyl-C₁₋₃-alkylen-, Phenyl-, C₁₋₅-Alkoxy-, C₁₋₃-Fluoralkoxy-, C₁₋₃-Alkyl-O-C₁₋₃-alkylen-, HO-C₁₋₃-Alkylen-, NO₂-, -CN-, C₁₋₃-Alkyl-X- oder C₁₋₃-Alkyl-X-C₁₋₃-alkylengruppe, worin X für S, SO oder SO₂ steht,
oder eine RₐR_{b}N-, RₐR_{b}N-C₁₋₃-Alkylen-, RₐR_{b}NC(O)-, C₁₋₃-Alkyl-C(O)-, C₁₋₄-Alkyl-CO₂- oder C₃₋₆-Cycloalkyl-C(O)-Gruppe
oder einen Oxorest steht
oder zwei benachbarte Reste Z gemeinsam eine C₁₋₃-Alkylendioxygruppe bilden;
• Rₐ und R_{b} jeweils unabhängig voneinander für ein Wasserstoffatom oder eine C₁₋₃-Alkyl- oder C₁₋₃-Alkyl-C(O)-Gruppe stehen oder gemeinsam mit dem Stickstoffatom, an das sie gebunden sind, einen gegebenenfalls durch eine oder mehrere C₁₋₃-Alkyl- oder Oxogruppen substituierten Heterocyclus bilden;
mit der Maßgabe, daß A und B beide für eine Ethylenylgruppe (-CH₂CH₂-) stehen, L für eine Einfachbindung steht und R₁ und R₄ nicht beide für eine unsubstituierte Phenylgruppe stehen können, wenn R₂, R₃, R₅, R₆, R₇ und R₈ alle für ein Wasserstoffatom stehen;
in Basen- oder Säureadditionssalzform sowie in Hydrat- oder Solvatform, sowie Enantiomere und Diastereomere davon und Gemische davon.

2. Verbindung der Formel (I) nach Anspruch 1, **dadurch gekennzeichnet, daß**:
• A für eine gegebenenfalls durch eine oder mehrere Gruppen R₉, die gleich oder voneinander verschieden sein können, substituierte C₁₋₄-Alkylengruppe steht;
• B für eine gegebenenfalls durch eine oder mehrere Gruppen R₁₀, die gleich oder voneinander verschieden sein können, substituierte C₁₋₄-Alkylengruppe steht;
• R₉ und R₁₀ jeweils für ein Wasserstoffatom stehen oder gemeinsam eine C₁₋₄-Alkylengruppe bilden;
• L für eine Einfachbindung oder -CH=CH- steht;
• R₁ für Aryl oder Heteroaryl steht, wobei die Aryl- und Heteroarylgruppen gegebenenfalls durch einen oder mehrere Reste Z, die gleich oder voneinander verschieden sein können, substituiert sind;
• R₂ und R₃ unabhängig voneinander für ein Wasserstoffatom oder eine C₁₋₃-Alkylgruppe stehen;
• R₄ für ein Wasserstoffatom oder eine C₁₋₅-Alkyl-, C₃₋₆-Cycloalkyl-, C₃₋₆-Cycloalkyl-C₁₋₃-alkylen-, C₁₋₃-Alkyl-O-C₁₋₃-alkylen-, Aryl-, Aryl-C₁₋₃-alkylen-, Aryl-C₁₋₃-alkylen-O-C₁₋₃-alkylen-, Heteroaryl- oder Heteroaryl-C₁₋₃-alkylengruppe, wobei die Aryl-, Aryl-C₁₋₃-alkylen-, Heteroaryl- und Heteroaryl-C₁₋₃-alkylengruppen gegebenenfalls durch einen oder mehrere Reste Z, die gleich oder voneinander verschieden sein können, substituiert sind, steht;
• R₅ für ein Wasserstoffatom oder eine C₁₋₅-Alkyl-, C₁₋₅-Alkoxy- oder Arylgruppe, wobei die Arylgruppe gegebenenfalls durch einen oder mehrere Reste Z, die gleich oder voneinander verschieden sein können, substituiert, steht;
• R₆ für ein Wasserstoffatom steht;
• R₇ für ein Halogenatom oder eine C₁₋₅-Alkyl-, C₁₋₅-Alkoxy- oder Arylgruppe steht;
• R₈ für ein Wasserstoffatom steht;
• Z für ein Wasserstoff- oder Halogenatom oder eine C₁₋₅-Alkyl-, C₁₋₃-Fluoralkyl-, C₃₋₆-Cycloalkyl-, C₃₋₆-Cycloalkyl-C₁₋₃-alkylen-, Phenyl-, C₁₋₅-Alkoxy-, C₁₋₃-Fluoralkoxy-, C₁₋₃-Alkyl-O-C₁₋₃-alkylen-, HO-C₁₋₃-Alkylen-, NO₂-, -CN-, C₁₋₃-Alkyl-X- oder C₁₋₃-Alkyl-X-C₁₋₃-alkylengruppe, worin X für S, SO oder SO₂ steht,
oder eine RₐR_{b}N-, RₐR_{b}N-C₁₋₃-Alkylen-, RₐR_{b}NC(O)-, C₁₋₃-Alkyl-C(O)-, C₃₋₆-Cycloalkyl-C(O)-Gruppe
oder einen Oxorest steht
oder zwei benachbarte Reste Z gemeinsam eine C₁₋₃-Alkylendioxygruppe bilden;
• Rₐ und R_{b} jeweils unabhängig voneinander für ein Wasserstoffatom oder eine C₁₋₃-Alkyl- oder C₁₋₃-Alkyl-C(O)-Gruppe stehen
oder gemeinsam mit dem Stickstoffatom, an das sie gebunden sind, einen gegebenenfalls durch eine oder mehrere C₁₋₃-Alkyl- oder Oxogruppen substituierten Heterocyclus bilden;
in Basen- oder Säureadditionssalzform sowie in Hydrat- oder Solvatform, sowie Enantiomere und Diastereomere davon und Gemische davon.

3. Verbindung der Formel (I) nach Anspruch 1 oder 2, **dadurch gekennzeichnet, daß**:
• A für eine gegebenenfalls durch eine Gruppe R₉ substituierte C₁₋₄-Alkylengruppe steht;
• B für eine gegebenenfalls durch eine Gruppe R₁₀ substituierte C₁₋₄-Alkylengruppe steht;
• R₉ und R₁₀ jeweils für ein Wasserstoffatom stehen oder gemeinsam eine C₁₋₄-Alkylengruppe bilden;
• L für eine Einfachbindung oder -CH=CH- steht;
• R₁ für gegebenenfalls durch einen oder mehrere Reste Z, die gleich oder voneinander verschieden sein können, substituiertes Aryl steht;
• R₂ und R₃ unabhängig voneinander für ein Wasserstoffatom oder eine C₁₋₃-Alkylgruppe stehen;
• R₄ für ein Wasserstoffatom oder eine C₁₋₅-Alkyl-, C₃₋₆-Cycloalkyl-, C₃₋₆-Cycloalkyl-C₁₋₃-alkylen-, C₁₋₃-Alkyl-O-C₁₋₃-alkylen-, Aryl- oder Heteroarylgruppe, wobei die Aryl- und Heteroarylgruppen gegebenenfalls durch einen oder mehrere Reste Z, die gleich oder voneinander verschieden sein können, substituiert sind, steht;
• R₅ für ein Wasserstoffatom steht;
• R₆ für ein Wasserstoffatom steht;
• R₇ für ein Halogenatom, Methyl oder Methoxy steht;
• R₈ für ein Wasserstoffatom steht;
• Z für ein Wasserstoff- oder Halogenatom oder eine C₁₋₅-Alkyl-, C₁₋₅-Alkoxy-, C₁₋₃-Alkyl-O-C₁₋₃-alkylen-, Phenyl-, HO-C₁₋₃-Alkylen-, -CN- oder C₁₋₃-Alkyl-X-Gruppe, worin X für ein Schwefelatom steht,
oder eine RₐR_{b}N-, RₐR_{b}N-C₁₋₃-Alkylen-, RₐR_{b}NC(O)- oder C₁₋₃-Alkyl-C(O)-Gruppe
oder einen Oxorest steht
oder zwei benachbarte Reste Z gemeinsam eine C₁₋₃-Alkylendioxygruppe bilden;
• Rₐ und R_{b} jeweils unabhängig voneinander für ein Wasserstoffatom oder eine C₁₋₃-Alkyl- oder C₁₋₃-Alkyl-C(O)-Gruppe stehen;
in Basen- oder Säureadditionssalzform sowie in Hydrat- oder Solvatform, sowie Enantiomere und Diastereomere davon und Gemische davon.

4. Verbindungen der Formel (I) nach Anspruch 1, ausgewählt unter:
• 7-Methoxy-4-(4-methoxyphenyl)-N-[8-(2-naphthylmethyl)-8-azabicyclo[3.2.1]oct-3-yl]phthalazin-1-amin;
• 7-Methoxy-4-(4-methoxyphenyl)-N-[1-(2-naphthylmethyl)piperidin-4-yl]phthalazin-1-amin;
• 7-Methoxy-4-(3-methoxyphenyl)-N-[1-(2-naphthylmethyl)piperidin-4-yl]phthalazin-1-amin;
• 7-Methoxy-4-(2-methoxyphenyl)-N-[1-(2-naphthylmethyl)piperidin-4-yl]phthalazin-1-amin;
• 7-Methoxy-N-[1-(2-naphthylmethyl)piperidin-4-yl]-4-phenylphthalazin-1-amin;
• 4-(4-Methoxyphenyl)-7-methyl-N-[1-(2-naphthylmethyl)piperidin-4-yl]phthalazin-1-amin;
• 4-(3,4-Dimethoxyphenyl)-7-methoxy-N-[1-(2-naphthylmethyl)piperidin-4-yl]phthalazin-1-amin;
• N-[1-(1,3-Benzodioxol-5-ylmethyl)piperidin-4-yl]-7-methoxy-4-(4-methoxyphenyl)phthalazin-1-amindihydrochlorid;
• 4-Ethyl-7-methoxy-N-[1-(2-naphthylmethyl)piperidin-4-yl]phthalazin-1-amin;
• 4-Benzyl-7-methoxy-N-[1-(2-naphthylmethyl)piperidin-4-yl]phthalazin-1-amin;
• 7-Methoxy-4-(methoxymethyl)-N-[1-(2-naphthylmethyl)piperidin-4-yl]phthalazin-1-aminhydrochlorid;
• N-[1-(1-Benzofuran-5-ylmethyl)piperidin-4-yl]-7-methoxy-4-(4-methoxyphenyl)phthalazin-1-amin;
• N-[1-(3,4-Dimethylbenzyl)piperidin-4-yl]-7-methoxy-4-(4-methoxyphenyl)phthalazin-1-amin;
• 7-Methoxy-4-(4-methoxyphenyl)-N-{1-[(1-methyl-1H-indol-2-yl)methyl]piperidin-4-yl}phthalazin-1-amin;
• 4-Cyclopropyl-7-methoxy-N-[1-(2-naphthylmethyl)piperidin-4-yl]phthalazin-1-amin;
• N-[1-(3-Fluor-4-methoxybenzyl)piperidin-4-yl]-7-methoxy-4-(4-methoxyphenyl)phthalazin-1-amin;
• N-[1-(2,3-Dihydro-1,4-benzodioxin-6-ylmethyl)piperidin-4-yl]-7-methoxy-4-(4-methoxyphenyl)phthalazin-1-amin;
• 4-(1,3-Benzodioxol-5-yl)-7-methoxy-N-[1-(2-naphthylmethyl)piperidin-4-yl]phthalazin-1-amindihydrochlorid;
• 4-(4-Chlorphenyl)-7-methoxy-N-[1-(2-naphthylmethyl)piperidin-4-yl]phthalazin-1-amin;
• 7-Methoxy-N-[1-(2-naphthylmethyl)piperidin-4-yl]-4-[4-(trifluormethyl)phenyl]phthalazin-1-amin;
• N-[1-(1-Benzothien-2-ylmethyl)piperidin-4-yl]-7-methoxy-4-(4-methoxyphenyl)phthalazin-1-amin;
• 4-Cyclopentyl-7-methoxy-N-[1-(2-naphthylmethyl)piperidin-4-yl]phthalazin-1-amin-dihydrochlorid;
• N-[1-(2,3-Dihydro-1-benzofuran-5-ylmethyl)piperidin-4-yl]-7-methoxy-4-(4-methoxyphenyl)phthalazin-1-amin;
• N-[1-(2,3-Dihydro-1H-inden-5-ylmethyl)piperidin-4-yl]-7-methoxy-4-(4-methoxyphenyl)phthalazin-1-amin;
• 7-Methoxy-4-(4-methoxyphenyl)-N-{1-[1-(2-naphthyl)ethyl]piperidin-4-yl}phthalazin-1-amindihydrochlorid;
• N-[1-(1,3-Benzothiazol-6-ylmethyl)piperidin-4-yl]-7-methoxy-4-(4-methoxyphenyl)phthalazin-1-amin;
• N-[1-(1-Benzothien-5-ylmethyl)piperidin-4-yl]-7-methoxy-4-(4-methoxyphenyl)phthalazin-1-amin;
• N-[1-(1H-Indol-3-ylmethyl)piperidin-4-yl]-7-methoxy-4-(4-methoxyphenyl)phthalazin-1-amin;
• 7-Methoxy-4-(4-methylphenyl)-N-[1-(2-naphthylmethyl)piperidin-4-yl]phthalazin-1-amin;
• 4-Butyl-7-methoxy-N-[1-(2-naphthylmethyl)piperidin-4-yl]phthalazin-1-amin;
• 7-Methoxy-4-(4-methoxyphenyl)-N-{1-[(2E)-3-phenylprop-2-en-1-yl]piperidin-4-yl}phthalazin-1-amin-dihydrochlorid;
• 7-Methoxy-N-[1-(2-naphthylmethyl)piperidin-4-yl]phthalazin-1-amin;
• 4-(Cyclopropylmethyl)-7-methoxy-N-[1-(2-naphthylmethyl)piperidin-4-yl]phthalazin-1-amin;
• N-[1-(3-Fluor-4-methylbenzyl)piperidin-4-yl]-7-methoxy-4-(4-methoxyphenyl)phthalazin-1-amin;
• 7-Methoxy-4-(4-methoxyphenyl)-N-[1-(4-methylbenzyl)piperidin-4-yl]phthalazin-1-amin;
• 4-(4-Fluorphenyl)-7-methoxy-N-[1-(2-naphthylmethyl)piperidin-4-yl]phthalazin-l-amin Verbindung Nr. 82;
• N-[1-(1H-Indol-5-ylmethyl)piperidin-4-yl]-7-methoxy-4-(4-methoxyphenyl)phthalazin-1-amin;
• 7-Methoxy-4-[4-(methoxymethyl)phenyl]-N-[1-(2-naphthylmethyl)piperidin-4-yl]phthalazin-1-amin;
• 7-Methoxy-N-[1-(4-methoxy-3-methylbenzyl)piperidin-4-yl]-4-(4-methoxyphenyl)phthalazin-1-amin;
• 7-Methoxy-4-methyl-N-[1-(2-naphthylmethyl)piperidin-4-yl]phthalazin-1-amin;
• 4-(2,5-Dimethoxyphenyl)-7-methoxy-N-[1-(2-naphthylmethyl)piperidin-4-yl]phthalazin-1-amindihydrochlorid;
• 7-Methoxy-4-(4-methoxyphenyl)-N-[1-(chinolin-6-ylmethyl)piperidin-4-yl]phthalazin-1-amin;
• 7-Methoxy-4-(4-methoxyphenyl)-N-{1-[(1-methyl-1H-indol-5-yl)methyl]piperidin-4-yl}phthalazin-1-amin;
• 4-(2,4-Dimethoxyphenyl)-7-methoxy-N-[1-(2-naphthylmethyl)piperidin-4-yl]phthalazin-1-amindihydrochlorid;
• 4-(3,5-Dimethoxyphenyl)-7-methoxy-N-[1-(2-naphthylmethyl)piperidin-4-yl]phthalazin-1-amindihydrochlorid;
• 7-Methoxy-N-[1-(2-naphthylmethyl)piperidin-4-yl]-4-(2-phenylethyl)phthalazin-1-amin-dihydrochlorid;
• 4-{4-[(Diethylamino)methyl]phenyl}-7-methoxy-N-[1-(2-naphthylmethyl)piperidin-4-yl]phthalazin-1-amin-trihydrochlorid;
• 7-Fluor-4-(4-fluorphenyl)-N-[1-(2-naphthylmethyl)piperidin-4-yl]phthalazin-1-amin;
• 7-Methoxy-N-[1-(2-naphthylmethyl)piperidin-4-yl]-4-pyridin-3-ylphthalazin-1-amin-trihydrochlorid;
• N-[1-(4-Chlorbenzyl)piperidin-4-yl]-7-methoxy-4-(4-methoxyphenyl)phthalazin-1-amin-dihydrochlorid;
• {4-[(4-{[7-Methoxy-4-(4-methoxyphenyl)phthalazin-1-yl]amino}piperidin-1-yl)methyl]phenyl}methanol-dihydrochlorid;
• N-{1-[4-(Aminomethyl)benzyl]piperidin-4-yl}-7-methoxy-4-(4-methoxyphenyl)phthalazin-1-amin-trihydrochlorid;
• N-{4-[(4-{[7-Methoxy-4-(4-methoxyphenyl)phthalazin-1-yl]amino}piperidin-1-yl)methyl]phenyl}acetamid-dihydrochlorid;
• 1-{4-[(4-{[7-Methoxy-4-(4-methoxyphenyl)phthalazin-1-yl]amino}piperidin-1-yl)methyl]phenyl}ethanon-dihydrochlorid;
• N-[1-(1-Benzofuran-5-ylmethyl)piperidin-4-yl]-4-(2,5-dimethoxyphenyl)-7-methoxyphthalazin-1-amindihydrochlorid;
• N-[1-(1,3-Benzodioxol-5-ylmethyl)piperidin-4-yl]-4-(2,5-dimethoxyphenyl)-7-methoxyphthalazin-1-amin-dihydrochlorid;
• 6-[(4-{[7-Methoxy-4-(4-methoxyphenyl)phthalazin-1-yl]amino}piperidin-1-yl)methyl]-2H-chromen-2-on(dihydrochlorid)-dihydrochlorid;
• N-[1-(1H-Indol-6-ylmethyl)piperidin-4-yl]-7-methoxy-4-(4-methoxyphenyl)phthalazin-1-amin;
• 7-Methoxy-N-[1-(4-methoxybenzyl)piperidin-4-yl]-4-(4-methoxyphenyl)phthalazin-1-amin-dihydrochlorid;
• N-[1-(4-Ethylbenzyl)piperidin-4-yl]-7-methoxy-4-(4-methoxyphenyl)phthalazin-1-amin-dihydrochlorid;
• N-[1-(1,3-Benzothiazol-6-ylmethyl)piperidin-4-yl]-4-(2,5-dimethoxyphenyl)-7-methoxyphthalazin-1-amin;
• 7-Methoxy-N-[1-(2-naphthylmethyl)piperidin-4-yl]-4-(phenoxymethyl)phthalazin-1-amin-dihydrochlorid;
• N-[1-(1H-Benzimidazol-5-ylmethyl)piperidin-4-yl]-7-methoxy-4-(4-methoxyphenyl)phthalazin-1-amin;
• 7-Chlor-4-(4-methoxyphenyl)-N-[1-(2-naphthylmethyl)piperidin-4-yl]phthalazin-1-amin-dihydrochlorid;
• 4-(4-Bromphenyl)-7-methoxy-N-[1-(2-naphthylmethyl)piperidin-4-yl]phthalazin-1-amin-dihydrochlorid;
• N-[1-(1,3-Benzodioxol-5-ylmethyl)piperidin-4-yl]-7-methoxy-4-(methoxymethyl)phthalazin-1-amindihydrochlorid;
• 4-[(Benzyloxy)methyl]-7-methoxy-N-[1-(2-naphthylmethyl)piperidin-4-yl]phthalazin-1-amindihydrochlorid;
• 7-Methoxy-5-phenyl-N-[1-(2-naphthylmethyl)piperidin-4-yl]phthalazin-1-amin-dihydrochlorid;
• 4-(Hydroxymethyl)-7-methoxy-N-[1-(2-naphthylmethyl)piperidin-4-yl]-4-(phenoxymethyl)phthalazin-1-amin;
• (-)-7-Methoxy-4-(4-methoxyphenyl)-N-{1-[1-(2-naphthyl)ethyl]piperidin-4-yl}phthalazin-1-amin;
• (+)-7-Methoxy-4-(4-methoxyphenyl)-N-{1-[1-(2-naphthyl)ethyl]piperidin-4-yl}phthalazin-1-amin;
• N-{1-[4-(Dimethylaminomethyl)benzyl]piperidin-4-yl}-7-methoxy-4-(4-methoxyphenyl)phthalazin-1-amin-trihydrochlorid.

5. Verfahren zur Herstellung einer Verbindung der Formel (I) nach einem der Ansprüche 1 bis 4, **dadurch gekennzeichnet, daß** man eine Verbindung der allgemeinen Formel (III) 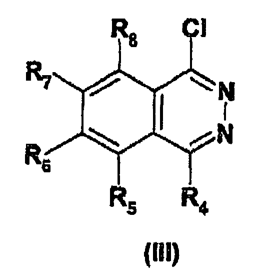 worin R₄, R₅, R₆, R₇ und R₈ die in der allgemeinen Formel (I) gemäß Anspruch 1 angegebene Bedeutung besitzen,
mit einer Verbindung der allgemeinen Formel (II) 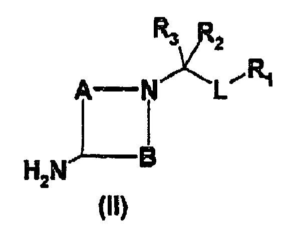 worin R₁, R₂, R₃, L, A und B die in der allgemeinen Formel (I) gemäß Anspruch 1 angegebene Bedeutung besitzen, umsetzt.

6. Medikament, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure oder auch ein Hydrat oder ein Solvat der Verbindung der Formel (I) enthält.

7. Pharmazeutische Zusammensetzung, **dadurch gekennzeichnet, daß** sie mindestens eine Verbindung der Formel (I) nach einem der Ansprüche 1 bis 4 oder ein pharmazeutisch unbedenkliches Salz, ein Hydrat oder ein Solvat dieser Verbindung sowie mindestens einen pharmazeutisch unbedenklichen Hilfsstoff enthält.

8. Verwendung einer Verbindung der Formel (I) nach einem der Ansprüche 1 bis 4 zur Herstellung eines Medikaments zur Behandlung von Erkrankungen mit Dysfunktion in Verbindung mit dem MCH₁-Rezeptor.

9. Verwendung einer Verbindung der Formel (I) nach einem der Ansprüche 1 bis 4 zur Herstellung eines Medikaments zur Behandlung von Adipositas, Cellulite, Stoffwechselstörungen und damit assoziierten Pathologien wie Diabetes, Herz-Kreislauf-Störungen und Syndrom X und bei der Behandlung von Pathologien in Verbindung mit Streß wie Angst und Depression.
